(19) Europäisches Patentamt
European Patent Office
Office européen des brevets

(11) EP 4 023 682 B1

(12) EUROPEAN PATENT SPECIFICATION

(45) Date of publication and mention of the grant of the patent:
**23.04.2025 Bulletin 2025/17**

(21) Application number: **20856031.8**

(22) Date of filing: **28.08.2020**

(51) International Patent Classification (IPC):
***C08F 8/34*** *(2006.01)* ***C08F 212/06*** *(2006.01)*
***C08F 220/32*** *(2006.01)* ***C08F 265/06*** *(2006.01)*
***G01N 33/543*** *(2006.01)* ***G01N 33/53*** *(2006.01)*

(52) Cooperative Patent Classification (CPC):
(C-Sets available)
**G01N 33/5304; C08F 8/32; C08F 8/34;**
**C08F 212/06; C08F 265/06; G01N 33/543** (Cont.)

(86) International application number:
**PCT/JP2020/032656**

(87) International publication number:
**WO 2021/039982 (04.03.2021 Gazette 2021/09)**

(54) **PARTICLES, AFFINITY PARTICLES HAVING LIGAND FOR TARGET SUBSTANCE, IN VITRO DIAGNOSTIC REAGENT AND KIT THAT INCLUDE SAME, AND METHOD FOR DETECTING TARGET SUBSTANCE**

PARTIKEL, AFFINITÄTSPARTIKEL MIT LIGAND FÜR ZIELSUBSTANZ, IN-VITRO-DIAGNOSTISCHES REAGENZ UND KIT DAMIT UND VERFAHREN ZUM NACHWEIS EINER ZIELSUBSTANZ

PARTICULES, PARTICULES D’AFFINITÉ AYANT UN LIGAND POUR SUBSTANCE CIBLE, RÉACTIF DE DIAGNOSTIC IN VITRO ET KIT LES COMPRENANT, ET PROCÉDÉ DE DÉTECTION D’UNE SUBSTANCE CIBLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.08.2019 JP 2019158952**
**30.08.2019 JP 2019158962**
**30.08.2019 JP 2019158138**

(43) Date of publication of application:
**06.07.2022 Bulletin 2022/27**

(73) Proprietors:
- **CANON KABUSHIKI KAISHA**
**Ohta-ku**
**Tokyo 146-8501 (JP)**
- **Canon Medical Systems Corporation**
**Otawara-shi, Tochigi 324-8550 (JP)**

(72) Inventors:
- **NATORI, Ryo**
**Tokyo 146-8501 (JP)**
- **SUDA, Sakae**
**Tokyo 146-8501 (JP)**
- **YAMAUCHI, Fumio**
**Tokyo 146-8501 (JP)**
- **KANAZAKI, Kengo**
**Tokyo 146-8501 (JP)**
- **SEKIGUCHI, Takeshi**
**Tokyo 146-8501 (JP)**
- **TANI,Yutaka**
**Tokyo 146-8501 (JP)**
- **NAKAHAMA, Kazumichi**
**Tokyo 146-8501 (JP)**
- **NAKASU, Minako**
**Tokyo 146-8501 (JP)**

(74) Representative: **TBK**
**Bavariaring 4-6**
**80336 München (DE)**

(56) References cited:
**WO-A1-2019/208672** **JP-A- 2010 260 877**
**JP-A- 2014 153 140** **JP-A- H0 445 111**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 023 682 B1

**JP-A- H01 115 923**
**JP-A- H04 218 772**
**JP-A- H09 100 320**
**JP-A- S 636 463**
**JP-A- S63 230 086**
**KR-B1- 101 176 905**
**US-A1- 2007 099 814**
**US-A1- 2007 224 424**

(52) Cooperative Patent Classification (CPC): (Cont.)

C-Sets
**C08F 8/34, C08F 220/325;**
C08F 220/325, C08F 212/08, C08F 212/36

## Description

[Technical Field]

The present invention relates to a particle, an affinity particle including a ligand for a target substance, and an in vitro diagnostic reagent and kit each including the affinity particle, and to a method of detecting a target substance.

[Background Art]

An example of a simple and rapid immunological test method is a latex agglutination method. In the method, a dispersion of an affinity particle obtained by bonding a particle and a ligand having an affinity for a target substance to each other, and a specimen that may contain the target substance are mixed. At this time, when the specimen contains the target substance, the affinity particle causes an agglutination reaction, and hence the presence or absence of a disease can be identified by optically detecting the agglutination reaction as a variation in, for example, scattered light intensity, transmitted light intensity, or absorbance.

The combination of an antibody and an antigen, or of an antigen and an antibody is generally used as the combination of the ligand and the target substance. The particle to be used in the latex agglutination method and the affinity particle characterized by including, on the surface of the particle, the ligand for the target substance are each desired to show such a characteristic as to adsorb to a substance except the target substance, that is, so-called nonspecific adsorption to a small extent for the purpose of preventing the occurrence of a nonspecific agglutination reaction that is not derived from the target substance. In addition, the dispersion of the affinity particle may be left at rest and stored for several weeks in some test institutes, and hence a contrivance to maintain the dispersed state of the affinity particle in the dispersion is an extremely important technical problem.

A method including coating a particle surface with a biologically derived substance, such as albumin, casein, or gelatin, is available as means for reducing the nonspecific adsorption of the particle or the affinity particle. However, the physical properties of such biologically derived substance may vary from production lot to production lot. In addition, an opinion is heard that concern is raised about future biological contamination due to the use of a large amount of such substance.

Means for coating the surface of the particle or the affinity particle with an amphipathic polymer compound is also effective as a method of reducing the nonspecific adsorption. However, the adsorption of the polymer compound to the particle or the affinity particle is derived from physical adsorption. Accordingly, the compound may be liberated by its dilution, and hence the nonspecific adsorption cannot be sufficiently suppressed in some cases.

A particle having polyglycidyl methacrylate arranged on its surface has been known as a particle causing small nonspecific adsorption. It has been assumed that the nonspecific adsorption is suppressed because part of the glycidyl groups of the polyglycidyl methacrylate arranged on the surface of the particle undergo ring opening to form a glycol.

In Patent Literature 1, there is a disclosure of a case in which a particle obtained by chemically bonding a ligand to the surface of a particle having arranged thereon polyglycidyl methacrylate through a polyethylene glycol chain is applied to bioseparation.

In Patent Literature 2, there is a disclosure of a case in which a particle obtained by chemically bonding a ligand to the surface of a particle having arranged thereon polyglycidyl methacrylate through an amino acid is applied to a latex agglutination method. Patent Literature 3 discloses affinity particles comprising phosphorylcholine groups covalently bonded onto the surface of organic particles.

[Citation List]

[Patent Literature]

PTL 1: Japanese Patent Application Laid-Open No. 2000-351814
PTL 2: International Publication No. WO2007/063616
PTL 3: Patent Publication No. US2007/0241054

[Summary of Invention]

[Technical Problem]

The inventors of the present invention have made extensive investigations, and as a result, have recognized, for

example, that each of the particles of Patent Literature 1 and Patent Literature 2 may cause nonspecific adsorption in a high-concentration specimen, that each of the particles may not show sufficient sensitivity when applied to the latex agglutination method, and that concern is raised about the occurrence of electrostatic heteroagglutination under a standing condition.

The present invention has been made in view of such background art and technical problems. An object of the present invention is to provide a particle, which causes small nonspecific adsorption, has a reactive functional group for chemically bonding a ligand, and is suitable for a latex agglutination method, and an affinity particle excellent in dispersion stability. Another object of the present invention is to provide an in vitro diagnostic reagent and kit each including the particle or the affinity particle as a particle for a latex agglutination method, and to a method of detecting a target substance.

The present invention relates to a particle, an affinity particle including a ligand for a target substance, and an in vitro diagnostic reagent and kit each including the affinity particle, and a method of detecting a target substance.

[Solution to Problem]

That is, the present invention relates to a particle including, in a surface layer thereof, a copolymer having a repeating unit A and a repeating unit B, wherein the repeating unit A has a side chain A, and the side chain A has, at a terminal thereof, a carboxy group to be bonded to a ligand, wherein the repeating unit B has a side chain B, and the side chain B has a hydroxy group at a terminal thereof, wherein the particle is configured such that, when the particle is dispersed in ion-exchanged water, the surface layer of the particle is hydrated to form a swollen layer, wherein a density of the carboxy groups to be incorporated into the swollen layer satisfies the formula (1-1), and wherein a dry particle diameter to be measured when the particle is dried and a particle diameter in water to be measured when the particle is dispersed in ion-exchanged water satisfy the formula (1-2).

$$0.040 \leq [\text{Carboxy group density (group/nm}^3)] \leq 0.150 \cdots \text{Formula (1-1)}$$

$$1.10 \leq [\text{Particle diameter in water/dry particle diameter}] \leq 1.40 \cdots \text{Formula (1-2)}$$

The present invention also relates to an affinity particle including: the particle; and a ligand bonded to the particle.

The present invention also relates to a reagent for use in detection of a target substance in a specimen by in vitro test diagnosis, the reagent including the affinity particle, and to the reagent for use in the detection of the target substance in the specimen by an agglutination method (latex agglutination method).

Another embodiment of the present invention relates to an affinity particle including: a particle; and a ligand on a surface of the particle, wherein a ratio of an area occupied by the ligand to the surface of the particle satisfies a relationship of the formula (2-1), wherein zeta potentials of the particle and the ligand satisfy a relationship of the formula (2-2), and wherein the particle has a repeating unit A represented by the formula (2-3).

$$10 \leq [\text{Occupied area ratio (\%)}] \leq 40 \cdots \text{Formula (2-1)}$$

$$0 \leq [||\text{Zeta potential of particle}|-|\text{zeta potential of ligand}||] \leq 20 \cdots \text{Formula (2-2)}$$

[Chem. 1]

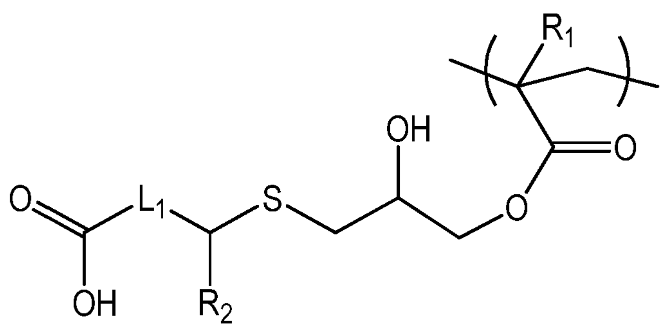

(2-3)

In the formula (2-3), $R_1$ represents a methyl group or a hydrogen atom, $R_2$ represents a carboxy group or a hydrogen atom, and $L_1$ represents an alkylene group having 1 to 15 carbon atoms that may have a substituent, or an

oxyalkylene group having 1 to 15 carbon atoms that may have a substituent.

In addition, another embodiment of the present invention relates to a reagent for use in detection of a target substance in a specimen by in vitro diagnosis, the reagent including the affinity particle, and to the reagent for use in the detection of the target substance in the specimen by an agglutination method (latex agglutination method).

That is, a first aspect of still another embodiment of the present invention relates to a particle including a copolymer containing a repeating unit A represented by the following formula (3-1), the particle being capable of chemically bonding a ligand to a surface thereof in high yield by having, in the repeating unit A, a structure containing a sulfide group.

[Chem. 2]

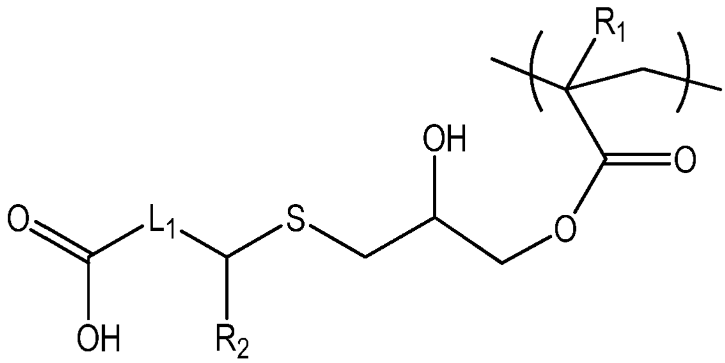

(3-1)

**In** addition, a second aspect of still another embodiment of the present invention relates to a particle for a latex agglutination method including a ligand chemically bonded thereto through the repeating unit A.

**In** addition, a third aspect of still another embodiment of the present invention relates to a reagent for use in detection of a target substance in a specimen by in vitro diagnosis, the reagent including the particle for a latex agglutination method, a kit for use in detection of the target substance in the specimen by in vitro diagnosis, the kit including at least the reagent, and to a detection method including mixing the particle for a latex agglutination method and the specimen that may contain the target substance.

[Advantageous Effects of Invention]

According to the present invention, the particle, which causes small nonspecific adsorption, has a reactive functional group for bonding a ligand, and is suitable for an agglutination method (latex agglutination method), can be provided.

Further, according to the present invention, the in vitro diagnostic reagent and kit each including, as a particle for an agglutination method (latex agglutination method), an affinity particle including a ligand bonded thereto, and the method of detecting a target substance can be provided.

**In** addition, according to another embodiment of the present invention, the affinity particle, which causes small nonspecific adsorption and is excellent in dispersion stability, can be provided. According to still another embodiment of the present invention, the high-sensitivity in vitro diagnostic reagent and kit each including the affinity particle as a particle for an agglutination method (latex agglutination method), and the method of detecting a target substance can be provided.

[Description of Embodiments]

[First Embodiment]

A first embodiment of the present invention is described in detail below, but the technical scope of the present invention is not limited to the embodiment.

A particle according to the first embodiment of the present invention is a particle including, in a surface layer thereof, a copolymer having a repeating unit A having a side chain A having, at a terminal thereof, a carboxy group to be bonded to a ligand and a repeating unit B having a side chain B having a hydroxy group at a terminal thereof.

A particle to be used in an agglutination method (e.g., a latex agglutination method) is preferably excellent in dispersion stability in an aqueous dispersion, and hence the carboxy group preferably forms a carboxylate. Examples of the carboxylate include: a metal salt, such as a sodium salt or a potassium salt; and an organic salt, such as an ammonium salt. In consideration of reactivity at the time of the bonding of the carboxy group and the ligand by a chemical reaction, an organic salt is more preferred. Examples of an organic base for forming the organic salt with the carboxy group include

ammonia, diethylamine, triethylamine, ethanolamine, and diethylaminoethanol. However, the present invention is not limited thereto. Triethylamine is easy to use in consideration of experimental operability, such as a boiling point or solubility in various solvents. The organic bases for forming the carboxylate may be used alone or in combination thereof to the extent that the object of the first embodiment can be achieved. Similarly, a metal base and the organic base may be used in combination.

When the particle of the first embodiment is dispersed in water or an aqueous solution, the surface layer of the particle is hydrated to form a hydrogel-like swollen layer. A feature of the swollen layer lies in that the density of the carboxy groups to be incorporated into the swollen layer satisfies the following formula (1-1), and a dry particle diameter to be measured when the particle is dried and a particle diameter in water to be measured when the particle is dispersed in ion-exchanged water satisfy the formula (1-2).

$$0.04 \leq [\text{Carboxy group density (group/nm}^3)] \leq 0.15 \cdots \text{Formula (1-1)}$$

$$1.10 \leq [\text{Particle diameter in water/dry particle diameter}] \leq 1.40 \cdots \text{Formula (1-2)}$$

When the carboxy group density is less than 0.04 group/nm$^3$, reactivity between the particle and the ligand (hereinafter represented as "sensitization rate") is not sufficient. A case in which the carboxy group density is more than 0.15 group/nm$^3$ is not preferred because the swollen layer has a high water-binding force, and hence when the particle of the first embodiment is applied to the latex agglutination method, osmotic pressure agglutination occurs to be observed as an artificial nonspecific adsorption phenomenon. **In** addition, when the carboxy group density is more than 0.15 group/nm$^3$, the zeta potential of the particle increases. Accordingly, when the ligand is bonded to the particle to provide an affinity particle, an electrostatic interaction between the ligand and the surface of the particle occurs to impair reactivity between the ligand and a target substance, thereby reducing the detection sensitivity of the latex agglutination method. Further, as the carboxy group density becomes larger, a difference in zeta potential between the particle and the ligand also becomes larger. The foregoing means that charges having different signs are imparted to the surfaces of the affinity particles each obtained by bonding the ligand to the particle, and hence the electrostatic agglutination of the affinity particles is accelerated. Accordingly, the zeta potential of the particle of the first embodiment preferably satisfies the formula (1-3).

$$-30 \leq [\text{Zeta potential (mV)}] \leq -10 \cdots \text{Formula (1-3)}$$

As described above, a preferred range exists for the carboxy group density from the viewpoints of the sensitization rate, the osmotic pressure agglutination, the detection sensitivity when the affinity particle is applied to the latex agglutination method, and the electrostatic agglutination of the affinity particles. Accordingly, the carboxy group density is preferably 0.04 group/nm$^3$ or more and 0.15 group/nm$^3$ or less, more preferably 0.10 group/nm$^3$ or more and 0.13 group/nm$^3$ or less.

The formula (1-2) correlates with the thickness of the swollen layer. A smaller value of the ratio represented by the formula (1-2) means that the swollen layer is thinner, and a larger value of the ratio represented by the formula (1-2) means that the swollen layer is thicker. When the ratio [particle diameter in water/dry particle diameter] is less than 1.10, it may be difficult to expect a nonspecific adsorption-suppressing effect based on the hydrous swollen layer. In addition, the swollen layer is not sufficiently hydrous, and hence the mobility of the carboxy group derived from the repeating unit A of the swollen layer in an aqueous dispersion is not large. Accordingly, it may be difficult to achieve a sufficient sensitization rate. Further, when the ligand is bonded to the particle to provide the affinity particle, the mobility of the ligand is inhibited to impair reactivity between the ligand and a target substance, and the impaired reactivity may be responsible for a reduction in detection sensitivity of the latex agglutination method. The fact that the ratio [particle diameter in water/dry particle diameter] is 1.10 or more largely contributes to the dispersion stability of the particle. The particle to be used in the latex agglutination method is preferably excellent in dispersion stability in a normal specimen or a buffer typified by physiological saline. In this regard, when the ratio [particle diameter in water/dry particle diameter] is 1.10 or more, the swollen layer is sufficiently hydrous to impart dispersion stability based on an excluded volume effect to the particle. Meanwhile, a case in which the ratio [particle diameter in water/dry particle diameter] is more than 1.40 is not preferred because the swollen layer has so large a water-binding force that, when the particle of the first embodiment is applied to the latex agglutination method, osmotic pressure agglutination occurs to be observed as an artificial nonspecific adsorption phenomenon.

As described above, a preferred range exists for the thickness of the swollen layer from the viewpoints of the sensitization rate, the detection sensitivity when the affinity particle is applied to the latex agglutination method, the dispersion stability of the particle, and the osmotic pressure agglutination. In view of the foregoing, the ratio [particle diameter in water/dry particle diameter] is preferably 1.10 or more and 1.40 or less, more preferably 1.15 or more and 1.30

or less.

It is preferred that the repeating unit A and repeating unit B of the copolymer for forming the surface layer of the particle of the first embodiment have the side chain A having, at the terminal thereof, the carboxy group to be bonded to the ligand and the side chain B having the hydroxy group at the terminal thereof, respectively, and the number of moles of the repeating unit A and the number of moles of the repeating unit B satisfy the formula (1-4).

$$0.05 \leq [\text{Number of moles of repeating unit A}]/[\text{number of moles of repeating unit B}] \leq 1.00 \qquad \text{Formula (1-4)}$$

When the ratio [number of moles of repeating unit A]/[number of moles of repeating unit B] is less than 0.05, concern is raised about a reduction in sensitization rate, though the reduction is not fatal. In addition, a reduction in ratio of the repeating unit A to the surface layer of the particle is identical in meaning to a reduction in amount of a carboxy group that is a negative charge-generating source, and a rise in difficulty with which dispersion stability derived from electrostatic repulsion is imparted to the particle may be of potential concern. When the ratio [number of moles of repeating unit A]/[number of moles of repeating unit B] is more than 1.00, the water-binding force of the swollen layer formed by the hydration of the surface layer of the particle becomes larger. Accordingly, when the particle is mixed with a high-concentration specimen, osmotic pressure agglutination may occur.

A specific chemical structure of the repeating unit A of the copolymer for forming the surface layer of the particle of the first embodiment is described. The repeating unit A is characterized by having, at the terminal of the side chain thereof, the carboxy group to be bonded to the ligand, and its chemical structure is not limited to the extent that the object of the first embodiment can be achieved. However, a side chain structure represented by the formula (1-5) is more preferred:

[Chem. 3]

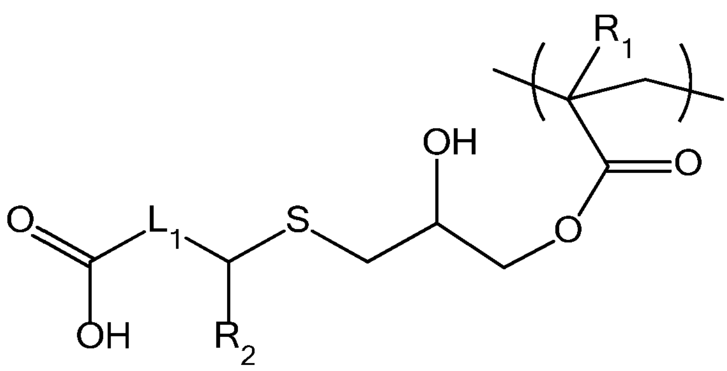

(1-5)

where $R_1$ represents a methyl group or a hydrogen atom, $R_2$ represents a carboxy group or a hydrogen atom, and $L_1$ represents an alkylene group or oxyalkylene group having 1 to 15 carbon atoms that may be substituted.

A hydroxy group adjacent to an ester bond in the formula (1-5) serves to reduce the nonspecific adsorption of the particle. A sulfide bond is a chemical structure showing a weak hydrophobic tendency. When the repeating unit A has the side chain structure represented by the formula (1-5), the water-binding force of the swollen layer formed by the hydration of the surface layer of the particle is moderately weakened. Accordingly, a suppressing action on osmotic pressure agglutination that may occur when the particle is mixed with a high-concentration specimen is expected.

In the formula (1-5), $L_1$ preferably represents an alkylene group having 1 carbon atom. The number of the carbon atoms of $L_1$ correlates with the hydrophilicity or hydrophobicity of the repeating unit A, and when the number of the carbon atoms becomes excessively large, the nonspecific adsorption of a target substance to the particle may be accelerated.

From the viewpoint of the sensitization rate of the particle of the first embodiment, $R_2$ in the formula (1-5) preferably represents a carboxy group.

A specific chemical structure of the repeating unit B of the copolymer for forming the surface layer of the particle of the first embodiment is described. The repeating unit B of the first embodiment is characterized by having the side chain B having the hydroxy group at the terminal thereof, and its chemical structure is not limited to the extent that the object of the first embodiment can be achieved. However, a side chain structure represented by the formula (1-6) is more preferred:

[Chem. 4]

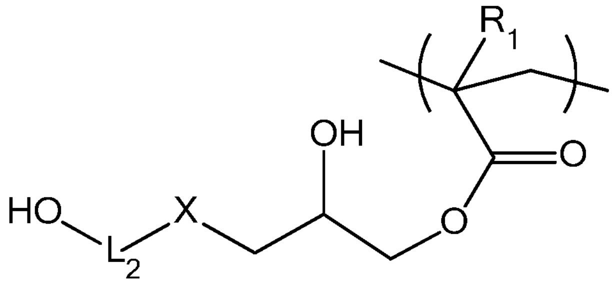

(1-6)

where $R_1$ represents a methyl group or a hydrogen atom, $L_2$ represents an alkylene group or oxyalkylene group having 2 to 15 carbon atoms that may be substituted, and has a relationship of [number of carbon atoms of $L_1$]+2≥[number of carbon atoms of $L_2$], and X represents a sulfur atom or a nitrogen atom that may be substituted.

A hydroxy group adjacent to an ester bond in the formula (1-6) and the hydroxy group at the terminal of the side chain serve to reduce the nonspecific adsorption of the particle. X in the formula (1-6), which may represent any one of a sulfur atom and a nitrogen atom, more preferably represents a sulfur atom. A sulfide bond is a structure showing a weak hydrophobic tendency. When the repeating unit B has the side chain structure represented by the formula (1-6), the water-binding force of the swollen layer formed by the hydration of the surface layer of the particle is moderately weakened. Accordingly, a suppressing action on osmotic pressure agglutination that may occur when the particle is mixed with a high-concentration specimen is expected.

When the formula (1-5) and the formula (1-6) are compared, a relationship between the number of the carbon atoms for forming $L_1$ in the formula (1-5) and the number of the carbon atoms for forming $L_2$ in the formula (1-6) is preferably a relationship of the formula (1-7). When the relationship of the formula (1-7) is not satisfied, the side chain B becomes relatively longer than the side chain A, and hence the reactivity of the carboxy group that the side chain A has at the terminal thereof may be inhibited. In view of the foregoing, when $L_1$ in the formula (1-5) represents an alkylene group having 1 carbon atom, $L_2$ in the formula (1-6) preferably represents an alkylene group having 2 carbon atoms or 3 carbon atoms.

[Number of carbon atoms of $L_1$]+2≥[number of carbon atoms of $L_2$]···Formula (1-7)

When $L_2$ in the formula (1-6) represents an alkylene group having 3 carbon atoms, a chemical structure in which one hydrogen atom of the alkylene group is substituted with a hydroxy group is more preferred from the viewpoint of reducing the nonspecific adsorption of the particle.

The particle of the first embodiment preferably has, as a repeating unit C, at least one kind selected from the group consisting of styrenes and (meth)acrylates, and more preferably contains a repeating unit derived from any one of the styrenes and glycidyl (meth)acrylate. As described in the "Background Art" section herein, a repeating unit derived from glycidyl (meth)acrylate has a reducing action on the nonspecific adsorption of the particle. Meanwhile, the reason why the particle preferably contains a repeating unit derived from any one of the styrenes is as described below. When the particle of the first embodiment is purified by a method such as centrifugal separation or ultrafiltration, the presence of the repeating unit derived from any one of the styrenes, which has a high glass transition temperature and is excellent in mechanical strength, contributes to the suppression of damage to the particle, such as cracking or chipping. Examples of the styrenes include styrene, α-methylstyrene, β-methylstyrene, o-methylstyrene, m-methylstyrene, p-methylstyrene, 2,4-dimethylstyrene, p-n-butylstyrene, p-tert-butylstyrene, p-n-hexylstyrene, p-n-octylstyrene, p-n-nonylstyrene, p-n-decylstyrene, p-n-dodecylstyrene, p-methoxystyrene, and p-phenylstyrene. However, the styrenes are not limited thereto to the extent that the object of the first embodiment can be achieved. In addition, the two or more kinds of styrenes may be used in combination. When the mass of the particle is defined as 100 parts by mass, the content of the styrenes is preferably 10 parts by mass or more and 70 parts by mass or less because sufficient strength can be imparted to the particle while the nonspecific adsorption is reduced.

A repeating unit derived from any one of radical-polymerizable monomers each having crosslinkability may be further incorporated into the particle of the first embodiment. Examples of the radical-polymerizable monomers each having crosslinkability include diethylene glycol diacrylate, triethylene glycol diacrylate, tetraethylene glycol diacrylate, polyethylene glycol diacrylate, 1,6-hexanediol diacrylate, neopentyl glycol diacrylate, tripropylene glycol diacrylate, polypropylene glycol diacrylate, 2,2'-bis(4-(acryloxydiethoxy)phenyl)propane, trimethylolpropane triacrylate, tetramethylolmethane tetraacrylate, ethylene glycol dimethacrylate, diethylene glycol dimethacrylate, triethylene glycol dimethacrylate, tetraethylene glycol dimethacrylate, polyethylene glycol dimethacrylate, 1,3-butylene glycol dimethacrylate, 1,6-hexanediol dimethacrylate, neopentyl glycol dimethacrylate, polypropylene glycol dimethacrylate, 2,2'-bis(4-(methacryloxydiethoxy)phenyl)propane, 2,2'-bis(4-(methacryloxypolyethoxy)phenyl)propane, trimethylolpropane trimethacrylate, tetramethylolmethane tetramethacrylate, divinylbenzene, divinylnaphthalene, and divinyl ether. However, the monomers are not limited thereto to the extent that the object of the first embodiment can be achieved. In addition, the two or more kinds of radical-polymerizable monomers each having crosslinkability may be used in combination.

The particle diameter of the particle of the first embodiment is preferably 0.05 μm or more and 1.00 μm or less, more preferably 0.05 μm or more and 0.50 μm or less, still more preferably 0.05 μm or more and 0.30 μm or less in terms of number-average particle diameter. When the particle diameter is 0.05 μm or more and 0.30 μm or less, the particle is easy to handle, and in the case of long-term storage of the particle as a dispersion, the sedimentation of the particle hardly occurs.

A typical method of producing the particle of the first embodiment is described. However, the method of producing the particle of the first embodiment is not limited to the extent that the object of the first embodiment can be achieved.

The method includes a step 1 of mixing glycidyl (meth)acrylate, styrene, divinylbenzene, water, and a radical polymerization initiator to form a particulate copolymer, thereby providing an aqueous dispersion of the particulate copolymer. The method includes a step 2 of mixing the aqueous dispersion, 3-mercapto-1,2-propanediol, and mercaptosuccinic acid to prepare a mixed liquid, followed by causing of an epoxy group derived from glycidyl (meth)acrylate of the particulate copolymer, and a thiol group derived from each of 3-mercapto-1,2-propanediol and mercaptosuccinic acid to react with each other to form the particle of the first embodiment. The radical polymerization initiator is at least one of 4,4'-azobis(4-cyanovaleric acid), 2,2'-azobis[2-methyl-N-(2-hydroxyethyl)propionamide], 2,2'-azobis[N-(2-carboxyethyl)-2-methylpropionamidine] tetrahydrate, 2,2'-azobis(2-methylpropionamidine) dihydrochloride, 2,2'-azobis[2-(2-imidazolin-2-yl)propane], 2,2'-azobis[2-(2-imidazolin-2-yl)propane] dihydrochloride, or 2,2'-azobis[2-(2-imidazolin-2-yl)propane] disulfate dihydrate. The method includes a step of adjusting the pH of the mixed liquid of the step 2 within an alkaline region with an organic base free of any primary amine. In the step 2, the epoxy group derived from glycidyl (meth)acrylate of the particulate copolymer is caused to react with the thiol group derived from each of 3-mercapto-1,2-propanediol and mercaptosuccinic acid from the surface of the particulate copolymer in its depth direction. To form a surface layer having a sufficient thickness on the particle of the first embodiment, triethylamine having permeability into the particulate copolymer is preferably selected as the organic base.

A method of forming the particulate copolymer is not limited to the use of radical polymerization to the extent that the object of the first embodiment can be achieved. When the radical polymerization is applied, emulsion polymerization, soap-free emulsion polymerization, or suspension polymerization is preferably used, and the emulsion polymerization or the soap-free emulsion polymerization is more preferably used. The soap-free emulsion polymerization is still more preferably used. In general, the emulsion polymerization and the soap-free emulsion polymerization can each provide a particulate copolymer having a particle diameter distribution sharper than that of a particulate copolymer provided by the suspension polymerization. In addition, when the particle is bonded to the ligand to provide the affinity particle, concern is raised about the modification of the ligand by the presence of an anionic surfactant or a cationic surfactant to be generally used in the emulsion polymerization as a residue. In view of the foregoing, when the particulate copolymer is formed by the emulsion polymerization, a nonionic surfactant is preferably used.

The ligand is a compound that is specifically bonded to a receptor that a specific target substance has. The site at which the ligand is bonded to the target substance is decided, and the ligand has a selectively or specifically high affinity for the target substance. Examples of the ligand include: an antigen and an antibody; an enzyme protein and a substrate thereof; a signal substance typified by a hormone or a neurotransmitter, and a receptor thereof; a nucleic acid; and avidin and biotin. However, the ligand is not limited thereto to the extent that the object of the first embodiment can be achieved. Specific examples of the ligand include an antigen, an antibody, an antigen-binding fragment (e.g., Fab, F(ab')2, F(ab'), Fv, or scFv), a naturally occurring nucleic acid, an artificial nucleic acid, an aptamer, a peptide aptamer, an oligopeptide, an enzyme, and a coenzyme.

In the first embodiment, the particle of the first embodiment is meant to be used as a particle for a latex agglutination method that may be applied to a latex agglutination method in an immunological test.

In the first embodiment, a conventionally known method may be applied as a method for a chemical reaction by which the carboxy group or the carboxylate derived from the repeating unit A and the ligand are bonded to each other to the extent that the object of the first embodiment can be achieved. For example, a carbodiimide-mediated reaction or an NHS ester activation reaction is a suitable example of the chemical reaction. In addition, the following may be performed: avidin is bonded to the carboxy group, and a biotin-modified ligand is bonded to the resultant. However, the method for the chemical reaction by which the carboxy group or the carboxylate derived from the repeating unit A and the ligand are bonded to each other is not limited thereto to the extent that the object of the first embodiment can be achieved.

In the first embodiment, when an antibody (antigen) is used as the ligand and an antigen (antibody) is used as the target substance, a latex agglutination method in an immunological test that has been widely utilized in fields such as a clinical test and biochemical research may be extremely preferably applied as a method of detecting the target substance in a specimen in in vitro diagnosis. When a general particle is used as a particle for a latex agglutination method, the antigen

(antibody) that is the target substance, foreign matter in serum or plasma, or the like nonspecifically adsorbs to the surface of the particle, and hence concern is raised in that unintended interparticle agglutination occurs owing to the adsorption to impair the accuracy of the immunological test.

A reagent for use in detection of a target substance in a specimen by in vitro diagnosis of the first embodiment is characterized by including a particle for a latex agglutination method. The amount of the particle for a latex agglutination method to be incorporated into the reagent of the first embodiment is preferably from 0.001 mass% to 20 mass%, more preferably from 0.01 mass% to 10 mass%. The reagent of the first embodiment may include a third substance, such as a solvent or a blocking agent, in addition to the particle for a latex agglutination method to the extent that the object of the first embodiment can be achieved. Examples of the solvent to be used in the first embodiment include various aqueous buffers, such as a phosphate buffer, a glycine buffer, a Good's buffer, a Tris buffer, a HEPES buffer, a MES buffer, and an ammonia buffer. However, the solvent to be incorporated into the reagent of the first embodiment is not limited thereto.

A kit for use in detection of a target substance in a specimen by in vitro diagnosis of the first embodiment is characterized by including at least the reagent of the first embodiment. The kit of the first embodiment preferably further includes a reaction buffer containing an albumin (hereinafter referred to as "reagent 2") in addition to the reagent of the first embodiment (hereinafter referred to as "reagent 1"). The albumin is, for example, serum albumin, and may be subjected to a protease treatment. As a guide, the amount of the albumin to be incorporated into the reagent 2 is from 0.001 mass% to 5 mass%, but the amount of the albumin in the kit of the first embodiment is not limited thereto. A sensitizer for latex agglutination assay may be incorporated into each of both, or one, of the reagent 1 and the reagent 2. Examples of the sensitizer for latex agglutination assay include a polyvinyl alcohol, a polyvinyl pyrrolidone, and alginic acid. However, the sensitizer to be used in the kit of the first embodiment is not limited thereto. In addition, the kit of the first embodiment may include, for example, a positive control, a negative control, or a serum diluent in addition to the reagent 1 and the reagent 2. In addition to serum or physiological saline free of the target substance that may be subjected to the assay, a solvent may be used as a medium for the positive control or the negative control. The kit of the first embodiment may be used in a method of detecting a target substance of the first embodiment as in a typical kit for use in detection of a target substance in a specimen by in vitro diagnosis. In addition, the concentration of the target substance can be measured by a conventionally known method, and the method is particularly suitable for the detection of the target substance in the specimen by a latex agglutination method.

The method of detecting a target substance in a specimen by in vitro diagnosis of the first embodiment is characterized by including mixing the affinity particle of the first embodiment and the specimen that may contain the target substance. In addition, the affinity particle of the first embodiment and the specimen are preferably mixed at a pH in the range of from 3.0 to 11.0. In addition, a mixing temperature falls within the range of from 20°C to 50°C, and a mixing time falls within the range of from 1 minute to 20 minutes. In addition, in this detection method, a solvent is preferably used. In addition, the concentration of the affinity particle of the first embodiment in the detection method of the first embodiment is preferably from 0.001 mass% to 5 mass%, more preferably from 0.01 mass% to 1 mass% in a reaction system. The detection method of the first embodiment is characterized by including optically detecting interparticle agglutination caused as a result of the mixing of the affinity particle of the first embodiment and the specimen. When the interparticle agglutination is optically detected, the target substance in the specimen is detected, and the concentration of the target substance can be measured. As a method of optically detecting the agglutination reaction, an optical instrument that can detect a scattered light intensity, a transmitted light intensity, an absorbance, and the like only needs to be used to measure the variations of these values.

A method of measuring the dry particle diameter of the particle in the first embodiment is described. The dry particle diameter in the first embodiment is a number-average particle diameter, and is measured under a state in which the particles are sufficiently dried. Specifically, the particles are dispersed at a concentration of 5 mass% in ion-exchanged water, and the dispersion is dropped onto aluminum foil, followed by drying at 25°C for 48 hours. After that, the dried product is further dried with a vacuum dryer for 24 hours, and then the measurement is performed. A scanning electron microscope and an image processing analyzer are used in the measurement of the dry particle diameter. Specifically, 100 individual particles are randomly sampled from an image of the particles in a dry state, the image being obtained with a scanning electron microscope (S-4800: Hitachi High-Technologies Corporation), and their number-average particle diameter is calculated by analyzing the image with an image processing analyzer "Luzex AP" (Nireco Corporation).

A method of measuring the particle diameter in water of the particle in the first embodiment is described. The particle diameter in water in the first embodiment is a number-average particle diameter, and is measured under a state in which the particles are dispersed in ion-exchanged water so that their concentration may be 0.001 mass%. Ion-exchanged water having an electrical conductivity of 10 $\mu$S/cm or less is used as the ion-exchanged water. A dynamic light scattering method is applied to the measurement of the particle diameter in water. Specifically, the measurement is performed with ZETASIZER (Nano-ZS: Spectris Co., Ltd.) at 25°C. With regard to analysis parameters, the refractive index of latex ($n \approx 1.59$) is selected as the refractive index of the particle, and pure water is selected as a solvent. The measurement is performed ten times, and the average of the ten measured values is adopted as the particle diameter in water.

A method of measuring the zeta potential of the particle in the first embodiment is described. The zeta potential in

the first embodiment is measured under a state in which the particle is dispersed in a 0.01 N aqueous solution of potassium having a pH of 7.8 so that its concentration may be 0.001 mass%. A solution obtained by appropriately mixing a 0.01 N aqueous solution of potassium chloride, a 0.01 N aqueous solution of potassium hydroxide, and a 0.01 N aqueous solution of hydrochloric acid, each of which has been prepared by using ion-exchanged water having an electrical conductivity of 10 μS/cm or less, is used as the 0.01 N aqueous solution of potassium having a pH of 7.8. The measurement is performed by using ZETASIZER (Nano-ZS: Spectris Co., Ltd.) as a measuring apparatus at 25°C. With regard to analysis parameters, the refractive index of latex (n≈1.59) is selected as the refractive index of the particle, and pure water is selected as a solvent. The measurement is performed ten times, and the average of the ten measured values is adopted as the zeta potential.

The carboxy group density in the first embodiment is calculated from a relationship between the carboxy group amount of the particle and the volume of the swollen layer to be formed on the surface of the particle when the particle is dispersed in ion-exchanged water.

First, a method of measuring the carboxy group amount of the particle is described. The carboxy group of the particle is turned into an active ester with N-hydroxysuccinimide (NHS) through use of 1-ethyl-3-(3-dimethylaminopropyl) carbodiimide hydrochloride (EDC/HCl) as a catalyst. After that, the active ester is caused to react with aminoethanol to liberate NHS, and a carboxy group amount per unit particle mass is calculated by determining the amount of the liberated NHS with a high-performance liquid chromatograph apparatus. The unit of the carboxy group amount per unit particle mass is nmol/mg. A specific method is described below.

<Active Esterification>

An aqueous dispersion of the particles containing 2.5 mg of the particles is added to a 1.5 mL microtube, and the particles are separated from the dispersion with a centrifugal separator. Further, the particles are redispersed in dimethylformamide (DMF). The foregoing operation is performed three times. Further, DMF is removed from the microtube under a state in which the particles are sedimented in the microtube with the centrifugal separator. Next, 400 μL of DMF, 19.2 mg of EDC·HCl, and 100 μL of a 1 mol/L solution of N-hydroxysuccinimide in DMF are added to the microtube, and the mixture is shaken at 25°C for 2 hours to turn the carboxy groups of the particles into active esters.

<Liberation of NHS>

To remove excess NHS from the DMF dispersion of the active esterified particles, the active esterified particles are separated from the dispersion with a centrifugal separator, and the particles are redispersed in DMF; the foregoing operation is performed three times. Further, DMF is removed from the microtube under a state in which the active esterified particles are sedimented in the microtube with the centrifugal separator. Next, 500 μL of a 1 mol/L solution of aminoethanol in DMF is added to the microtube, and the mixture is shaken at 25°C for 2 hours to liberate NHS from the particles.

<Determination of Amount of NHS>

The DMF dispersion of the particles containing the liberated NHS is centrifuged, and a DMF solution containing the liberated NHS is recovered under a state in which the particles are sedimented in the microtube, followed by the determination of the amount of the liberated NHS in the DMF solution with a high-performance liquid chromatograph apparatus. The carboxy group amount per unit particle mass is calculated by using the determined value.

An instrument and reagents to be used in the determination of the carboxy group amount are as described below.

<Instrument>

High-performance liquid chromatograph apparatus: LC20A-FL (Shimadzu Corporation)
Column: Kinetex 5 μm C18 100A LC Column 150×4.6 mm (Phenomenex)
Eluent: 4 mmol/L aqueous solution of ammonium acetate
Flow rate: 1.0 mL/min
Oven temperature: 40°C
Sample injection amount: 1 μL

At the time of the determination of the amount of the liberated NHS incorporated into the DMF solution, a calibration curve between a peak area derived from NHS and the amount of NHS is produced in the range of from 0.1 mmol/L to 10 mmol/L, and the amount of the liberated NHS is determined from the NHS peak area.

<Reagents>

EDC/HCl (Dojindo Laboratories)
NHS (Kishida Chemical Co., Ltd.)
Aminoethanol (Tokyo Chemical Industry Co., Ltd.)
DMF (FUJIFILM Wako Pure Chemical Corporation)
Ammonium acetate (Tokyo Chemical Industry Co., Ltd.)

Next, a method of calculating the carboxy group density of the particle is described. At the time of the calculation of the amount of carboxy groups per unit volume to be incorporated into the swollen layer to be formed on the surface layer of the particle when the particle is dispersed in water or an aqueous solution, that is, the carboxy group density, the values of a particle diameter in water at the time of the dispersion of the particle in ion-exchanged water and the dry particle diameter of the particle are used. In addition, the carboxy group density is calculated by applying the formula (1-8) while regarding the density of the particle as 1 (g/cm$^3$) in the calculation of each of the dry particle diameter and the particle diameter in water.

$$[\text{Carboxy group density (group/nm}^3)] = Dd^3/(Dw^3 - Dd^3) \times A \times N_A \times 10^{-27} \cdots \text{Formula (1-8)}$$

In the formula (1-8), Dw represents the particle diameter in water (nm) of the particle, Dd represents the dry particle diameter (nm) of the particle, A represents the carboxy group amount (nmol/mg) per unit particle mass, and $N_A$ represents the Avogadro constant.

[Second Embodiment]

A second embodiment of the present invention is described in detail below, but the technical scope of the present invention is not limited to the embodiment.

The second embodiment relates to an affinity particle including: a particle; and a ligand on the surface of the particle, the affinity particle being characterized in that the ratio of an area occupied by the ligand to the surface of the particle satisfies a relationship of the formula (2-1), that the zeta potentials of the particle and the ligand satisfy a relationship of the formula (2-2), and that the particle has a repeating unit A represented by the formula (2-3).

$$10 \leq [\text{Occupied area ratio (\%)}] \leq 40 \cdots \text{Formula (2-1)}$$

$$0 \leq [\,||\text{Zeta potential of particle}| - |\text{zeta potential of ligand}||\,] \leq 20 \cdots \text{Formula (2-2)}$$

[Chem. 5]

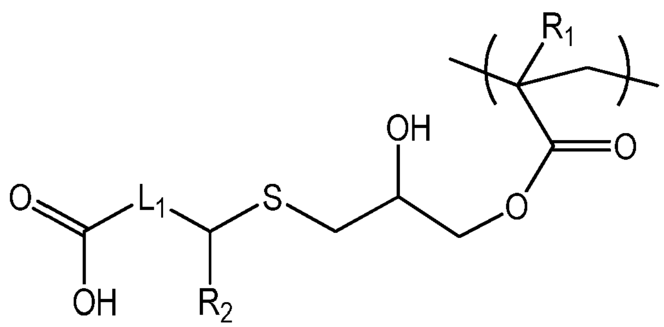

(2-3)

In the formula (2-3), $R_1$ represents a methyl group or a hydrogen atom, $R_2$ represents a carboxy group or a hydrogen atom, and $L_1$ represents an alkylene group having 1 to 15 carbon atoms that may have a substituent, or an oxyalkylene group having 1 to 15 carbon atoms that may have a substituent.

First, the particle is described. In the second embodiment, the particle for obtaining the affinity particle by bonding the ligand thereto is simply referred to as "particle".

The particle has the repeating unit A represented by the formula (2-3).

A hydroxy group adjacent to an ester bond in the formula (2-3) serves to reduce the nonspecific adsorption of the affinity particle according to the second embodiment.

In the formula (2-3), $L_1$ preferably represents a methylene group having 1 carbon atom. The number of the carbon atoms of $L_1$ correlates with the hydrophilicity or hydrophobicity of the repeating unit A, and when the number of the carbon atoms of $L_1$ becomes excessively large, the nonspecific adsorption of a target substance to the affinity particle may be accelerated.

It is preferred that the surface layer of the particle contain a copolymer having the repeating unit A, and when the particle is dispersed in water or an aqueous solution, the surface layer be hydrated to form a swollen layer. The thickness of the swollen layer correlates with a ratio between the dry particle diameter of the particle to be measured when the particle is dried and the particle diameter in water of the particle to be measured when the particle is dispersed in ion-exchanged water, and the dry particle diameter and the particle diameter in water preferably satisfy the formula (2-5).

$$1.10 \leq [\text{Particle diameter in water/dry particle diameter}] \leq 1.40 \cdots \text{Formula (2-5)}$$

…

The formation of the swollen layer by the hydration of the surface layer of the particle in the water or the aqueous solution contributes to the following. That is, the formation contributes to a reduction in nonspecific adsorption of the target substance to the affinity particle, an improvement in sensitivity when the affinity particle is applied to a particle for an agglutination method (e.g., a latex agglutination method), and an improvement in dispersion stability of the affinity particle.

In particular, the fact that the ratio [particle diameter in water/dry particle diameter] is 1.10 or more means that the swollen layer is sufficiently hydrous to make the surface of the particle hydrophilic, and hence the nonspecific adsorption of the target substance to the affinity particle is significantly suppressed. In addition, the fact that the ratio [particle diameter in water/dry particle diameter] is 1.10 or more means that the swollen layer is sufficiently hydrous to improve the mobility of the ligand of the affinity particle, and hence reactivity between the ligand and the target substance is improved. In addition, when the swollen layer is sufficiently hydrous, dispersion stability based on an excluded volume effect is imparted to the affinity particle.

In contrast, when the ratio [particle diameter in water/dry particle diameter] is more than 1.40, the swollen layer has a large water-binding force, and hence the application of the affinity particle of the second embodiment to the latex agglutination method may cause osmotic pressure agglutination. It is difficult to distinguish the osmotic pressure agglutination from an artificial nonspecific agglutination phenomenon in a test institute.

As described above, a preferred range exists for the thickness of the swollen layer from the viewpoints of the nonspecific adsorption, the sensitivity when the affinity particle is applied to the latex agglutination method, the dispersion stability of the affinity particle, and the osmotic pressure agglutination. In view of the foregoing, the ratio [particle diameter in water/dry particle diameter] is preferably 1.10 or more and 1.40 or less, more preferably 1.15 or more and 1.30 or less.

When the surface layer of the particle is hydrated to form the swollen layer in the water or the aqueous solution, the sulfide bond of the repeating unit A represented by the formula (2-3) is a chemical structure showing a weak hydrophobic tendency, and hence moderately weakens the water-binding force of the swollen layer. Accordingly, a suppressing action on osmotic pressure agglutination that may occur when the affinity particle according to the second embodiment is mixed with a specimen is expected.

$R_2$ in the formula (2-3) preferably represents a carboxy group. When $R_2$ represents a carboxy group, the surface layer of the particle is easily hydrated with the water or the aqueous solution, and the easy hydration is advantageous for the formation of the swollen layer.

Next, the ligand is described. The ligand is a compound that is specifically bonded to the receptor of a specific target substance. The site at which the ligand is bonded to the target substance is decided, and the ligand has a selectively or specifically high affinity for the target substance. Examples of the ligand include: an antigen and an antibody; an enzyme protein and a substrate thereof; a signal substance typified by a hormone or a neurotransmitter, and a receptor thereof; a nucleic acid; and avidin and biotin. However, the ligand is not limited thereto to the extent that the object of the second embodiment can be achieved. Specific examples of the ligand include an antigen, an antibody, an antigen-binding fragment (e.g., Fab, F(ab')2, F(ab'), Fv, or scFv), a naturally occurring nucleic acid, an artificial nucleic acid, an aptamer, a peptide aptamer, an oligopeptide, an enzyme, and a coenzyme.

Although a method of bonding the particle and the ligand to each other is not limited to the extent that the object of the second embodiment can be achieved, the ligand is preferably bonded to the surface of the particle by using a chemical reaction through a carboxy group derived from the repeating unit A represented by the formula (2-3).

In the second embodiment, a conventionally known method may be applied as a method for the chemical reaction by which the carboxy group derived from the repeating unit A and the ligand are bonded to each other to the extent that the object of the second embodiment can be achieved. For example, a carbodiimide-mediated reaction or an NHS ester

activation reaction is a suitable example. In addition, the following may be performed: avidin is bonded to the carboxy group, and a biotin-modified ligand is bonded to the resultant.

When the carboxy group derived from the repeating unit A and the ligand are bonded to each other, part of the carboxy group may be transformed into another chemical structure by using a chemical reaction. In the second embodiment, such transformation reaction is represented as "masking treatment," and a reagent to be used in the masking treatment is represented as "masking agent." Amines are each often used as the masking agent, and trishydroxymethylaminomethane out of the amines is generally used. In contrast, as a result of an investigation by the inventors of the present invention, it has been recognized that the following largely improves detection sensitivity when the affinity particle according to the second embodiment is applied to the latex agglutination method. That is, the carboxy group of part of the repeating unit A is transformed by using ethanolamine as the masking agent to provide a chemical structure represented by the formula (2-6).

[Chem. 6]

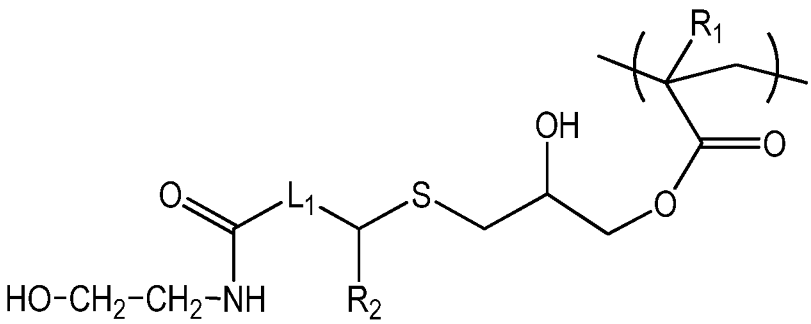

(2-6)

In the formula (2-6), $R_1$ represents a methyl group or a hydrogen atom, $R_2$ represents a carboxy group or a hydrogen atom, and $L_1$ represents an alkylene group having 1 to 15 carbon atoms that may have a substituent, or an oxyalkylene group having 1 to 15 carbon atoms that may have a substituent.

In the second embodiment, when an antibody (antigen) is used as the ligand and an antigen (antibody) is used as the target substance, a latex agglutination method in an immunological test may be extremely preferably applied. The immunological test has been widely utilized as a method of detecting a target substance in a specimen in in vitro diagnosis in fields such as a clinical test and biochemical research. When a general affinity particle is used as a particle for a latex agglutination method, the antigen (antibody) that is the target substance, foreign matter in serum or plasma, or the like nonspecifically adsorbs to the surface of the particle. In addition, concern is raised in that unintended agglutination between the affinity particles occurs owing to the adsorption to impair the accuracy of the immunological test.

The relationship between the zeta potential of the particle and the zeta potential of the ligand in the second embodiment is described. The affinity particle according to the second embodiment is characterized in that the zeta potential of the particle and the zeta potential of the ligand satisfy the relationship of the formula (2-2).

$$0 \leq [\,||\text{Zeta potential (mV) of particle}| - |\text{zeta potential (mV) of ligand}||\,] \leq 20 \cdots \text{Formula (2-2)}$$

A case in which the difference [||zeta potential (mV) of particle|-|zeta potential (mV) of ligand||] is larger than 20 mV means that charges having different signs are imparted to the surfaces of the affinity particles each obtained by bonding the ligand to the particle. In this case, when the affinity particles are left at rest and stored, there is a high risk in that their electrostatic heteroagglutination occurs, and hence attention needs to be paid to the handling of the affinity particles in a test institute. In the second embodiment, when an antibody or an antigen is used as the ligand, the zeta potential of a general antigen or antibody is around -10 mV, and hence the zeta potential of the particle is preferably -30 mV or more and -10 mV or less.

The affinity particle according to the second embodiment is characterized in that the ratio of the area occupied by the ligand to the surface of the particle satisfies the relationship of the formula (2-1).

$$10 \leq [\text{Occupied area ratio (\%)}] \leq 40 \cdots \text{Formula (2-1)}$$

The occupied area ratio is described. An area occupied by one ligand is calculated by using a known formula in which the size of the ligand is approximated to a true sphere and the area of a circle having the same diameter as that of the sphere is calculated. Meanwhile, a surface area per one particle is calculated by using a known formula in which the

particle is approximated to a true sphere and the surface area of the true sphere is calculated. The particle diameter in water of the particle is used as the particle diameter thereof. Meanwhile, the amount (μg/mg) of the ligand sensitized to the particle is determined from an experiment, and the number of the ligands per one affinity particle is calculated from the determined value. The occupied area ratio is calculated by applying a monomolecular layer adsorption model assuming that the affinity particle of the second embodiment is obtained by bonding a single layer of the ligand to the surface of the particle. That is, the occupied area ratio is defined as a value calculated by using the formula (2-7).

[Occupied area ratio (%)]=[area ($nm^2$) occupied by one ligand]×[number (ligands) of ligands per one affinity particle]/[surface area ($nm^2$) per one particle]×100 Formula (2-7)

When the ligand is an antibody, a general antibody is considered to be a true sphere having a diameter of 8 nm, and hence an area occupied by one antibody is determined to be about 50.2 $nm^2$. An occupied area ratio of 50% means that 50% of the surface of the affinity particle is occupied by the antibody, and the surface of the particle serving as a ground corresponding to the remaining 50% is exposed.

When the occupied area ratio is less than 10%, at the time of the application of the affinity particles as particles for a latex agglutination method, sufficient test sensitivity may not be obtained because the frequency at which the affinity particles agglutinate with each other through the target substance is small. In addition, when the occupied area ratio of the ligand is excessively small, concern is raised in that the nonuniformity of chemical composition occurs on the surface of the affinity particle to impair the dispersion stability of the affinity particle. Meanwhile, when the occupied area ratio is more than 40%, an interaction may occur between the ligands to impair the intrinsic reactivity of the ligand with the target substance. In the second embodiment, the occupied area ratio more preferably falls within the range of from 15% or more to less than 30%.

A more preferred form of the particle is described.

The particle preferably has a repeating unit B characterized by having a hydroxy group at a terminal of a side chain thereof in addition to having the repeating unit A represented by the formula (2-3). An affinity particle obtained by bonding the ligand to the particle having such feature is significantly suppressed from causing nonspecific adsorption. For the purpose of reducing the nonspecific adsorption, it is more preferred that the surface layer of the particle contain a copolymer having the repeating unit A and the repeating unit B, and when the particle is dispersed in water or an aqueous solution, the surface layer be hydrated to form a swollen layer.

A specific chemical structure of the repeating unit B is described. The repeating unit B is characterized by having the hydroxy group at the terminal of the side chain thereof, and its chemical structure is not limited to the extent that the object of the second embodiment can be achieved. However, a repeating unit having a side chain structure represented by the formula (2-4) is more preferred.

[Chem. 7]

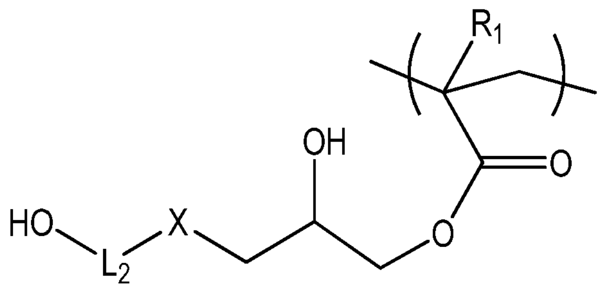

(2-4)

In the formula (2-4), $R_1$ represents a methyl group or a hydrogen atom, $L_2$ represents an alkylene group having 2 to 15 carbon atoms that may have a substituent, or an oxyalkylene group having 2 to 15 carbon atoms that may have a substituent, X represents a sulfur atom or a nitrogen atom that may have a substituent, and $L_1$ in the formula (2-3) and $L_2$ in the formula (2-4) satisfy a relationship of [number of carbon atoms of $L_1$]+2≥[number of carbon atoms of $L_2$].

A hydroxy group adjacent to an ester bond in the formula (2-4) and the hydroxy group at the terminal of the side chain serve to reduce the nonspecific adsorption of the particle. X in the formula (2-4), which may represent any one of a sulfur atom and a nitrogen atom, more preferably represents a sulfur atom. A sulfide bond is a structure showing a weak hydrophobic tendency. Accordingly, when the swollen layer formed by the hydration of the surface layer of the particle is formed, the water-binding force of the swollen layer is moderately weakened, and hence a suppressing action on osmotic pressure agglutination that may occur when the particle is mixed with a high-concentration specimen is expected.

When the formula (2-3) and the formula (2-4) are compared, a relationship between the number of the carbon

atoms for forming $L_1$ in the formula (2-3) and the number of the carbon atoms for forming $L_2$ in the formula (2-4) preferably satisfies the relationship of the formula (2-8). When the relationship of the formula (2-8) is not satisfied, a side chain derived from the repeating unit B becomes relatively longer than a side chain derived from the repeating unit A, and hence the carboxy group that the repeating unit A has at a terminal of a side chain thereof may be blocked. In view of the foregoing, when $L_1$ in the formula (2-3) represents an alkylene group having 1 carbon atom, $L_2$ in the formula (2-4) preferably represents an alkylene group having 2 carbon atoms or 3 carbon atoms.

$$[\text{Number of carbon atoms of } L_1]+2 \geq [\text{number of carbon atoms of } L_2] \cdots \text{Formula (2-8)}$$

When $L_2$ in the formula (2-4) represents an alkylene group having 3 carbon atoms, a chemical structure in which one hydrogen atom of the alkylene group is substituted with a hydroxy group is more preferred from the viewpoint of reducing the nonspecific adsorption of the particle.

The particle preferably has, as a repeating unit C, at least one kind selected from the group consisting of styrenes and (meth)acrylates, and more preferably contains a repeating unit derived from any one of the styrenes and glycidyl (meth)acrylate. Herein, the "(meth)acrylate" refers to "acrylate or methacrylate." As described in the "Background Art" section herein, a repeating unit derived from glycidyl (meth)acrylate has a reducing action on the nonspecific adsorption of the particle. Meanwhile, the reason why the particle preferably contains a repeating unit derived from any one of the styrenes is as described below. When the particle is purified by a method such as centrifugal separation or ultrafiltration, the presence of the repeating unit derived from any one of the styrenes, which has a high glass transition temperature and is excellent in mechanical strength, contributes to the suppression of damage to the particle, such as cracking or chipping. Examples of the styrenes include styrene, α-methylstyrene, β-methylstyrene, o-methylstyrene, m-methylstyrene, p-methylstyrene, 2,4-dimethylstyrene, p-n-butylstyrene, p-tert-butylstyrene, p-n-hexylstyrene, p-n-octylstyrene, p-n-nonylstyrene, p-n-decylstyrene, p-n-dodecylstyrene, p-methoxystyrene, and p-phenylstyrene. However, the styrenes are not limited thereto to the extent that the object of the second embodiment can be achieved. In addition, the two or more kinds of styrenes may be used in combination.

When the mass of the particle is defined as 100 parts by mass, the content of the styrenes is preferably 10 parts by mass or more and 70 parts by mass or less because sufficient strength can be imparted to the particle while the nonspecific adsorption is reduced.

A repeating unit derived from any one of radical-polymerizable monomers each having crosslinkability may be further incorporated into the particle. Examples of the radical-polymerizable monomers each having crosslinkability include diethylene glycol diacrylate, triethylene glycol diacrylate, tetraethylene glycol diacrylate, polyethylene glycol diacrylate, 1,6-hexanediol diacrylate, neopentyl glycol diacrylate, tripropylene glycol diacrylate, polypropylene glycol diacrylate, 2,2'-bis(4-(acryloxydiethoxy)phenyl)propane, trimethylolpropane triacrylate, tetramethylolmethane tetraacrylate, ethylene glycol dimethacrylate, diethylene glycol dimethacrylate, triethylene glycol dimethacrylate, tetraethylene glycol dimethacrylate, polyethylene glycol dimethacrylate, 1,3-butylene glycol dimethacrylate, 1,6-hexanediol dimethacrylate, neopentyl glycol dimethacrylate, polypropylene glycol dimethacrylate, 2,2'-bis(4-(methacryloxydiethoxy)phenyl) propane, 2,2'-bis(4-(methacryloxypolyethoxy)phenyl)propane, trimethylolpropane trimethacrylate, tetramethylolmethane tetramethacrylate, divinylbenzene, divinylnaphthalene, and divinyl ether. However, the monomers are not limited thereto to the extent that the object of the second embodiment can be achieved. In addition, the two or more kinds of radical-polymerizable monomers each having crosslinkability may be used in combination.

The particle diameter of the particle is preferably 0.05 μm or more and 1.00 μm or less, more preferably 0.05 μm or more and 0.50 μm or less, still more preferably 0.05 μm or more and 0.30 μm or less in terms of number-average particle diameter. When the particle diameter is 0.05 μm or more and 0.30 μm or less, the particle is easy to handle, and in the case of long-term storage of the particle as a dispersion, the sedimentation of the particle hardly occurs.

A typical method of producing the particle is described. However, the method of producing the particle is not limited to the extent that the object of the second embodiment can be achieved.

The method of producing the particle includes a step 1 of mixing glycidyl (meth)acrylate, styrene, divinylbenzene, water, and a radical polymerization initiator to form a particulate copolymer, thereby providing an aqueous dispersion of the particulate copolymer.

In addition, the method of producing the particle includes a step 2 of forming the particle through the following step. That is, first, the aqueous dispersion, 3-mercapto-1,2-propanediol, and mercaptosuccinic acid are mixed to prepare a mixed liquid. Subsequently, an epoxy group derived from glycidyl (meth)acrylate of the particulate copolymer, and a thiol group derived from each of 3-mercapto-1,2-propanediol and mercaptosuccinic acid are caused to react with each other.

The radical polymerization initiator is preferably at least one of 4,4'-azobis(4-cyanovaleric acid), 2,2'-azobis[2-methyl-N-(2-hydroxyethyl)propionamide], 2,2'-azobis[N-(2-carboxyethyl)-2-methylpropionamidine] tetrahydrate, 2,2'-azobis(2-methylpropionamidine) dihydrochloride, 2,2'-azobis[2-(2-imidazolin-2-yl)propane], 2,2'-azobis[2-(2-imidazolin-2-yl)propane] dihydrochloride, or 2,2'-azobis[2-(2-imidazolin-2-yl)propane] disulfate dihydrate.

In addition, the method of producing the particle includes a step of adjusting the pH of the mixed liquid of the step 2 within an alkaline region with an organic base free of any primary amine. In the step 2, the epoxy group derived from glycidyl (meth)acrylate of the particulate copolymer is caused to react with the thiol group derived from each of 3-mercapto-1,2-propanediol and mercaptosuccinic acid from the surface of the particulate copolymer in its depth direction. To form a surface layer having a sufficient thickness on the particle, triethylamine having permeability into the particulate copolymer is preferably selected as the organic base.

A method of forming the particulate copolymer is not limited to the use of radical polymerization to the extent that the object of the second embodiment can be achieved.

When the radical polymerization is applied, emulsion polymerization, soap-free emulsion polymerization, or suspension polymerization is preferably used, and the emulsion polymerization or the soap-free emulsion polymerization is more preferably used. The soap-free emulsion polymerization is still more preferably used.

In general, the emulsion polymerization and the soap-free emulsion polymerization can each provide a particulate copolymer having a particle diameter distribution sharper than that of a particulate copolymer provided by the suspension polymerization. In addition, when the ligand is bonded to the particle to provide the affinity particle, concern is raised about the modification of the ligand by the presence of an anionic surfactant or a cationic surfactant to be generally used in the emulsion polymerization as a residue. In view of the foregoing, when the particulate copolymer is formed by the emulsion polymerization, a nonionic surfactant is preferably used.

A reagent for use in detection of a target substance in a specimen by in vitro diagnosis according to the second embodiment is characterized by including the affinity particle according to the second embodiment as a particle for a latex agglutination method.

The amount of the particle for a latex agglutination method to be incorporated into the reagent is preferably 0.001 mass% or more and 20 mass% or less, more preferably 0.01 mass% or more and 10 mass% or less.

The reagent according to the second embodiment may include a third substance, such as a solvent or a blocking agent, in addition to the affinity particle according to the second embodiment the extent that the object of the second embodiment can be achieved.

Examples of the solvent to be used in the second embodiment include various aqueous buffers, such as a phosphate buffer, a glycine buffer, a Good's buffer, a Tris buffer, a HEPES buffer, a MES buffer, and an ammonia buffer. However, the solvent to be incorporated into the reagent according to the second embodiment is not limited thereto.

A kit for use in detection of a target substance in a specimen by in vitro diagnosis according to the second embodiment is characterized by including at least the reagent according to the second embodiment.

The kit according to the second embodiment preferably further includes a reaction buffer containing an albumin (hereinafter referred to as "reagent 2") in addition to the reagent according to the second embodiment (hereinafter referred to as "reagent 1"). The albumin is, for example, serum albumin, and may be subjected to a protease treatment. As a guide, the amount of the albumin to be incorporated into the reagent 2 is 0.001 mass% or more and 5 mass% or less, but the amount of the albumin in the kit according to the second embodiment is not limited thereto.

A sensitizer for latex agglutination assay may be incorporated into each of both, or one, of the reagent 1 and the reagent 2. Examples of the sensitizer for latex agglutination assay include a polyvinyl alcohol, a polyvinyl pyrrolidone, and alginic acid. However, the sensitizer to be used in the kit according to the second embodiment is not limited thereto.

In addition, the kit according to the second embodiment may include, for example, a positive control, a negative control, or a serum diluent in addition to the reagent 1 and the reagent 2. In addition to serum or physiological saline free of the target substance that may be subjected to the assay, a solvent may be used as a medium for the positive control or the negative control.

The kit according to the second embodiment may be used in a method of detecting a target substance according to the second embodiment as in a typical kit for use in detection of a target substance in a specimen by in vitro diagnosis. In addition, the concentration of the target substance can be measured by a conventionally known method, and the method is particularly suitable for the detection of the target substance in the specimen by a latex agglutination method.

The method of detecting a target substance in a specimen by in vitro diagnosis according to the second embodiment is characterized by including mixing the affinity particle according to the second embodiment and the specimen that may contain the target substance.

The affinity particle according to the second embodiment and the specimen are preferably mixed at a pH in the range of from 3.0 to 11.0. In addition, a mixing temperature falls within the range of from 20°C to 50°C, and a mixing time falls within the range of from 1 minute to 20 minutes. In addition, in this detection method, a solvent is preferably used. In addition, the concentration of the affinity particle according to the second embodiment in the detection method according to the second embodiment is preferably 0.001 mass% or more and 5 mass% or less, more preferably 0.01 mass% or more and 1 mass% or less in a reaction system. The detection method according to the second embodiment is characterized by including optically detecting interparticle agglutination caused as a result of the mixing of the affinity particle according to the second embodiment and the specimen. When the interparticle agglutination is optically detected, the target substance

in the specimen is detected, and the concentration of the target substance can be measured. As a method of optically detecting the agglutination reaction, an optical instrument that can detect a scattered light intensity, a transmitted light intensity, an absorbance, and the like only needs to be used to measure the variations of these values.

A method of measuring the dry particle diameter of the particle in the second embodiment is described. The dry particle diameter in the second embodiment is a number-average particle diameter, and is measured under a state in which the particles are sufficiently dried. Specifically, the particles are dispersed at a concentration of 5 mass% in ion-exchanged water, and the dispersion is dropped onto aluminum foil, followed by drying at 25°C for 48 hours. After that, the dried product is further dried with a vacuum dryer for 24 hours, and then the measurement is performed. A scanning electron microscope and an image processing analyzer are used in the measurement of the dry particle diameter. Specifically, for example, 100 individual particles are randomly sampled from an image of the particles in a dry state, the image being obtained with a scanning electron microscope (product name: S-4800, manufactured by Hitachi High-Technologies Corporation), and their number-average particle diameter is calculated by analyzing the image with an image processing analyzer (product name: Luzex AP, manufactured by Nireco Corporation).

A method of measuring the particle diameter in water of the particle in the second embodiment is described. The particle diameter in water in the second embodiment is a number-average particle diameter, and is measured under a state in which the particles are dispersed in ion-exchanged water so that their concentration may be 0.001 mass%. Ion-exchanged water having an electrical conductivity of 10 μS/cm or less is used as the ion-exchanged water. A dynamic light scattering method is applied to the measurement of the particle diameter in water. Specifically, the measurement is performed with ZETASIZER (product name: Nano-ZS, manufactured by Spectris Co., Ltd.) at 25°C. With regard to analysis parameters, the refractive index of latex (n≈1.59) is selected as the refractive index of the particle, and pure water is selected as a solvent. The measurement is performed ten times, and the average of the ten measured values is adopted as the particle diameter in water.

A method of measuring the particle diameter in water of the affinity particle in the second embodiment is described. The particle diameter in water in the second embodiment is a number-average particle diameter, and is measured under a state in which the affinity particles are dispersed in a 0.01 N aqueous solution of potassium having a pH of 7.8 so that their concentration may be 0.001 mass%. A solution obtained by appropriately mixing a 0.01 N aqueous solution of potassium chloride, a 0.01 N aqueous solution of potassium hydroxide, and a 0.01 N aqueous solution of hydrochloric acid, each of which has been prepared by using ion-exchanged water having an electrical conductivity of 10 μS/cm or less, is used as the 0.01 N aqueous solution of potassium having a pH of 7.8. A dynamic light scattering method is applied to the measurement of the particle diameter in water. Specifically, the measurement is performed with ZETASIZER (product name: Nano-ZS, manufactured by Spectris Co., Ltd.) at 25°C. With regard to analysis parameters, the refractive index of latex (n≈1.59) is selected as the refractive index of the affinity particle, and pure water is selected as a solvent. The measurement is performed ten times, and the average of the ten measured values is adopted as the particle diameter in water.

A method of measuring the zeta potential of the particle or the affinity particle in the second embodiment is described. The zeta potential in the second embodiment is measured under a state in which the particle is dispersed in a 0.01 N aqueous solution of potassium having a pH of 7.8 so that its concentration may be 0.001 mass%. A solution obtained by appropriately mixing a 0.01 N aqueous solution of potassium chloride, a 0.01 N aqueous solution of potassium hydroxide, and a 0.01 N aqueous solution of hydrochloric acid, each of which has been prepared by using ion-exchanged water having an electrical conductivity of 10 μS/cm or less, is used as the 0.01 N aqueous solution of potassium having a pH of 7.8. The measurement is performed by using ZETASIZER (product name: Nano-ZS, manufactured by Spectris Co., Ltd.) as a measuring apparatus at 25°C. With regard to analysis parameters, the refractive index of latex (n≈1.59) is selected as the refractive index of the particle, and pure water is selected as a solvent. The measurement is performed ten times, and the average of the ten measured values is adopted as the zeta potential.

A method of measuring the zeta potential of an antibody in the second embodiment is described. The zeta potential of the antibody in the second embodiment is measured under a state in which an antibody solution is diluted with a 0.01 N aqueous solution of potassium having a pH of 7.8 whose amount is at least ten times as large as that of the solution to have a protein concentration of from 0.5 mg/mL to 2.0 mg/mL. A solution obtained by appropriately mixing a 0.01 N aqueous solution of potassium chloride, a 0.01 N aqueous solution of potassium hydroxide, and a 0.01 N aqueous solution of hydrochloric acid, each of which has been prepared by using ion-exchanged water having an electrical conductivity of 10 μS/cm or less, is used as the 0.01 N aqueous solution of potassium having a pH of 7.8. The measurement is performed by using ZETASIZER (product name: Nano-ZS, manufactured by Spectris Co., Ltd.) as a measuring apparatus at 25°C. With regard to analysis parameters, the refractive index of a protein (n≈1.4) is selected as the refractive index of the antibody, and pure water is selected as a solvent. The measurement is performed ten times, and the average of the ten measured values is adopted as the zeta potential.

[Third Embodiment]

A third embodiment of the present invention is described in detail below, but the technical scope of the present invention is not limited to the embodiment. First, the background art of the third embodiment and a problem to be solved by the embodiment are described.

(Background Art of Third Embodiment and Problem to be solved by the Embodiment)

In recent years, research in which an affinity particle obtained by chemically bonding a ligand having an affinity for a target substance and a particle to each other is used to purify the target substance or to determine its amount has been widely performed. The particle to be used for such purposes is desired to show such a characteristic as to adsorb to a substance except the target substance, that is, so-called nonspecific adsorptivity to a small extent. In, for example, Bioseparation using Affinity Latex (1995), p. 11 to p. 30, there is a disclosure of a resin particle (hereinafter referred to as "SG particle") whose surface is coated with polyglycidyl methacrylate obtained by emulsion polymerization including using both of the following monomers: styrene and glycidyl methacrylate. **In** addition, in Japanese Patent Application Laid-Open No. 2014-193972, there is a disclosure of a method of controlling the particle diameter of the SG particle.

**In** the SG particle, part of epoxy groups derived from the polyglycidyl methacrylate undergo ring opening to provide a glycol, and nonspecific adsorption is suppressed as a result of the hydrophilicity of the glycol. Meanwhile, when a ligand is chemically bonded to the surface of the SG particle, the epoxy groups derived from the polyglycidyl methacrylate may be utilized as they are. **In** normal cases, however, the following method is generally adopted: after a step of transforming each of the epoxy groups into another reactive functional group, such as a carboxy group, an amino group, or a thiol group, has been passed through, the reactive functional group and the ligand are caused to chemically react with each other. A particle obtained by transforming the epoxy groups of the SG particle into carboxy groups out of such groups is a preferred form because the particle has the highest general-purpose property in the chemical bonding of the ligand to the surface of the particle.

In addition, in recent years, an immunological latex agglutination assay method has been attracting attention as a simple and rapid immunological test method. In the method, a dispersion of a particle obtained by chemically bonding an antibody or an antigen as a ligand and a specimen that may contain a target substance (an antigen or an antibody) are mixed. At this time, when the specimen contains the target substance (the antigen or the antibody), the particle causes an agglutination reaction, and hence the presence or absence of a disease can be identified by optically detecting the agglutination reaction as a variation in, for example, scattered light intensity, transmitted light intensity, or absorbance. The particle to be used in the immunological latex agglutination assay method preferably has small nonspecific adsorptivity and a reactive functional group for immobilizing the ligand like the SG particle for the purpose of reducing false positive noise.

The inventors of the present invention have synthesized the SG particle in accordance with Bioseparation using Affinity Latex (1995), p. 11 to p. 30, and have caused an amino acid to chemically react with an epoxy group derived from glycidyl methacrylate of the SG particle to provide a particle in which the epoxy group is transformed into a carboxy group. However, the nonspecific adsorption-suppressing ability of the particle obtained as described above deteriorated as compared to that of the SG particle.

Meanwhile, a method of introducing a carboxy group into the SG particle has been known. A carboxy group-introduced particle is obtained by: treating the SG particle with ammonia water; then causing the treated product to chemically react with ethylene glycol diglycidyl ether; and causing an epoxy group derived from ethylene glycol diglycidyl ether described above and an amino acid to chemically react with each other. Although the particle obtained as described above astonishingly suppresses nonspecific adsorption, the particle is so excellent in dispersion stability that the particles hardly agglutinate with each other, and hence when the particle is used in an immunological latex agglutination method, sufficient sensitivity has not been obtained in some cases. **In** addition, the particle obtained as described above involves a problem in that the particle is not suitable for industrialization because the chemical reactions require many steps, and the steps are complicated.

The third embodiment has been made in view of such background art and problems. An object of the third embodiment is to provide a particle, which causes small nonspecific adsorption, has a reactive functional group for chemically bonding a ligand, and is suitable for a latex agglutination method. Another object of the third embodiment is to provide a particle for a latex agglutination method obtained by chemically bonding a ligand, and an in vitro diagnostic reagent and kit each including the particle, and a method of detecting a target substance.

Specifically, an object of the third embodiment is to provide a particle, which has a nonspecific adsorption-suppressing ability equal to or more excellent than that of the SG particle and has a reactive functional group capable of chemically bonding a ligand to the surface of the particle in high yield. Another object thereof is to provide a novel approach for producing the particle simply and in high yield. Still another object of the third embodiment is to provide an affinity particle obtained by chemically bonding an antigen or an antibody as a ligand, and an in vitro diagnostic reagent and kit

each including the particle, and a method of detecting a target substance.

(With Regard to Effect of Invention according to Third Embodiment)

According to the third embodiment, the particle, which has a nonspecific adsorption-suppressing ability equal to or more excellent than that of the SG particle and has a reactive functional group for chemically bonding a ligand to the surface of the particle in high yield, and the novel approach for producing the particle simply and in high yield can be provided. Further, according to the third embodiment, the affinity particle obtained by chemically bonding the antigen or the antibody as a ligand, the in vitro diagnostic reagent and kit each including the particle, and the method of detecting a target substance can be provided.

Details about the particle of the third embodiment are described below.

The particle according to the third embodiment of the present invention includes a copolymer. A repeating unit A in the copolymer has a reactive functional group for chemically bonding a ligand to a side chain thereof. The repeating unit has a carboxy group as the reactive functional group for chemically bonding the ligand to the side chain. Specifically, the particle according to this embodiment is characterized in that the particle includes the copolymer containing the "repeating unit A," and the repeating unit A is represented by the formula (3-1).

[Chem. 8]

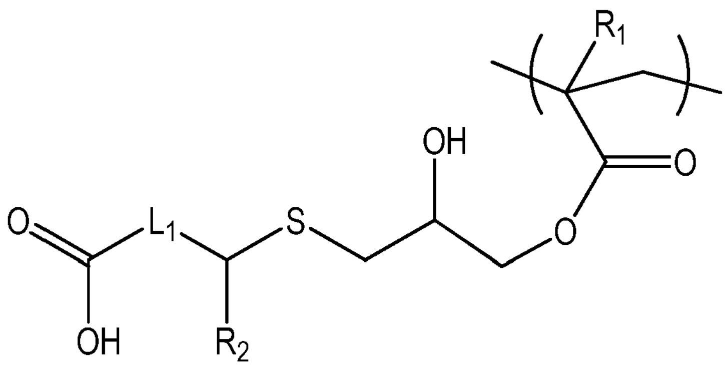

(3-1)

In the formula (3-1), $R_1$ represents a methyl group or a hydrogen atom, $R_2$ represents a carboxy group or a hydrogen atom, and $L_1$ represents an alkylene group having 1 to 15 carbon atoms that may be substituted, or an oxyalkylene group having 1 to 15 carbon atoms that may be substituted.

The "repeating unit A" in the third embodiment has the carboxy group through a sulfide group. The carboxy group is to be chemically bonded to the ligand, and when the particle has the structure of the repeating unit A, the ligand can be chemically bonded to the surface of the particle in high yield. Accordingly, when the particle is used in an immunological latex agglutination assay method, a target substance can be detected with high sensitivity. Although details about the foregoing are unknown, the foregoing is assumed to be because the side chain of the repeating unit is hardly protonated as compared to the case where the repeating unit has the carboxy group through an amino group, and hence the particle is electrostatically stabilized. In addition, when a distance between a carboxylic acid and the sulfide group is appropriately controlled, the target substance can be detected with high sensitivity, though details about a mechanism for the foregoing are unknown.

Further, the particle of the third embodiment has a carboxylic acid amount per unit mass of preferably 5 [nmol/mg] or more, more preferably 100 [nmol/mg] or more. As a result, a large amount of the ligand can be chemically bonded to the surface of the particle in high yield. Accordingly, when the particle is used in the immunological latex agglutination assay method, the target substance can be detected with high sensitivity. In addition, the carboxylic acid amount per unit mass of the particle of the third embodiment is preferably 300 [nmol/mg] or less. When the carboxylic acid amount per unit mass is controlled within the ranges, the nonspecific adsorption of a substance except the target substance can be suppressed.

In addition, the particle of the third embodiment preferably has a number-average particle diameter of 0.05 μm or more and 1 μm or less. When the number-average particle diameter is controlled within the range, in the case where the particle is used in the immunological latex agglutination assay method, both of the dispersion stability of the particle at the time of non-agglutination and a change in turbidity at the time of agglutination, that is, excellent detection sensitivity can be achieved.

In addition, the particle of the third embodiment may be represented in terms of carboxylic acid amount per unit area, and in that case, preferably has a carboxylic acid amount per unit area of from 0.1 to 20 [molecules/$nm^2$]. The foregoing enables the suppression of the nonspecific adsorption of a substance except the target substance, and the achievement of both of the dispersion stability of the particle at the time of the non-agglutination and a change in turbidity at

the time of the agglutination, that is, excellent detection sensitivity.

In addition, the particle of the third embodiment may include a "repeating unit B" having a hydrophilic structure for suppressing the nonspecific adsorption. A glycol structure obtained by the ring opening of part of epoxy groups derived from polyglycidyl methacrylate, or a structure having a sulfide group or a secondary amine and two hydroxy groups on side chains thereof is preferably used as the "repeating unit B." The presence of those side chains can suppress the nonspecific adsorption of a substance except the target substance.

In addition, the particle of the third embodiment may include a "repeating unit C" having a hydrophobic structure from the viewpoints of particle strength and solvent resistance. Although the chemical structure of the "repeating unit C" is not limited to the extent that the objects of the third embodiment can be achieved, at least one kind selected from the group consisting of styrenes and (meth)acrylates is preferred. A case in which the repeating unit C is derived from styrene or methyl methacrylate, or both of the compounds out of the foregoing compounds is preferred because the repeating unit C has a high glass transition temperature, and hence the particle has sufficient strength. Examples of the styrenes and the (meth)acrylates that may be used in the "repeating unit C" are described below, but the "repeating unit C" is not limited thereto. In addition, the hydrophobic "repeating unit C" may use two or more kinds of oily radical-polymerizable monomers.

Examples of the styrenes include styrene, α-methylstyrene, β-methylstyrene, o-methylstyrene, m-methylstyrene, p-methylstyrene, 2,4-dimethylstyrene, p-n-butylstyrene, p-tert-butylstyrene, p-n-hexylstyrene, p-n-octylstyrene, p-n-nonylstyrene, p-n-decylstyrene, p-n-dodecylstyrene, p-methoxystyrene, and p-phenylstyrene.

Examples of the (meth)acrylates include methyl acrylate, ethyl acrylate, n-propyl acrylate, iso-propyl acrylate, n-butyl acrylate, iso-butyl acrylate, tert-butyl acrylate, n-amyl acrylate, n-hexyl acrylate, 2-ethylhexyl acrylate, n-octyl acrylate, n-nonyl acrylate, cyclohexyl acrylate, benzyl acrylate, dimethyl phosphate ethyl acrylate, diethyl phosphate ethyl acrylate, dibutyl phosphate ethyl acrylate, 2-benzoyloxyethyl acrylate, methyl methacrylate, ethyl methacrylate, n-propyl methacrylate, iso-propyl methacrylate, n-butyl methacrylate, iso-butyl methacrylate, tert-butyl methacrylate, n-amyl methacrylate, n-hexyl methacrylate, 2-ethylhexyl methacrylate, n-octyl methacrylate, n-nonyl methacrylate, diethyl phosphate ethyl methacrylate, and dibutyl phosphate ethyl methacrylate.

In addition, in the third embodiment, a crosslinkable radical-polymerizable monomer may be incorporated for further improving the strength of the particle. The crosslinkable radical-polymerizable monomer is a compound having two or more radical-polymerizable groups in a molecule thereof, and examples thereof may include: radical-polymerizable aromatic compounds each having two or more radical-polymerizable groups and an aromatic ring, for example, aromatic divinyl compounds, such as divinylbenzene (DVB), divinyltoluene, divinylxylene, divinylanthracene, divinylnaphthalene, and divinyldurene, aromatic trivinyl compounds, such as trivinylbenzene, and aromatic tetravinyl compounds, such as tetravinylbenzene; and radical-polymerizable aliphatic compounds each having two or more radical-polymerizable groups and an aliphatic group, such as pentaerythritol tetraacrylate.

In addition, when the hydrophobic repeating unit C is used, a composition ratio among the repeating units A, B, and C is not limited to the extent that the objects of the third embodiment can be achieved.

The particle diameter of the particle of the third embodiment is 0.05 μm or more and 1 μm or less, preferably 0.1 μm or more and 0.5 μm or less, more preferably 0.15 μm or more and 0.3 μm or less in terms of number-average particle diameter in water. When the particle diameter is 0.15 μm or more and 0.3 μm or less, the particle is excellent in handleability in a centrifugal operation, and a large specific surface area that is a feature of the particle becomes conspicuous. The number-average particle diameter of the particle of the third embodiment was evaluated by a dynamic light scattering method.

In addition, the third embodiment relates to an affinity particle obtained by chemically bonding a carboxy group derived from the "repeating unit A" of the particle of the third embodiment and a ligand to each other.

The ligand is a compound that is specifically bonded to a receptor that a specific target substance has. The site at which the ligand is bonded to the target substance is decided, and the ligand has a selectively or specifically high affinity for the target substance. Examples of the ligand include: an antigen and an antibody; an enzyme protein and a substrate thereof; a signal substance, such as a hormone or a neurotransmitter, and a receptor thereof; a nucleic acid; and avidin and biotin. However, the ligand is not limited thereto. Specific examples of the ligand include an antigen, an antibody, an antigen-binding fragment (e.g., Fab, F(ab')2, F(ab'), Fv, or scFv), a naturally occurring nucleic acid, an artificial nucleic acid, an aptamer, a peptide aptamer, an oligopeptide, an enzyme, and a coenzyme. The affinity particle in the third embodiment means a particle having a selectively or specifically high affinity for the target substance.

In the third embodiment, a conventionally known method may be applied as a method for a chemical reaction by which the carboxy group derived from the "repeating unit A" of the particle of the third embodiment and the ligand are chemically bonded to each other to the extent that the objects of the third embodiment can be achieved. For example, a carbodiimide-mediated reaction or an NHS ester activation reaction is a frequently used chemical reaction method. In addition, the following method is available: avidin is bonded to the carboxy group of the particle, and a biotin-modified ligand is bonded to the avidin. However, the method for the chemical reaction by which the carboxy group derived from the "repeating unit A" of the particle of the third embodiment and the ligand are chemically bonded to each other is not limited thereto.

When an antibody (antigen) is used as the ligand and an antigen (antibody) is used as the target substance, the affinity particle of the third embodiment may be preferably applied to an immunological latex agglutination assay method that has been widely utilized in fields such as a clinical test and biochemical research. When a general particle is applied to the immunological latex agglutination assay method, there is a problem in that the antigen (antibody) that is a target substance, foreign matter in serum, or the like nonspecifically adsorbs to the surface of the particle, and unintended particle agglutination resulting from the adsorption is detected to inhibit accurate measurement.

Accordingly, for the purpose of reducing false positive noise or the like, the particle is typically used after having been coated with a biologically derived substance, such as an albumin, as a blocking agent so that the nonspecific adsorption to the surface of the particle may be suppressed. However, the characteristics of such biologically derived substance vary a little from lot to lot, and hence the nonspecific adsorption-suppressing ability of the particle coated with such substance varies from coating treatment to coating treatment. Accordingly, there is a problem in terms of stable supply of particles having the same level of nonspecific adsorption-suppressing ability. In addition, the biologically derived substance with which the surface of the particle has been coated may show hydrophobicity when modified, and is hence not necessarily excellent in nonspecific adsorption-suppressing ability. Biological contamination is also given as a problem.

In Patent Literature 2, there is a disclosure of an affinity particle for use in in vitro diagnosis characterized in that a particle is coated with a polymer having a repeating unit having a sulfenyl group on a side chain thereof, the polymer serving as a blocking agent. However, the polymer having the repeating unit having the sulfenyl group on the side chain thereof is water-soluble and coats the surface of the particle through physical adsorption, and hence essential concern is raised about the liberation of the copolymer by its dilution. In addition, the inventors of the present invention have evaluated the nonspecific adsorption-suppressing abilities of the SG particle obtained by a method described in Bioseparation using Affinity Latex (1995), p. 11 to p. 30 and a particle obtained by causing the polymer to adsorb to a polystyrene particle obtained by a method described in Japanese Patent Application Laid-Open No. 2014-153140 in chyle.

In the particle obtained by the method described in Japanese Patent Application Laid-Open No. 2014-153140, the polymer having the repeating unit having the sulfenyl group on the side chain thereof was obtained by soap-free emulsion polymerization.

As a result, the SG particle was more excellent in nonspecific adsorption-suppressing ability than the particle obtained by the method described in Japanese Patent Application Laid-Open No. 2014-153140 was, though there is a possibility that Bioseparation using Affinity Latex (1995), p. 11 to p. 30 cannot be completely reproduced. Herein, the SG particle is a particle obtained by transforming an epoxy group derived from glycidyl methacrylate into a glycol through heating in an acidic aqueous solution.

A reagent for use in detection of a target substance in a specimen by in vitro diagnosis of the third embodiment is characterized by including the affinity particle of the third embodiment. The amount of the affinity particle of the third embodiment to be incorporated into the reagent of the third embodiment is preferably from 0.001 mass% to 20 mass%, more preferably from 0.01 mass% to 10 mass%. The reagent of the third embodiment may include a third substance, such as a solvent or a blocking agent, in addition to the affinity particle of the third embodiment to the extent that the objects of the third embodiment can be achieved. The reagent may include the two or more kinds of third substances, such as the solvent and the blocking agent, in combination. Examples of the solvent to be used in the third embodiment include various buffers, such as a phosphate buffer, a glycine buffer, a Tris buffer, an ammonia buffer, and Good's buffers of MES (2-morpholinoethanesulfonic acid), ADA (N-(2-acetamido)iminodiacetic acid), PIPES (piperazine-1,4-bis(2-ethanesulfonic acid)), ACES (N-(2-acetamido)-2-aminoethanesulfonic acid), cholamine chloride, BES (N,N-bis(2-hydroxyethyl)-2-aminoethanesulfonic acid), TES (N-tris(hydroxymethyl)methyl-2-aminoethanesulfonic acid), HEPES (2-[4-(2-hydroxyethyl)-1-piperazinyl]ethanesulfonic acid), acetamidoglycine, tricine, glycinamide, and bicine. However, the solvent to be incorporated into the reagent of the third embodiment is not limited thereto.

A kit for use in detection of a target substance in a specimen by in vitro diagnosis of the third embodiment is characterized by including at least the reagent of the third embodiment. The kit of the third embodiment preferably further includes a reaction buffer containing an albumin (hereinafter referred to as "reagent 2") in addition to the reagent of the third embodiment (hereinafter referred to as "reagent 1"). The albumin is, for example, serum albumin, and may be subjected to a protease treatment. As a guide, the amount of the albumin to be incorporated into the reagent 2 is from 0.001 mass% to 5 mass%, but the amount of the albumin in the kit of the third embodiment is not limited thereto. A sensitizer for latex agglutination assay may be incorporated into each of both, or one, of the reagent 1 and the reagent 2. Examples of the sensitizer for latex agglutination assay include a polyvinyl alcohol, a polyvinyl pyrrolidone, and alginic acid. However, the sensitizer to be used in the kit of the third embodiment is not limited thereto. In addition, the kit of the third embodiment may include, for example, a positive control, a negative control, or a serum diluent in addition to the reagent 1 and the reagent 2. In addition to serum or physiological saline free of the target substance that may be subjected to the assay, a solvent may be used as a medium for the positive control or the negative control. The kit of the third embodiment may be used in a method of detecting a target substance of the third embodiment as in a typical kit for use in detection of a target substance in a specimen by in vitro diagnosis. In addition, the concentration of the target substance can be measured by a

conventionally known method, and the method is suitably used in the detection of the target substance in the specimen by an agglutination method, in particular, a latex agglutination method.

The method of detecting a target substance in a specimen by in vitro diagnosis of the third embodiment is characterized by including mixing the affinity particle of the third embodiment and the specimen that may contain the target substance, and may be used in an agglutination method. In addition, the affinity particle of the third embodiment and the specimen are preferably mixed at a pH in the range of from 3.0 to 11.0. In addition, a mixing temperature falls within the range of from 20°C to 50°C, and a mixing time falls within the range of from 1 minute to 20 minutes. In addition, in this detection method, a solvent is preferably used. In addition, the concentration of the affinity particle of the third embodiment in the detection method of the third embodiment is preferably from 0.001 mass% to 5 mass%, more preferably from 0.01 mass% to 1 mass% in a reaction system. The detection method of the third embodiment is characterized by including optically detecting an agglutination reaction caused as a result of the mixing of the affinity particle of the third embodiment and the specimen. When the agglutination reaction is optically detected, the target substance in the specimen is detected, and the concentration of the target substance can be measured. As a method of optically detecting the agglutination reaction, an optical instrument that can detect a scattered light intensity, a transmitted light intensity, an absorbance, and the like only needs to be used to measure the variations of these values.

Next, a preferred method of producing the particle of the third embodiment is described.

The third embodiment is a method of producing a particle, the method being characterized by including: a step 1 of mixing glycidyl (meth)acrylate, styrene or methyl (meth)acrylate, water, and a radical polymerization initiator to form a particulate copolymer, thereby providing an aqueous dispersion of the particulate copolymer; and a step 2 of mixing the aqueous dispersion and mercaptosuccinic acid or mercaptopropionic acid to prepare a mixed liquid, followed by causing of an epoxy group derived from glycidyl (meth)acrylate of the particulate copolymer and a thiol group derived from mercaptosuccinic acid or mercaptopropionic acid to react with each other.

First, the step 1 is described. The step 1 is a step of forming the particulate copolymer, but a method of forming the particulate copolymer is not limited to radical polymerization to the extent that the objects of the third embodiment can be achieved. Of various kinds of the radical polymerization, emulsion polymerization, soap-free emulsion polymerization, or suspension polymerization is preferably used, and the emulsion polymerization or the soap-free emulsion polymerization is more preferably used. The soap-free emulsion polymerization is still more preferably used. In general, the emulsion polymerization and the soap-free emulsion polymerization can each provide a particulate copolymer having a particle diameter distribution sharper than that of a particulate copolymer provided by the suspension polymerization. In addition, when the particle is chemically bonded to the ligand, concern is raised about the modification of the ligand by the presence of such an anionic surfactant to be generally used in the emulsion polymerization. Accordingly, the method of forming the particulate copolymer is most preferably the soap-free emulsion polymerization.

The radical polymerization initiator to be preferably used in the step 1 is one of 4,4'-azobis(4-cyanovaleric acid), 2,2'-azobis[2-methyl-N-(2-hydroxyethyl)propionamide], 2,2'-azobis[N-(2-carboxyethyl)-2-methylpropionamidine] tetrahydrate, 2,2'-azobis(2-methylpropionamidine) dihydrochloride, 2,2'-azobis[2-(2-imidazolin-2-yl)propane], 2,2'-azobis [2-(2-imidazolin-2-yl)propane] dihydrochloride, and 2,2'-azobis[2-(2-imidazolin-2-yl)propane] disulfate dihydrate. Of those, one of 2,2'-azobis[2-(2-imidazolin-2-yl)propane] dihydrochloride, 2,2'-azobis[2-(2-imidazolin-2-yl)propane] disulfate dihydrate, 2,2'-azobis(2-methylpropionamidine) dihydrochloride, and 2,2'-azobis[2-(2-imidazolin-2-yl)propane] is more preferably used. This is because in the step 1 of providing the aqueous dispersion of the particulate copolymer, the ring opening of the epoxy group derived from glycidyl (meth)acrylate needs to be prevented. For example, when potassium persulfate is used as the radical polymerization initiator, a radical polymerization reaction field becomes acidic under the influence of the initiator residue, and hence the epoxy group derived from glycidyl (meth)acrylate may react with water to form a glycol. In addition, when ammonium persulfate is used as the radical polymerization initiator, the epoxy group derived from glycidyl (meth)acrylate and ammonia may react with each other. In addition, when an anionic radical polymerization initiator having a carboxy group is used as the radical polymerization initiator, the epoxy group derived from glycidyl (meth)acrylate and the carboxy group derived from the polymerization initiator react with each other to agglutinate the particles of the particulate copolymer. Possible means for avoiding the agglutination is as follows: the particulate copolymer is formed at a temperature considerably lower than the 10-hour half-life temperature of the radical polymerization initiator. However, the means is not suitable for industrialization because a large amount of the radical polymerization initiator is required and a radical polymerization time becomes longer.

In addition, in the step 1, a crosslinkable radical-polymerizable monomer is preferably further incorporated in addition to glycidyl (meth)acrylate and styrene or methyl (meth)acrylate. The incorporation of the crosslinkable radical-polymerizable monomer makes the particulate copolymer to be obtained physically strong, and hence eliminates concern about the cracking or chipping of the copolymer even when a centrifugal operation is repeated at the time of the purification thereof.

Examples of the crosslinkable radical-polymerizable monomer that may be used in the third embodiment are listed below, but the third embodiment is not limited thereto. In addition, two or more kinds of oily radical-polymerizable monomers may be used. Of the exemplified radical-polymerizable monomers, divinylbenzene is preferably used because

of its excellent handleability at the time of the radical polymerization reaction, though the reason why divinylbenzene is excellent in handleability is unknown.

Examples of the crosslinkable radical-polymerizable monomer include diethylene glycol diacrylate, triethylene glycol diacrylate, tetraethylene glycol diacrylate, polyethylene glycol diacrylate, 1,6-hexanediol diacrylate, neopentyl glycol diacrylate, tripropylene glycol diacrylate, polypropylene glycol diacrylate, 2,2'-bis(4-(acryloxydiethoxy)phenyl) propane, trimethylolpropane triacrylate, tetramethylolmethane tetraacrylate, ethylene glycol dimethacrylate, diethylene glycol dimethacrylate, triethylene glycol dimethacrylate, tetraethylene glycol dimethacrylate, polyethylene glycol dimethacrylate, 1,3-butylene glycol dimethacrylate, 1,6-hexanediol dimethacrylate, neopentyl glycol dimethacrylate, polypropylene glycol dimethacrylate, 2,2'-bis(4-(methacryloxydiethoxy)phenyl)propane, 2,2'-bis(4-(methacryloxypolyethoxy)phenyl)propane, trimethylolpropane trimethacrylate, tetramethylolmethane tetramethacrylate, divinylbenzene, divinylnaphthalene, and divinyl ether.

In addition, the step 1 preferably further includes a step of further mixing glycidyl (meth)acrylate into the mixture in a process for the formation of the particulate copolymer to coat the surface of the particulate copolymer with polyglycidyl (meth)acrylate. Herein, a state in which the surface is coated with the polymer may be any bonding state. That is, the polymer is not necessarily required to coat the entirety of the particle, and may coat part of the particle, and the layer of the coating polymer may not be uniform.

Next, the step 2 is described. The step 2 is a step of causing the epoxy group derived from glycidyl (meth)acrylate of the particulate copolymer and the thiol group derived from mercaptosuccinic acid or mercaptopropionic acid to react with each other to introduce a carboxy group through a sulfide group.

In the step 2, a basic component may be added for adjusting the pH of the mixed liquid. Although the basic component is not particularly limited, an organic base having a secondary or tertiary amine is preferably used. For example, pyridine, triethylamine, diazabicycloundecene, or 1,8-bis(dimethylamino)naphthalene is preferably used as the organic base having a secondary or tertiary amine, and triethylamine is more preferably used. The two or more kinds of organic bases may be used in combination.

In addition, in the step 2, a carboxylic acid amount can be controlled by adjusting the pH of the mixed liquid. In particular, when the pH is adjusted within the range of more than 9.0, a large amount of a carboxylic acid can be added to the particle to be produced. Further, the pH is more preferably set to more than 10.0 because a larger amount of the carboxylic acid can be added to the particle to be produced.

<Carboxylic Acid Quantitative Analysis Method>

First, a method of measuring the carboxyl group amount of the particle is described. The carboxy group of the particle is turned into an active ester with N-hydroxysuccinimide (NHS) through use of 1-ethyl-3-(3-dimethylaminopropyl) carbodiimide hydrochloride (EDC/HCl) as a catalyst. After that, the active ester is caused to react with aminoethanol to liberate NHS, and a carboxy group amount per unit particle mass is calculated by determining the amount of the liberated NHS with a high-performance liquid chromatograph apparatus. The unit of the carboxyl group amount per unit particle mass is nmol/mg. A specific method is described below.

<Active Esterification>

An aqueous dispersion of the particles containing 2.5 mg of the particles is added to a 1.5 mL microtube, and the particles are separated from the dispersion with a centrifugal separator. Further, the particles are redispersed in dimethylformamide (DMF). The foregoing operation is performed three times. Further, DMF is removed from the microtube under a state in which the particles are sedimented in the microtube with the centrifugal separator. Next, 400 μL of DMF, 19.2 mg of EDC·HCl, and 100 μL of a 1 mol/L solution of N-hydroxysuccinimide in DMF are added to the microtube, and the mixture is shaken at 25°C for 2 hours to turn the carboxy groups of the particles into active esters.

<Liberation of NHS>

To remove excess NHS from the DMF dispersion of the active esterified particles, the active esterified particles are separated from the dispersion with a centrifugal separator, and the particles are redispersed in DMF; the foregoing operation is performed three times. Further, DMF is removed from the microtube under a state in which the active esterified particles are sedimented in the microtube with the centrifugal separator. Next, 500 μL of a 1 mol/L solution of aminoethanol in DMF is added to the microtube, and the mixture is shaken at 25°C for 2 hours to liberate NHS from the particles.

<Determination of Amount of NHS>

The DMF dispersion of the particles containing the liberated NHS is centrifuged, and a DMF solution containing

the liberated NHS is recovered under a state in which the particles are sedimented in the microtube, followed by the determination of the amount of the liberated NHS in the DMF solution with a high-performance liquid chromatograph apparatus. The carboxy group amount per unit particle mass is calculated by using the determined value.

An instrument and reagents to be used in the determination of the carboxy group amount are as described below.

<Instrument>

High-performance liquid chromatograph apparatus: LC20A-FL (Shimadzu Corporation)
Column: Kinetex 5 μm C18 100A LC Column 150×4.6 mm (Phenomenex)
Eluent: 4 mmol/L aqueous solution of ammonium acetate
Flow rate: 1.0 mL/min
Oven temperature: 40°C
Sample injection amount: 1 μL

At the time of the determination of the amount of the liberated NHS incorporated into the DMF solution, a calibration curve between a peak area derived from NHS and the amount of NHS is produced in the range of from 0.1 mmol/L to 10 mmol/L, and the amount of the liberated NHS is determined from the NHS peak area.

<Reagents>

EDC/HCl (Dojindo Laboratories)
NHS (Kishida Chemical Co., Ltd.)
Aminoethanol (Tokyo Chemical Industry Co., Ltd.)
DMF (FUJIFILM Wako Pure Chemical Corporation)
Ammonium acetate (Tokyo Chemical Industry Co., Ltd.)

Examples

Now, the present invention is described in detail by way of Examples corresponding to the first embodiment. However, the present invention is not limited to these Examples.

[Example 1-1]

(Synthesis of Particulate Copolymer 1-1)

22.7 g of styrene (St: Kishida Chemical Co., Ltd.), 33.9 g of glycidyl methacrylate (GMA: Tokyo Chemical Industry Co., Ltd.), 0.86 g of divinylbenzene (DVB: Kishida Chemical Co., Ltd.), and 2,168.6 g of ion-exchanged water were weighed in a 2 L four-necked separable flask to provide a mixed liquid. After that, the mixed liquid was held at 70°C while being stirred at 200 rpm, and nitrogen was allowed to flow at a flow rate of 200 ml/min to remove oxygen from the inside of the three-necked separable flask. Next, a separately prepared dissolved liquid, which had been obtained by dissolving 1.13 g of V-50 (FUJIFILM Wako Pure Chemical Corporation) in 30 g of ion-exchanged water, was added to the mixed liquid to initiate soap-free emulsion polymerization. Two hours after the initiation of the polymerization, 5.8 g of GMA was added to the four-necked separable flask, and the mixture was held at 70°C while being stirred for 22 hours at 200 rpm. Thus, an aqueous dispersion containing a particulate copolymer 1-1 was obtained. After the dispersion had been gradually cooled to room temperature, part of the dispersion was collected, and its polymerization conversion ratio was evaluated by using proton NMR, gas chromatography, and gel permeation chromatography. As a result, it was recognized that the polymerization conversion ratio was substantially 100%. The particulate copolymer 1-1 had a dry particle diameter of 196.6 nm and a particle diameter in water of 206.9 nm. The particulate copolymer 1-1 was subjected to ultrafiltration concentration, or was diluted by the addition of ion-exchanged water, so as to be a 2.5 wt% aqueous dispersion, and the dispersion was stored under a light-shielding condition at 4°C.

[Example 1-2]

(Synthesis of Particulate Copolymer 1-2)

An aqueous dispersion of a particulate copolymer 2 was obtained in the same manner as in Example 1-1 except that, in Example 1-1, 22.7 g of St was changed to 12.0 g thereof, 33.9 g of GMA was changed to 17.9 g thereof, 0.86 g of divinylbenzene was changed to 0.45 g thereof, and the amount of GMA to be added to the three-necked separable flask 2 hours after the initiation of the polymerization was changed from 5.8 g to 3.1 g. After the dispersion had been gradually cooled to room temperature, part of the dispersion was collected, and its polymerization conversion ratio was evaluated by using proton NMR, gas chromatography, and gel permeation chromatography. As a result, it was recognized that the polymerization conversion ratio was substantially 100%. The particulate copolymer 1-2 had a dry particle diameter of 151.4 nm and a particle diameter in water of 160.2 nm. The particulate copolymer 1-2 was subjected to ultrafiltration concentration, or was diluted by the addition of ion-exchanged water, so as to be a 2.5 wt% aqueous dispersion, and the dispersion was stored under a light-shielding condition at 4°C.

[Example 1-3]

(Synthesis of Particulate Copolymer 1-3)

An aqueous dispersion of a particulate copolymer 1-3 was obtained in the same manner as in Example 1-1 except that, in Example 1-1, 22.7 g of St was changed to 5.0 g thereof, 33.9 g of GMA was changed to 7.5 g thereof, 0.86 g of divinylbenzene was changed to 0.19 g thereof, and the amount of GMA to be added to the three-necked separable flask 2 hours after the initiation of the polymerization was changed from 5.8 g to 1.3 g. After the dispersion had been gradually cooled to room temperature, part of the dispersion was collected, and its polymerization conversion ratio was evaluated by using proton NMR, gas chromatography, and gel permeation chromatography. As a result, it was recognized that the polymerization conversion ratio was substantially 100%. The particulate copolymer 1-3 had a dry particle diameter of 96.8 nm and a particle diameter in water of 105.2 nm. The particulate copolymer 1-3 was subjected to ultrafiltration concentration, or was diluted by the addition of ion-exchanged water, so as to be a 2.5 wt% aqueous dispersion, and the dispersion was stored under a light-shielding condition at 4°C.

[Example 1-4]

(Synthesis of Particles 1-1)

24 g of the 2.5 wt% aqueous dispersion of the particulate copolymer 1-1, 3.3 g of ion-exchanged water, 40 mg (0.26 mmol) of mercaptosuccinic acid (FUJIFILM Wako Pure Chemical Corporation), and 0.214 mL (2.34 mmol) of 3-mercapto-1,2-propanediol (FUJIFILM Wako Pure Chemical Corporation) were weighed in a 100 ml round-bottom flask, and triethylamine (Kishida Chemical Co., Ltd.) was added to the mixture to adjust its pH to 10. Next, the temperature of the contents of the round-bottom flask was increased to 70°C while the contents were stirred at 200 rpm. Further, the contents were held in this state for 4 hours to provide a dispersion of particles 1-1. The particles 1-1 were separated from the dispersion with a centrifugal separator, and the particles 1-1 were redispersed in ion-exchanged water; the operation was repeated eight times to purify the particles 1-1, which were stored in the state of an aqueous dispersion in which the concentration of the particles 1-1 was finally adjusted to 1.0 wt%. Storage conditions were set to 4°C under a light-shielding condition. Table 1-1 shows a summary of the particle physical properties of the particles 1-1.

[Example 1-5]

(Synthesis of Particles 1-2)

A 1.0 wt% aqueous dispersion of particles 1-2 was obtained in the same manner as in Example 1-4 except that, in Example 1-4, the period of time for which the temperature of the contents of the round-bottom flask was held after having been increased to 70°C was changed from 4 hours to 6 hours. Table 1-1 shows a summary of the particle physical properties of the particles 1-2.

[Example 1-6]

(Synthesis of Particles 1-3)

A 1.0 wt% aqueous dispersion of particles 1-3 was obtained in the same manner as in Example 1-4 except that, in Example 1-4, the period of time for which the temperature of the contents of the round-bottom flask was held after having

been increased to 70°C was changed from 4 hours to 8 hours. Table 1-1 shows a summary of the particle physical properties of the particles 1-3.

[Example 1-7]

(Synthesis of Particles 1-4)

A 1.0 wt% aqueous dispersion of particles 1-4 was obtained in the same manner as in Example 1-4 except that, in Example 1-4, the period of time for which the temperature of the contents of the round-bottom flask was held after having been increased to 70°C was changed from 4 hours to 12 hours. Table 1-1 shows a summary of the particle physical properties of the particles 1-4.

[Example 1-8]

(Synthesis of Particles 1-5)

A 1.0 wt% aqueous dispersion of particles 1-5 was obtained in the same manner as in Example 1-4 except that, in Example 1-4, the period of time for which the temperature of the contents of the round-bottom flask was held after having been increased to 70°C was changed from 4 hours to 18 hours. Table 1-1 shows a summary of the particle physical properties of the particles 1-5.

[Example 1-9]

(Synthesis of Particles 1-6)

A 1.0 wt% aqueous dispersion of particles 1-6 was obtained in the same manner as in Example 1-8 except that the particulate copolymer 1-1 of Example 1-8 was changed to the particulate copolymer 1-2. Table 1-1 shows a summary of the particle physical properties of the particles 1-6.

[Example 1-10]

(Synthesis of Particles 1-7)

A 1.0 wt% aqueous dispersion of particles 1-7 was obtained in the same manner as in Example 1-8 except that the particulate copolymer 1-1 of Example 1-8 was changed to the particulate copolymer 1-3. Table 1-1 shows a summary of the particle physical properties of the particles 1-7.

[Example 1-11]

(Synthesis of Particles 1-8)

24 g of the 2.5 wt% aqueous dispersion of the particulate copolymer 1-1, 3.3 g of ion-exchanged water, 0.046 mL (0.52 mmol) of mercaptopropionic acid (FUJIFILM Wako Pure Chemical Corporation), and 0.190 mL (2.08 mmol) of 3-mercapto-1,2-propanediol (FUJIFILM Wako Pure Chemical Corporation) were weighed in a 100 ml round-bottom flask, and triethylamine (Kishida Chemical Co., Ltd.) was added to the mixture to adjust its pH to 10. Next, the temperature of the contents of the round-bottom flask was increased to 70°C while the contents were stirred at 200 rpm. Further, the contents were held in this state for 18 hours to provide a dispersion of particles 1-8. The particles 1-8 were separated from the dispersion with a centrifugal separator, and the particles 1-8 were re-dispersed in ion-exchanged water; the operation was repeated eight times to purify the particles 1-8, which were stored in the state of an aqueous dispersion in which the concentration of the particles 1-8 was finally adjusted to 1.0 wt%. Storage conditions were set to 4°C under a light-shielding condition. Table 1-1 shows a summary of the particle physical properties of the particles 1-8.

[Example 1-12]

(Synthesis of Particles 1-9)

A 1.0 wt% aqueous dispersion of particles 1-9 was obtained in the same manner as in Example 1-4 except that, in Example 1-4, the pH was changed from a pH of 10 to a pH of 11, and the period of time for which the temperature of the

contents of the round-bottom flask was held after having been increased to 70°C was changed from 4 hours to 18 hours. Table 1-1 shows a summary of the particle physical properties of the particles 1-9.

[Example 1-13]

(Synthesis of Particles 1-10)

A 1.0 wt% aqueous dispersion of particles 1-10 was obtained in the same manner as in Example 1-4 except that, in Example 1-4, the pH was changed from a pH of 10 to a pH of 11, and the period of time for which the temperature of the contents of the round-bottom flask was held after having been increased to 70°C was changed from 4 hours to 30 hours. Table 1-1 shows a summary of the particle physical properties of the particles 1-10.

[Example 1-14]

(Synthesis of Particles 1-11)

A 1.0 wt% aqueous dispersion of particles 1-11 was obtained in the same manner as in Example 1-4 except that, in Example 1-4, the pH was changed from a pH of 10 to a pH of 11, and the period of time for which the temperature of the contents of the round-bottom flask was held after having been increased to 70°C was changed from 4 hours to 48 hours. Table 1-1 shows a summary of the particle physical properties of the particles 1-11.

[Example 1-15]

(Synthesis of Particles 1-12)

A 1.0 wt% aqueous dispersion of particles 1-12 was obtained in the same manner as in Example 1-11 except that, in Example 1-11, mercaptopropionic acid was changed to 323 mg (2.08 mmol) of mercaptosuccinic acid, and the amount of 3-mercapto-1,2-propanediol was changed to 0.047 mL (0.52 mmol). Table 1-1 shows a summary of the particle physical properties of the particles 1-12.

[Example 1-16]

(Synthesis of Particles 1-13)

A 1.0 wt% aqueous dispersion of particles 1-13 was obtained in the same manner as in Example 1-15 except that, in Example 1-15, the amount of mercaptosuccinic acid was changed to 282 mg (1.82 mmol), and the amount of 3-mercapto-1,2-propanediol was changed to 0.071 mL (0.78 mmol). Table 1-1 shows a summary of the particle physical properties of the particles 1-13.

[Example 1-17]

(Synthesis of Particles 1-14)

A 1.0 wt% aqueous dispersion of particles 1-14 was obtained in the same manner as in Example 1-15 except that, in Example 1-15, the amount of mercaptosuccinic acid was changed to 242 mg (1.56 mmol), and the amount of 3-mercapto-1,2-propanediol was changed to 0.095 mL (1.04 mmol). Table 1-1 shows a summary of the particle physical properties of the particles 1-14.

[Example 1-18]

(Synthesis of Particles 1-15)

A 1.0 wt% aqueous dispersion of particles 1-15 was obtained in the same manner as in Example 1-15 except that, in Example 1-15, the amount of mercaptosuccinic acid was changed to 202 mg (1.30 mmol), and the amount of 3-mercapto-1,2-propanediol was changed to 0.119 mL (1.30 mmol). Table 1-1 shows a summary of the particle physical properties of the particles 1-15.

[Example 1-19]

(Synthesis of Particles 1-16)

A 1.0 wt% aqueous dispersion of particles 1-16 was obtained in the same manner as in Example 1-15 except that, in Example 1-15, the amount of mercaptosuccinic acid was changed to 81 mg (0.52 mmol), and the amount of 3-mercapto-1,2-propanediol was changed to 0.191 mL (2.08 mmol). Table 1-1 shows a summary of the particle physical properties of the particles 1-16.

[Example 1-20]

(Synthesis of Particles 1-17)

A 1.0 wt% aqueous dispersion of particles 1-17 was obtained in the same manner as in Example 1-15 except that, in Example 1-15, the amount of mercaptosuccinic acid was changed to 20 mg (0.13 mmol), and the amount of 3-mercapto-1,2-propanediol was changed to 0.225 mL (2.47 mmol). Table 1-1 shows a summary of the particle physical properties of the particles 1-17.

[Comparative Example 1-1]

(Synthesis of Particles 1-18)

A 1.0 wt% aqueous dispersion of particles 1-18 was obtained in the same manner as in Example 1-4 except that, in Example 1-4, the period of time for which the temperature of the contents of the round-bottom flask was held after having been increased to 70°C was changed from 4 hours to 3 hours. Table 1-1 shows a summary of the particle physical properties of the particles 1-18.

[Comparative Example 1-2]

(Synthesis of Particles 1-19)

A 1.0 wt% aqueous dispersion of particles 1-19 was obtained in the same manner as in Example 1-4 except that, in Example 1-4, the pH was changed from a pH of 10 to a pH of 11, and the period of time for which the temperature of the contents of the round-bottom flask was held after having been increased to 70°C was changed from 4 hours to 60 hours. Table 1-1 shows a summary of the particle physical properties of the particles 1-19.

[Comparative Example 1-3]

(Synthesis of Particles 1-20)

A 1.0 wt% aqueous dispersion of particles 1-20 was obtained in the same manner as in Example 1-15 except that, in Example 1-15, the amount of mercaptosuccinic acid was changed to 403 mg (2.60 mmol), and 3-mercapto-1,2-propanediol was not used. Table 1-1 shows a summary of the particle physical properties of the particles 1-20.

[Comparative Example 1-4]

(Synthesis of Particles 1-21)

A 1.0 wt% aqueous dispersion of particles 1-21 was obtained in the same manner as in Example 1-15 except that, in Example 1-15, the amount of mercaptosuccinic acid was changed to 8 mg (0.05 mmol), and the amount of 3-mercapto-1,2-propanediol was changed to 0.232 mL (2.55 mmol). Table 1-1 shows a summary of the particle physical properties of the particles 1-21.

[Comparative Example 1-5]

(Synthesis of Particles 1-22)

The 2.5 wt% aqueous dispersion of the particulate copolymer 1-1 obtained in Example 1-1 was concentrated to a 10 wt% aqueous dispersion with a centrifugal separator to give a concentrated dispersion. 20 g of the concentrated dispersion was weighed in a 200 mL round-bottom flask. Under a state in which the concentrated dispersion was held at

4°C, 50-fold mol of 28% ammonia water (Kishida Chemical Co., Ltd.) with respect to the theoretical amount of the GMA-derived epoxy groups contained in the 20 g of the concentrated dispersion was added thereto, and the pH of the mixture was adjusted to 11 by using a 2 N hydrochloric acid aqueous solution and a 2 N sodium hydroxide aqueous solution. After that, the temperature of the contents of the round-bottom flask was increased to 70°C while the contents were stirred at 100 rpm. The contents were held in this state for 24 hours to provide a dispersion of particles 1-22-1. The particles 1-22-1 were separated from the dispersion with a centrifugal separator, and the particles 1-22-1 were re-dispersed in ion-exchanged water; the operation was repeated eight times to purify the particles 1-22-1, which were stored in the state of an aqueous dispersion in which the concentration of the particles 1-22-1 was finally adjusted to 10 wt%. Storage conditions were set to 4°C under a light-shielding condition.

6.3 g of the 10 wt% aqueous dispersion of the particles 1-22-1 and 3.81 g of ethylene glycol diglycidyl ether (Tokyo Chemical Industry Co., Ltd.) were weighed in a 50 ml round-bottom flask, and the pH of the mixture was adjusted to 9 by using a 0.1 N hydrochloric acid aqueous solution and a 0.1 N sodium hydroxide aqueous solution. After that, the temperature of the contents of the round-bottom flask was increased to 30°C while the contents were stirred at 100 rpm. The contents were held in this state for 24 hours to provide a dispersion of particles 1-22-2. The particles 1-22-2 were separated from the dispersion with a centrifugal separator, and the particles 1-22-2 were re-dispersed in ion-exchanged water; the operation was repeated eight times to purify the particles 1-22-2, which were stored in the state of an aqueous dispersion in which the concentration of the particles 1-22-2 was finally adjusted to 10 wt%. Storage conditions were set to 4°C under a light-shielding condition.

6.0 g of the 10 wt% aqueous dispersion of the particles 1-22-2, and 10-fold mol of a 28% ammonia aqueous solution with respect to the theoretical amount of the ethylene glycol diglycidyl ether-derived epoxy groups of the particles contained in the aqueous dispersion (assuming that 100% of the glycidyl methacrylate-derived epoxy groups of the particulate copolymer 1-1 had reacted with ammonia to be transformed into primary amines, and further assuming that 100% of the primary amines had reacted with one of the two terminal epoxy groups of ethylene glycol diglycidyl ether) were added to a 50 ml round-bottom flask, and the pH of the mixture was adjusted to 11 by using a 1 N hydrochloric acid aqueous solution and a 1 N sodium hydroxide aqueous solution. After that, the temperature of the contents of the round-bottom flask was increased to 70°C while the contents were stirred at 100 rpm. The contents were held in this state for 24 hours to provide a dispersion of particles 1-22-3. The particles 1-22-3 were separated from the dispersion with a centrifugal separator, and the particles 1-22-3 were re-dispersed in ion-exchanged water; the operation was repeated eight times to purify the particles 1-22-3, which were stored in the state of an aqueous dispersion in which the concentration of the particles 1-22-3 was finally adjusted to 10 wt%. Storage conditions were set to 4°C under a light-shielding condition.

The particles 1-22-3 were separated from the 10 wt% aqueous dispersion of the particles 1-22-3 with a centrifugal separator, and the particles 1-22-3 were re-dispersed in methanol; the operation was repeated three times to prepare a methanol dispersion of the particles 1-22-3 in which the concentration of the particles 1-22-3 was finally adjusted to 1 wt%. 63 g of the methanol dispersion of the particles 1-22-3 and 2.77 g of succinic anhydride (Tokyo Chemical Industry Co., Ltd.) were weighed in a 200 ml round-bottom flask. After that, the temperature of the contents of the round-bottom flask was increased to 30°C while the contents were stirred at 100 rpm. The contents were held in this state for 5 hours to provide a dispersion of particles 1-22. The particles 1-22 were separated from the dispersion with a centrifugal separator, and the particles 1-22 were re-dispersed in ion-exchanged water; the operation was repeated eight times to purify the particles 1-22, which were stored in the state of an aqueous dispersion in which the concentration of the particles 1-22 was finally adjusted to 1.0 wt%.

[Comparative Example 1-6]

(Synthesis of Particles 1-23)

The 2.5 wt% aqueous dispersion of the particulate copolymer 1-1 obtained in Example 1-1 was concentrated to a 10 wt% aqueous dispersion with a centrifugal separator to give a concentrated dispersion. 20 g of the concentrated dispersion was weighed in a 200 ml round-bottom flask. Under a state in which the concentrated dispersion was held at 4°C, 10-fold mol of glycine (Kishida Chemical Co., Ltd.) with respect to the theoretical amount of the GMA-derived epoxy groups contained in the 20 g of the concentrated dispersion was added thereto, and the pH of the mixture was adjusted to 11 by using a 0.1 N hydrochloric acid aqueous solution and a 0.1 N sodium hydroxide aqueous solution. After that, the temperature of the contents of the round-bottom flask was increased to 70°C while the contents were stirred at 100 rpm. The contents were held in this state for 24 hours to provide a dispersion of particles 1-23. The particles 1-23 were separated from the dispersion with a centrifugal separator, and the particles 1-23 were re-dispersed in ion-exchanged water; the operation was repeated eight times to purify the particles 1-23, which were stored in the state of an aqueous dispersion in which the concentration of the particles 1-23 was finally adjusted to 10 wt%. Storage conditions were set to 4°C under a light-shielding condition.

[Example 1-21]

(Evaluation of Nonspecific Adsorption to Particles)

The particles 1-1 to the particles 1-23 were each dispersed in a phosphate buffer (containing 0.01% Tween 20) at 0.1 wt% to prepare a dispersion (liquid P). Next, 51 μL of a diluted specimen liquid (liquid Q) formed of a human normal specimen (serum specimen, 1 μL) and a phosphate buffer (50 μL) was added to 50 μL of each dispersion, and the absorbance of the mixed liquid immediately after its stirring at a wavelength of 572 nm was measured. A spectrophotometer GeneQuant 1300 manufactured by Biochrom was used in the absorbance measurement. Then, each mixed liquid was left at rest at 37°C for 5 minutes, and then its absorbance at a wavelength of 572 nm was measured again, followed by the calculation of the value "variation ΔABS in absorbance×10,000". The results are summarized in Table 1-1. It is understood that, as the value becomes larger, nonspecific adsorption occurs to a larger extent. However, when particles having a larger carboxy group density or particles having a large value of [particle diameter in water/dry particle diameter] have a large value, the particles may have detected osmotic pressure agglutination instead of agglutination resulting from nonspecific adsorption. In any case, when particles having a large value of the "variation ΔABS in absorbance×10,000" in this evaluation are used as particles for a latex agglutination method in a specimen test, concern is raised in that a normal specimen is interpreted as being false positive owing to noise. A case in which the liquid Q contains 1 μL of the human normal specimen is represented as "human normal specimen concentration ×1". A case in which the liquid Q contains 2.5 μL of the human normal specimen is represented as "human normal specimen concentration ×2.5". A case in which the liquid Q contains 5 μL of the human normal specimen is represented as "human normal specimen concentration ×5". The amounts of the normal specimen are 1 times, 2.5 times, and 5 times, respectively. The threshold values for excellent, good, fair, and bad in Table 1-1 are criteria determined from noise risks in the detection of a target substance having a low concentration by the latex agglutination method.

[Example 1-22]

(Production of Affinity Particles by Antibody Sensitization to Particles)

0.1 mL (1 mg in terms of particles) of the particle dispersion (solution having a concentration of 1.0 wt%, 10 mg/mL) of each of the particles 1-1 to 1-23 was transferred to a microtube (volume: 1.5 mL), 0.12 mL of an activation buffer (25 mM MES, pH: 6.0) was added thereto, and the mixture was centrifuged at 4°C and 15,000 rpm (20,400 g) for 5 minutes. After the centrifugation, the supernatant was discarded. 0.12 mL of an activation buffer (25 mM MES, pH: 6.0) was added to the residue, and the particles were re-dispersed with an ultrasonic wave. The centrifugation and the re-dispersion were repeated once.

Next, 60 μL each of a WSC solution (solution obtained by dissolving 50 mg of WSC in 1 mL of an activation buffer, the term "WSC" means 1-[3-(dimethylaminopropyl)-3-ethylcarbodiimide] hydrochloride) and a Sulfo NHS solution (solution obtained by dissolving 50 mg of Sulfo NHS in 1 mL of an activation buffer, the term "Sulfo NHS" means sulfo-N-hydroxysuccinimide) were added to the resultant, and were dispersed therein with an ultrasonic wave. The dispersion was stirred at room temperature for 30 minutes to transform the carboxy groups of its particles into active esters. The resultant was centrifuged at 4°C and 15,000 rpm (20,400 g) for 5 minutes, and the supernatant was discarded. 0.2 mL of an immobilization buffer (25 mM MES, pH: 5.0) was added to the residue, and the particles were dispersed with an ultrasonic wave. The dispersion was centrifuged at 4°C and 15,000 rpm (20,400 g) for 5 minutes, and the supernatant was discarded. 50 μL of the immobilization buffer was added to the residue, and the particles whose carboxy groups had been activated were dispersed with an ultrasonic wave.

50 μL of an antibody solution (solution obtained by diluting an anti-CRP antibody with the immobilization buffer so that its concentration became 25 μg/50 μL) was added to 50 μL of the solution of the particles whose carboxy groups had been activated, and the particles were dispersed with an ultrasonic wave. The loading amount of the antibody is 25 μg per 1 mg of the particles (25 μg/mg). An antibody final concentration is 0.25 mg/mL, and a particle final concentration is 10 mg/mL. The contents in the microtube were stirred at room temperature for 60 minutes to bond the antibody to the carboxy groups of the particles. Next, the resultant was centrifuged at 4°C and 15,000 rpm (20,400 g) for 5 minutes, and the supernatant was discarded. 0.24 mL of a masking buffer (buffer obtained by incorporating 0.1% Tween 20 into 1 M Tris having a pH of 8.0) was added to the residue, and the particles were dispersed with an ultrasonic wave. The dispersion was stirred at room temperature for 1 hour, and was then left at rest at 4°C overnight to bond Tris to the remaining activated carboxy groups. Next, the resultant was centrifuged at 4°C and 15,000 rpm (20,400 g) for 5 minutes, and the supernatant was discarded. 0.2 mL of a washing buffer (10 mM HEPES, pH: 7.9) was added to the residue, and the particles were dispersed with an ultrasonic wave. The washing operation (the centrifugation and the re-dispersion) with the washing buffer (10 mM HEPES, pH: 7.9) was repeated once. A washing operation was performed with 0.2 mL of a storage buffer (10 mM HEPES, pH: 7.9, containing 0.01% Tween 20) once. 1.0 mL of the storage buffer was added to the washed product,

and the particles were dispersed with an ultrasonic wave. The particle concentration of the dispersion finally became 0.1 wt% (1 mg/mL). The dispersion was stored in a refrigerator. The names of affinity particles are hereinafter represented as "affinity particles 1-1" and the like directly after the particle names.

[Example 1-23]

(Evaluation of Antibody Sensitization Ratio of Affinity Particles)

The antibody sensitization ratio (%) of the affinity particles produced in Example 1-21 was determined by protein determination. Herein, the term "antibody sensitization ratio (%)" means the ratio of the amount of the antibody bonded to the particles to the amount of the antibody used in the reaction with the particles (antibody loading amount). An evaluation example of the protein determination is described below.

First, 7 mL of the liquid A of PROTEIN ASSAY BCA KIT (Wako Pure Chemical Industries, Ltd.) and 140 μL of the liquid B thereof were mixed, and the prepared liquid was adopted as a liquid AB. Next, 25 μL (particle amount: 25 μg) of the dispersion (0.1% solution) of the affinity particles was taken, and was loaded into a 1.5 mL tube. Next, 200 μL of the liquid AB was added to the dispersion (25 μL), and the mixture was incubated at 60°C for 30 minutes. The resultant solution was centrifuged at 4°C and 15,000 rpm (20,400 g) for 5 minutes, and 200 μL of the supernatant was loaded into a 96-well microwell with a pipetter. The absorbance of the supernatant at 562 nm was measured with a microplate reader together with standard samples (several samples were obtained by diluting the antibody with 10 mM HEPES so that its concentration fell within the range of from 0 μg/mL to 200 μg/mL). The amount of the antibody was calculated from a standard curve. The amount of the antibody sensitized to the particles (the amount of the bonded antibody per weight of the particles (μg/mg)) was determined by dividing the calculated antibody amount by the weight of the particles (herein, 0.025 mg). Finally, the sensitization ratio was calculated. In the case where the antibody loading amount is 25 μg per 1 mg of the particles, when the antibody sensitization amount is 12.5 μg/mg, the sensitization ratio is 50%. The results are summarized in Table 1-1.

[Example 1-24]

(Evaluation of Latex Agglutination Sensitivity of Affinity Particles)

1 μL of human CRP (Denka Seiken Co., Ltd., C-reactive protein, derived from human plasma, 40 μg/ml) and 50 μL of a buffer (PBS containing 0.01% Tween 20) were mixed to prepare a mixed liquid (hereinafter represented as "R1+"), and its temperature was kept at 37°C. In addition, 1 μL of physiological saline and 50 μL of the buffer (PBS containing 0.01% Tween 20) were mixed to prepare a mixed liquid (hereinafter represented as "R1-") as a control, and its temperature was similarly kept at 37°C. Next, 50 μL of the solution containing each of the affinity particles prepared in Example 1-21 (particle concentration: 0.1 wt%, referred to as "R2") was mixed with R1+ or R1-, and the absorbance of the mixed liquid (volume: 101 μL) immediately after its stirring at a wavelength of 572 nm was measured. A spectrophotometer GeneQuant 1300 manufactured by Biochrom was used in the absorbance measurement. Then, the mixed liquid was left at rest at 37°C for 5 minutes, and then its absorbance at a wavelength of 572 nm was measured again, followed by the calculation of the value "variation ΔABS in absorbance×10,000". This series of evaluations was performed for each of: R2 immediately after its preparation in Example 1-21; R2 after a lapse of 24 hours from the preparation; and R2 after a lapse of 72 hours from the preparation. The results are summarized in Table 1-1. A larger value of the R- in Table 1-1 means that agglutination resulting from nonspecific adsorption or osmotic pressure agglutination occurs in the affinity particles to a larger extent. Accordingly, when the particles are used as particles for a latex agglutination method in a specimen test, concern is raised in that a normal specimen is interpreted as being false positive owing to noise. Meanwhile, affinity particles having a larger value of the R+ in Table 1-1 are expected to be capable of detecting a target substance with higher sensitivity when used as particles for the latex agglutination method in the specimen test. The threshold values for excellent, good, fair, and bad in R- in Table 1-1 are criteria determined from noise risks in the detection of a target substance having a low concentration by the latex agglutination method. In addition, the threshold values for excellent, good, fair, and bad in R+ are criteria determined with reference to the following values obtained by using CRP-L Auto "TBA" as a kit for CRP detection and a CRP standard solution "TBA" for Latex, which are manufactured by Denka Seiken Co., Ltd.

ΔABS×10,000 of 0 μg/ml (physiological saline): -80
ΔABS×10,000 of 5 μg/ml: 1,410
ΔABS×10,000 of 20 μg/ml: 3,530
ΔABS×10,000 of 40 μg/ml: 5,130
ΔABS×10,000 of 160 μg/ml: 9,750
ΔABS×10,000 of 320 μg/ml: 12,150

[Table 1-1]

Table 1-1. Summary of Particle Characteristics, Affinity Particles (Antibody Sensitization), and Latex Agglutination Sensitivity

| | | Example | | | | | | | | | | | | | | | | |
|---|---|---|---|---|---|---|---|---|---|---|---|---|---|---|---|---|---|---|
| Particle name | | Particles 1-1 | Particles 1-2 | Particles 1-3 | Particles 1-4 | Particles 1-5 | Particles 1-6 | Particles 1-7 | Particles 1-8 | Particles 1-9 | Particles 1-10 | Particles 1-11 | Particles 1-12 | Particles 1-13 | Particles 1-14 | Particles 1-15 | Particles 1-16 | Particles 1-17 |
| Particle characteristics | (1) Particulate copolymer (dry particle diameter/nm) | 196.6 | 196.6 | 196.6 | 196.6 | 196.6 | 151.4 | 96.8 | 196.6 | 196.6 | 196.6 | 196.6 | 196.6 | 196.6 | 196.6 | 196.6 | 196.6 | 196.6 |
| | (2) Particulate copolymer (particle diameter in water/nm) | 206.9 | 206.9 | 206.9 | 206.9 | 206.9 | 160.2 | 105.2 | 206.9 | 206.9 | 206.9 | 206.9 | 206.9 | 206.9 | 206.9 | 206.9 | 206.9 | 206.9 |
| | (3) Particles (dry particle diameter/nm) | 205.9 | 206.2 | 205.3 | 205.8 | 205.6 | 161.6 | 119 | 205.9 | 205.8 | 205.7 | 206.5 | 207.6 | 205.9 | 206.1 | 207.2 | 205.8 | 205.4 |
| | (4) Particles (particle diameter in water/nm) | 230.6 | 237.1 | 242.3 | 247 | 257 | 202 | 144 | 263.8 | 267.6 | 279.8 | 289.1 | 259.5 | 259.4 | 257.6 | 256.9 | 257.25 | 262.9 |
| | (4)-(2) (nm) | 23.7 | 30.2 | 35.4 | 40.1 | 50.1 | 41.8 | 38.8 | 56.9 | 60.7 | 72.9 | 82.2 | 52.6 | 52.5 | 50.7 | 50 | 50.4 | 56 |
| | (4)-(3) (nm) | 24.7 | 30.9 | 37 | 41.2 | 51.4 | 40.4 | 25 | 57.9 | 61.8 | 74.1 | 82.6 | 51.9 | 53.5 | 21.5 | 49.7 | 51.5 | 57.5 |
| | (4)/(3) | 1.12 | 1.15 | 1.18 | 1.2 | 1.25 | 1.25 | 1.21 | 1.28 | 1.3 | 1.36 | 1.4 | 1.25 | 1.26 | 1.25 | 1.24 | 1.25 | 1.28 |
| | Number of moles of repeating unit A/number of moles of repeating unit B | 0.11 | 0.11 | 0.11 | 0.11 | 0.11 | 0.11 | 0.11 | 0.25 | 0.11 | 0.11 | 0.11 | 4 | 2.3 | 1.5 | 1 | 0.25 | 0.05 |
| | Carboxyl group density (groups/nm3) | 0.071 | 0.059 | 0.059 | 0.070 | 0.066 | 0.064 | 0.062 | 0.088 | 0.069 | 0.078 | 0.055 | 0.142 | 0.132 | 0.129 | 0.113 | 0.082 | 0.043 |
| | Zeta potential (mV) | -16 | -15 | -14 | -17 | -16 | -16 | -15 | -21 | -16 | -18 | -14 | -43 | -37 | -35 | -29 | -20 | -13 |

(continued)

Table 1-1. Summary of Particle Characteristics, Affinity Particles (Antibody Sensitization), and Latex Agglutination Sensitivity

| | | | | Example | | | | | | | | | | | | | | | | |
|---|---|---|---|---|---|---|---|---|---|---|---|---|---|---|---|---|---|---|---|---|
| Particle name | | | | Particles 1-1 | Particles 1-2 | Particles 1-3 | Particles 1-4 | Particles 1-5 | Particles 1-6 | Particles 1-7 | Particles 1-8 | Particles 1-9 | Particles 1-10 | Particles 1-11 | Particles 1-12 | Particles 1-13 | Particles 1-14 | Particles 1-15 | Particles 1-16 | Particles 1-17 |
| | | Human normal specimen concentration ×1 (ΔABS×10,000) | | 36 | 32 | 29 | 11 | -10 | -2 | -1 | 15 | 9 | 42 | 41 | 352 | 181 | 32 | -10 | 9 | 32 |
| | | Human normal specimen concentration ×2.5 (ΔABS×10,000) | | 98 | 74 | 62 | 15 | 2 | 4 | 2 | 11 | 59 | 291 | 459 | 724 | 421 | 103 | 67 | -10 | 54 |
| | | Human normal specimen concentration ×5 (ΔABS×10,000) | | 152 | 92 | 41 | -9 | 10 | 3 | 4 | 22 | 214 | 623 | 1,271 | 2,138 | 1,301 | 201 | 152 | 15 | 162 |
| Reactivity 1 | | Antibody sensitization ratio (%) | | 90 | 91 | 95 | 100 | 100 | 100 | 100 | 78 | 100 | 99 | 100 | 95 | 99 | 100 | 99 | 100 | 89 |
| Reactivity 2 | Latex agglutination sensitivity (ΔABS×10, 000) | Immediately after sensitization | R1- | 12 | 9 | 7 | 6 | 12 | 4 | 1 | 11 | 18 | 26 | 12 | 12 | 5 | 12 | 8 | 9 | 12 |
| | | | R1+ | 8,121 | 8,342 | 10,162 | 12,102 | 13,524 | 12,103 | 7,201 | 8,991 | 13,275 | 13,852 | 13,561 | 9,541 | 10,261 | 12,102 | 11,528 | 13,395 | 12,151 |
| | | 24 hours after sensitization | R1- | 28 | 11 | -15 | -10 | 6 | 2 | 3 | 6 | 8 | 35 | -41 | 38 | -19 | -15 | 15 | 5 | -41 |
| | | | R1+ | 8,218 | 8,523 | 10,098 | 12,238 | 13,259 | 11,987 | 7,093 | 9,002 | 13,711 | 13,569 | 13,821 | 9,657 | 10,198 | 12,238 | 11,593 | 13,521 | 12,328 |
| | | 72 hours after sensitization | R1- | 9 | 7 | -9 | 7 | -11 | -5 | -2 | 9 | -9 | 42 | 22 | 21 | -12 | 17 | -19 | -9 | 22 |
| | | | R1+ | 8,162 | 8,426 | 10,128 | 12,185 | 13,622 | 12,095 | 7,124 | 8,899 | 13,328 | 13,294 | 13,211 | 9,429 | 10,120 | 12,185 | 11,479 | 13,382 | 12,296 |

| Particle name | | | | Comparative Example | | | | | |
|---|---|---|---|---|---|---|---|---|---|
| | | | | Particles 1-18 | Particles 1-19 | Particles 1-20 | Particles 1-21 | Particles 1-22 | Particles 1-23 |
| Particle characteristics | (1) Particulate copolymer (dry particle diameter/nm) | | | 196.6 | 196.6 | 196.6 | 196.6 | 196.6 | 196.6 |
| | (2) Particulate copolymer (particle diameter in water/nm) | | | 206.9 | 206.9 | 206.9 | 206.9 | 206.9 | 206.9 |
| | (3) Particles (dry particle diameter/nm) | | | 198 | 209.2 | 208.1 | 205.9 | 198.2 | 201.8 |
| | (4) Particles (particle diameter in water/nm) | | | 213.8 | 303.3 | 264.3 | 253.3 | 212 | 211.9 |
| | (4)-(2) (nm) | | | 6.9 | 96.4 | 57.4 | 46.4 | 5.2 | 5 |
| | (4)-(3) (nm) | | | 15.8 | 94.1 | 56.2 | 47.4 | 13.9 | 10.1 |
| | (4)/(3) | | | 1.08 | 1.45 | 1.27 | 1.23 | 1.07 | 1.05 |
| | Number of moles of repeating unit A/number of moles of repeating unit B | | | 0.11 | 0.11 | Only repeating unit A | 0.02 | - | - |
| | Carboxyl group density (groups/nm3) | | | 0.131 | 0.044 | 0.163 | 0.027 | - | - |
| | Zeta potential (mV) | | | -29 | -15 | -48 | -9 | -51 | -48 |
| | Human normal specimen concentration ×1 (ΔABS×10,000) | | | 43 | 1,032 | 581 | 51 | 121 | 191 |
| | Human normal specimen concentration ×2.5 (ΔABS×10,000) | | | 572 | 1,988 | 2,184 | 475 | 1,083 | 2,192 |
| | Human normal specimen concentration ×5 (ΔABS×10,000) | | | 1,867 | 3,218 | 5,621 | 1,021 | 2,721 | 5,425 |
| Reactivity 1 | Antibody sensitization ratio (%) | | | 59 | 99 | 90 | 69 | 62 | 51 |
| Reactivity 2 | Latex agglutination sensitivity (ΔABS×10,000) | Immediately after sensitization | R1- | 11 | 1,021 | 42 | 1,021 | 95 | 109 |
| | | | R1+ | 6,782 | 14,968 | 8,920 | 6,102 | 2,861 | 629 |
| | | 24 hours after sensitization | R1- | 98 | 1,538 | 44 | 1,538 | 85 | 131 |
| | | | R1+ | 6,238 | 14,196 | 8,862 | 5,969 | 2,468 | 735 |
| | | 72 hours after sensitization | R1- | 301 | 2,197 | -31 | 2,197 | 49 | 127 |
| | | | R1+ | 5,725 | 14,085 | 8,876 | 6,002 | 2,597 | 694 |

| | | |
|---|---|---|
| Human normal specimen: | Less than 50: | excellent |
| *Nonspecific adsorption standards | 50 or more and less than 100: | good |
| | 100 or more and less than 500: | fair |
| | 500 or more: | bad |
| Reactivity 2 R1-: | Less than 50: | excellent |
| *Nonspecific adsorption standards | 50 or more and less than 100: | good |
| | 100 or more and less than 500: | fair |
| | 500 or more: | bad |
| Reactivity 2 R1+: | 10,000 or more: | excellent |
| | 8,000 or more and less than 10,000: | good |
| | 5,000 or more and less than 8,000: | fair |
| | Less than 5,000: | bad |

[Example 1-25]

(Production of Affinity Particles for KL-6)

An anti-KL-6 antibody was sensitized to the particles 1-5, the particles 1-6, and the particles 1-7 in the same manner as in Example 1-22 except that the antibody of Example 1-22 was changed from the anti-CRP antibody to the anti-KL-6 antibody, and the loading amount of the antibody was changed from 25 to 100. The resultant affinity particles for KL-6 are represented as "affinity particles 1-5K", "affinity particles 1-6K", and "affinity particles 1-7K", respectively.

The antibody sensitization ratios of the affinity particles 1-5K, the affinity particles 1-6K, and the affinity particles 1-7K were evaluated in the same manner as in Example 1-23. As a result, the antibody sensitization ratios were found to be 93%, 100%, and 94%, respectively.

[Example 1-26]

(Evaluation of Latex Agglutination Sensitivity to KL-6)

The latex agglutination sensitivities of the affinity particles 1-5K, the affinity particles 1-6K, and the affinity particles 1-7K were evaluated in the same manner as in Example 1-24 except that the human CRP of Example 1-24 was changed to human KL-6.

Absorbance variations (ΔABS×10,000) obtained with the affinity particles 1-5K are as shown below.

0 U/mL (physiological saline): -140
500 U/mL: 410
1,000 U/mL: 1,380
2,000 U/mL: 4,840
5,000 U/mL: 7,520

Absorbance variations (ΔABS×10,000) obtained with the affinity particles 1-6K are as shown below.

0 U/mL (physiological saline): -240
500 U/mL: -90
1,000 U/mL: 480
2,000 U/mL: 3,320
5,000 U/mL: 6,160

Absorbance variations (ΔABS×10,000) obtained with the affinity particles 1-7K are as shown below.

0 U/mL (physiological saline): -320
500 U/mL: -50
1,000 U/mL: -340
2,000 U/mL: 550
5,000 U/mL: 1,200

All the particles gave linear absorbance variations with respect to the concentration of KL-6. It was found that, when the concentration of KL-6 was constant, as the particle diameter became larger, the absorbance variation increased. The latex agglutination sensitivity can be controlled by controlling the particle diameter.

[Example 1-27]

(Effect of Sensitizer on Latex Agglutination Sensitivity)

The latex agglutination sensitivities of the affinity particles 1-5K and the affinity particles 1-6K were evaluated in the same manner as in Example 1-24 except that the human CRP of Example 1-24 was changed to human KL-6, and the buffer (PBS containing 0.01% Tween 20) was changed to a buffer containing a sensitizer (PBS containing 0.58% PVP K90 or 0.68% sodium alginate 80-120 and containing 0.01% Tween 20).

Absorbance variations (ΔABS×10,000) obtained with the affinity particles 1-5K in the case of using alginic acid as the sensitizer are as shown below.

0 U/mL (physiological saline): -240
500 U/mL: 1,090
1,000 U/mL: 2,780
2,000 U/mL: 9,960
5,000 U/mL: 11,520

Absorbance variations (ΔABS×10,000) obtained with the affinity particles 1-6K in the case of using alginic acid as the sensitizer are as shown below.

0 U/mL (physiological saline): -50
500 U/mL: 420
1,000 U/mL: 1,260
2,000 U/mL: 6,590
5,000 U/mL: 8,720

Absorbance variations (ΔABS×10,000) obtained with the affinity particles 1-5K in the case of using PVP as the sensitizer are as shown below.

0 U/mL (physiological saline): -70
500 U/mL: 480
1,000 U/mL: 1,020
2,000 U/mL: 3,860
5,000 U/mL: 5,970

Absorbance variations (ΔABS×10,000) obtained with the affinity particles 1-6K in the case of using PVP as the sensitizer are as shown below.

0 U/mL (physiological saline): -280
500 U/mL: -60
1,000 U/mL: 670
2,000 U/mL: 4,080
5,000 U/mL: 6,820

The sensitizing effects of PVP and alginic acid were recognized for the affinity particles 1-5K and the affinity particles 1-6K. In particular, alginic acid was found to have a high sensitizing effect.

[Example 1-28]

(Production of Affinity Particles for IgE)

An anti-IgE antibody was sensitized to the particles 1-5 in the same manner as in Example 1-22 except that the antibody of Example 1-22 was changed from the anti-CRP antibody to the anti-IgE antibody, and the loading amount of the antibody was changed from 25 to 100. The resultant affinity particles for IgE are represented as "affinity particles 1-5I".

The antibody sensitization ratio of the affinity particles 1-5I was evaluated in the same manner as in Example 1-23. As a result, the antibody sensitization ratio was found to be 80%.

[Example 1-29]

(Evaluation of Latex Agglutination Sensitivity to IgE)

The latex agglutination sensitivity of the affinity particles 1-5I was evaluated in the same manner as in Example 1-24 except that the human CRP of Example 1-24 was changed to human IgE. The amount of the specimen was set to 1.5 μL, the amount of the specimen diluent (Good's buffer, LT Auto Wako IgE, Wako Pure Chemical Industries, Ltd.) was set to 75 μL, and the amount of the affinity particle dispersion was set to 25 μL.

Absorbance variations (ΔABS×10,000) obtained with the affinity particles 1-5I are as shown below.

0 IU/mL (physiological saline): -60
100 IU/mL: -50
300 IU/mL: 100
1,000 IU/mL: 200
2,000 IU/mL: 510
3,000 IU/mL: 1,790

The affinity particles 5I gave a linear absorbance variation with respect to the concentration of IgE. Further, the latex agglutination sensitivity of the affinity particles 1-5I was evaluated with a buffer containing a sensitizer (using a Good's buffer containing 0.045% alginic acid as the specimen diluent) in the same manner as in Example 1-27.

Absorbance variations (ΔABS×10,000) obtained with the affinity particles 1-5K in the case of using alginic acid as the sensitizer are as shown below.

0 IU/mL (physiological saline): 30
100 IU/mL: 140
300 IU/mL: 300
1,000 IU/mL: 1,130
2,000 IU/mL: 3,870
3,000 IU/mL: 5,910

The sensitizing effect of alginic acid was recognized for the affinity particles 1-5I.

Now, the present invention is described in detail by way of Examples corresponding to the second embodiment. However, the present invention is not limited to these Examples.

(Example 2-1: Synthesis of Particulate Copolymer)

The following materials were weighed in a 2 L four-necked separable flask to provide a mixed liquid.

·22.7 g of styrene (St, manufactured by Kishida Chemical Co., Ltd.)
·33.9 g of glycidyl methacrylate (GMA, manufactured by Tokyo Chemical Industry Co., Ltd.)
·0.86 g of divinylbenzene (DVB, manufactured by Kishida Chemical Co., Ltd.)
·2,168.6 g of ion-exchanged water

After that, the mixed liquid was held at 70°C while being stirred at 200 rpm, and nitrogen was allowed to flow at a flow rate of 200 mL/min to remove oxygen from the inside of the three-necked separable flask. Next, a separately prepared dissolved liquid, which had been obtained by dissolving 1.13 g of a polymerization initiator (product name: V-50, manufactured by FUJIFILM Wako Pure Chemical Corporation) in 30 g of ion-exchanged water, was added to the mixed liquid to initiate soap-free emulsion polymerization. Two hours after the initiation of the polymerization, 5.8 g of GMA was added to the four-necked separable flask, and the mixture was held at 70°C while being stirred for 22 hours at 200 rpm. Thus, an aqueous dispersion containing a particulate copolymer 2-1 was obtained.

After the dispersion had been gradually cooled to room temperature, part of the dispersion was collected, and its polymerization conversion ratio was evaluated by using proton NMR, gas chromatography, and gel permeation chromatography. As a result, it was recognized that the polymerization conversion ratio was substantially 100%. The particulate copolymer had a dry particle diameter of 196.6 nm and a particle diameter in water of 206.9 nm.

The particulate copolymer was subjected to ultrafiltration concentration, or was diluted by the addition of ion-exchanged water, so as to be a 2.5 mass% aqueous dispersion, and the dispersion was stored under a light-shielding condition at 4°C.

(Example 2-2: Synthesis of Particles 2-1)

The following materials were weighed in a 100 mL round-bottom flask, and triethylamine (manufactured by Kishida Chemical Co., Ltd.) was added to the mixture to adjust its pH to 10.

·24 g of the 2.5 mass% aqueous dispersion of the particulate copolymer 2-1
·3.3 g of ion-exchanged water
·202 mg (1.30 mmol) of mercaptosuccinic acid (manufactured by FUJIFILM Wako Pure Chemical Corporation)
·0.119 mL (1.30 mmol) of 3-mercapto-1,2-propanediol (manufactured by FUJIFILM Wako Pure Chemical Corporation)

Next, the temperature of the contents of the round-bottom flask was increased to 70°C while the contents were stirred at 200 rpm. Further, the contents were held in this state for 18 hours to provide a dispersion of particles 2-1. The particles 2-1 were separated from the dispersion with a centrifugal separator, and the particles 2-1 were re-dispersed in ion-exchanged water; the operation was repeated eight times to purify the particles 2-1, which were stored in the state of an aqueous dispersion in which the concentration of the particles 2-1 was finally adjusted to 1.0 mass%. Storage conditions were set to 4°C under a light-shielding condition. Table 2-1 shows a summary of the particle physical properties of the particles 2-1.

(Example 2-3: Synthesis of Particles 2-2)

The following materials were weighed in a 100 mL round-bottom flask, and triethylamine (manufactured by Kishida Chemical Co., Ltd.) was added to the mixture to adjust its pH to 10.

·24 g of the 2.5 mass% aqueous dispersion of the particulate copolymer 2-1
·3.3 g of ion-exchanged water
·40 mg (0.26 mmol) of mercaptosuccinic acid (manufactured by FUJIFILM Wako Pure Chemical Corporation)
·0.214 mL (2.34 mmol) of 3-mercapto-1,2-propanediol (manufactured by FUJIFILM Wako Pure Chemical Corporation)

Next, the temperature of the contents of the round-bottom flask was increased to 70°C while the contents were stirred at 200 rpm. Further, the contents were held in this state for 6 hours to provide a dispersion of particles 2-2. The particles 2-2 were separated from the dispersion with a centrifugal separator, and the particles 2-2 were re-dispersed in ion-exchanged water; the operation was repeated eight times to purify the particles 2-2, which were stored in the state of an aqueous dispersion in which the concentration of the particles 2-2 was finally adjusted to 1.0 mass%. Storage conditions were set to 4°C under a light-shielding condition. Table 2-1 shows a summary of the particle physical properties of the particles 2-2.

(Example 2-4: Synthesis of Particles 2-3)

The following materials were weighed in a 100 mL round-bottom flask, and triethylamine (manufactured by Kishida Chemical Co., Ltd.) was added to the mixture to adjust its pH to 10.

·24 g of the 2.5 mass% aqueous dispersion of the particulate copolymer 2-1
·3.3 g of ion-exchanged water
·40 mg (0.26 mmol) of mercaptosuccinic acid (manufactured by FUJIFILM Wako Pure Chemical Corporation)
·0.214 mL (2.34 mmol) of 3-mercapto-1,2-propanediol (manufactured by FUJIFILM Wako Pure Chemical Corporation)

Next, the temperature of the contents of the round-bottom flask was increased to 70°C while the contents were stirred at 200 rpm. Further, the contents were held in this state for 8 hours to provide a dispersion of particles 2-3. The particles 2-3 were separated from the dispersion with a centrifugal separator, and the particles 2-3 were re-dispersed in ion-exchanged water; the operation was repeated eight times to purify the particles 2-3, which were stored in the state of an aqueous dispersion in which the concentration of the particles 2-3 was finally adjusted to 1.0 mass%. Storage conditions were set to 4°C under a light-shielding condition. Table 2-1 shows a summary of the particle physical properties of the

particles 2-3.

(Example 2-5: Synthesis of Particles 2-4)

The following materials were weighed in a 100 mL round-bottom flask, and triethylamine (manufactured by Kishida Chemical Co., Ltd.) was added to the mixture to adjust its pH to 10.

·24 g of the 2.5 mass% aqueous dispersion of the particulate copolymer 2-1
·3.3 g of ion-exchanged water
·40 mg (0.26 mmol) of mercaptosuccinic acid (manufactured by FUJIFILM Wako Pure Chemical Corporation)
·0.214 mL (2.34 mmol) of 3-mercapto-1,2-propanediol (manufactured by FUJIFILM Wako Pure Chemical Corporation)

Next, the temperature of the contents of the round-bottom flask was increased to 70°C while the contents were stirred at 200 rpm. Further, the contents were held in this state for 12 hours to provide a dispersion of particles 2-4. The particles 2-4 were separated from the dispersion with a centrifugal separator, and the particles 2-4 were re-dispersed in ion-exchanged water; the operation was repeated eight times to purify the particles 2-4, which were stored in the state of an aqueous dispersion in which the concentration of the particles 2-4 was finally adjusted to 1.0 mass%. Storage conditions were set to 4°C under a light-shielding condition. Table 2-1 shows a summary of the particle physical properties of the particles 2-4.

(Example 2-6: Synthesis of Particles 2-5)

The following materials were weighed in a 100 mL round-bottom flask, and triethylamine (manufactured by Kishida Chemical Co., Ltd.) was added to the mixture to adjust its pH to 10.

·24 g of the 2.5 mass% aqueous dispersion of the particulate copolymer 2-1
·3.3 g of ion-exchanged water
·40 mg (0.26 mmol) of mercaptosuccinic acid (manufactured by FUJIFILM Wako Pure Chemical Corporation)
·0.214 mL (2.34 mmol) of 3-mercapto-1,2-propanediol (manufactured by FUJIFILM Wako Pure Chemical Corporation)

Next, the temperature of the contents of the round-bottom flask was increased to 70°C while the contents were stirred at 200 rpm. Further, the contents were held in this state for 18 hours to provide a dispersion of particles 2-5. The particles 2-5 were separated from the dispersion with a centrifugal separator, and the particles 2-5 were re-dispersed in ion-exchanged water; the operation was repeated eight times to purify the particles 2-5, which were stored in the state of an aqueous dispersion in which the concentration of the particles 2-5 was finally adjusted to 1.0 mass%. Storage conditions were set to 4°C under a light-shielding condition. Table 2-1 shows a summary of the particle physical properties of the particles 2-5.

(Example 2-7: Synthesis of Particles 2-6)

The following materials were weighed in a 100 mL round-bottom flask, and triethylamine (manufactured by Kishida Chemical Co., Ltd.) was added to the mixture to adjust its pH to 11.

·24 g of the 2.5 mass% aqueous dispersion of the particulate copolymer 2-1
·3.3 g of ion-exchanged water
·40 mg (0.26 mmol) of mercaptosuccinic acid (manufactured by FUJIFILM Wako Pure Chemical Corporation)
·0.214 mL (2.34 mmol) of 3-mercapto-1,2-propanediol (manufactured by FUJIFILM Wako Pure Chemical Corporation)

Next, the temperature of the contents of the round-bottom flask was increased to 70°C while the contents were stirred at 200 rpm. Further, the contents were held in this state for 30 hours to provide a dispersion of particles 2-6. The particles 2-6 were separated from the dispersion with a centrifugal separator, and the particles 2-6 were re-dispersed in ion-exchanged water; the operation was repeated eight times to purify the particles 2-6, which were stored in the state of an aqueous dispersion in which the concentration of the particles 2-6 was finally adjusted to 1.0 mass%. Storage conditions were set to 4°C under a light-shielding condition. Table 2-1 shows a summary of the particle physical properties of the particles 2-6.

(Example 2-8: Synthesis of Particles 2-7)

The following materials were weighed in a 100 mL round-bottom flask, and triethylamine (manufactured by Kishida Chemical Co., Ltd.) was added to the mixture to adjust its pH to 10.

·24 g of the 2.5 mass% aqueous dispersion of the particulate copolymer 2-1
·3.3 g of ion-exchanged water
·81 mg (0.52 mmol) of mercaptosuccinic acid (manufactured by FUJIFILM Wako Pure Chemical Corporation)
·0.191 mL (2.08 mmol) of 3-mercapto-1,2-propanediol (manufactured by FUJIFILM Wako Pure Chemical Corporation)

Next, the temperature of the contents of the round-bottom flask was increased to 70°C while the contents were stirred at 200 rpm. Further, the contents were held in this state for 18 hours to provide a dispersion of particles 2-7. The particles 2-7 were separated from the dispersion with a centrifugal separator, and the particles 2-7 were re-dispersed in ion-exchanged water; the operation was repeated eight times to purify the particles 2-7, which were stored in the state of an aqueous dispersion in which the concentration of the particles 2-7 was finally adjusted to 1.0 mass%. Storage conditions were set to 4°C under a light-shielding condition. Table 2-1 shows a summary of the particle physical properties of the particles 2-7.

(Example 2-9: Synthesis of Particles 2-8)

The following materials were weighed in a 100 mL round-bottom flask, and triethylamine (manufactured by Kishida Chemical Co., Ltd.) was added to the mixture to adjust its pH to 10.

·24 g of the 2.5 mass% aqueous dispersion of the particulate copolymer 2-1
·3.3 g of ion-exchanged water
·323 mg (2.08 mmol) of mercaptosuccinic acid (manufactured by FUJIFILM Wako Pure Chemical Corporation)
·0.047 mL (0.52 mmol) of 3-mercapto-1,2-propanediol (manufactured by FUJIFILM Wako Pure Chemical Corporation)

Next, the temperature of the contents of the round-bottom flask was increased to 70°C while the contents were stirred at 200 rpm. Further, the contents were held in this state for 18 hours to provide a dispersion of particles 2-8. The particles 2-8 were separated from the dispersion with a centrifugal separator, and the particles 2-8 were re-dispersed in ion-exchanged water; the operation was repeated eight times to purify the particles 2-8, which were stored in the state of an aqueous dispersion in which the concentration of the particles 2-8 was finally adjusted to 1.0 mass%. Storage conditions were set to 4°C under a light-shielding condition. Table 2-1 shows a summary of the particle physical properties of the particles 2-8.

(Example 2-10: Synthesis of Particles 2-9)

The following materials were weighed in a 100 mL round-bottom flask, and triethylamine (manufactured by Kishida Chemical Co., Ltd.) was added to the mixture to adjust its pH to 10.

·24 g of the 2.5 mass% aqueous dispersion of the particulate copolymer 2-1
·3.3 g of ion-exchanged water
·282 mg (1.82 mmol) of mercaptosuccinic acid (manufactured by FUJIFILM Wako Pure Chemical Corporation)
·0.071 mL (0.78 mmol) of 3-mercapto-1,2-propanediol (manufactured by FUJIFILM Wako Pure Chemical Corporation)

Next, the temperature of the contents of the round-bottom flask was increased to 70°C while the contents were stirred at 200 rpm. Further, the contents were held in this state for 18 hours to provide a dispersion of particles 2-9. The particles 2-9 were separated from the dispersion with a centrifugal separator, and the particles 2-9 were re-dispersed in ion-exchanged water; the operation was repeated eight times to purify the particles 2-9, which were stored in the state of an aqueous dispersion in which the concentration of the particles 2-9 was finally adjusted to 1.0 mass%. Storage conditions were set to 4°C under a light-shielding condition. Table 2-1 shows a summary of the particle physical properties of the particles 2-9.

(Example 2-11: Synthesis of Particles 2-10)

The following materials were weighed in a 100 mL round-bottom flask, and triethylamine (manufactured by Kishida Chemical Co., Ltd.) was added to the mixture to adjust its pH to 10.

·24 g of the 2.5 mass% aqueous dispersion of the particulate copolymer 2-1
·3.3 g of ion-exchanged water
·242 mg (1.56 mmol) of mercaptosuccinic acid (manufactured by FUJIFILM Wako Pure Chemical Corporation)
·0.095 mL (1.04 mmol) of 3-mercapto-1,2-propanediol (manufactured by FUJIFILM Wako Pure Chemical Corporation)

Next, the temperature of the contents of the round-bottom flask was increased to 70°C while the contents were stirred at 200 rpm. Further, the contents were held in this state for 18 hours to provide a dispersion of particles 2-10. The particles 2-10 were separated from the dispersion with a centrifugal separator, and the particles 2-10 were re-dispersed in ion-exchanged water; the operation was repeated eight times to purify the particles 2-10, which were stored in the state of an aqueous dispersion in which the concentration of the particles 2-10 was finally adjusted to 1.0 mass%. Storage conditions were set to 4°C under a light-shielding condition. Table 2-1 shows a summary of the particle physical properties of the particles 2-10.

(Example 2-12: Production of Affinity Particles 2-1 to 2-6 by Antibody Sensitization to Particles)

0.1 mL (1 mg in terms of particles) of the particle dispersion (solution having a concentration of 1.0 mass%, 10 mg/mL) of each of the particles 2-1 to 2-6 was transferred to a microtube (volume: 1.5 mL). After that, 0.12 mL of an activation buffer (25 mM MES, pH: 6.0) was added thereto, and the mixture was centrifuged at 4°C and 15,000 rpm (20,400 g) for 5 minutes. After the centrifugation, the supernatant was discarded. 0.12 mL of an activation buffer (25 mM MES, pH: 6.0) was added to the residue, and the particles were redispersed with an ultrasonic wave. The centrifugation and the re-dispersion were repeated once.

Next, 60 μL each of the following materials were added to the resultant, and were dispersed therein with an ultrasonic wave.

·A 1-[3-(dimethylaminopropyl)-3-ethylcarbodiimide] hydrochloride (WSC) solution (solution obtained by dissolving 50 mg of WSC in 1 mL of an activation buffer)
·A sulfo-N-hydroxysuccinimide (SulfoNHS) solution (solution obtained by dissolving 50 mg of SulfoNHS in 1 mL of an activation buffer)

The dispersion was stirred at room temperature for 30 minutes to transform the carboxy groups of its particles into active esters. The resultant was centrifuged at 4°C and 15,000 rpm (20,400 g) for 5 minutes, and the supernatant was discarded. 0.2 mL of an immobilization buffer (25 mM MES, pH: 5.0) was added to the residue, and the particles were dispersed with an ultrasonic wave. The dispersion was centrifuged at 4°C and 15,000 rpm (20,400 g) for 5 minutes, and the supernatant was discarded. 50 μL of the immobilization buffer was added to the residue, and the particles whose carboxy groups had been activated were dispersed with an ultrasonic wave.

50 μL of an antibody solution (solution obtained by diluting an anti-C reactive protein (CRP) antibody with the immobilization buffer so that its concentration became 25 μg/50 μL) was added to 50 μL of the solution of the particles whose carboxy groups had been activated, and the particles were dispersed with an ultrasonic wave. The loading amount of the antibody is 25 μg per 1 mg of the particles (25 μg/mg). An antibody final concentration is 0.25 mg/mL, and a particle final concentration is 10 mg/mL. The contents of the tube were stirred at room temperature for 60 minutes to bond the antibody to the carboxy groups of the particles.

Next, the resultant was centrifuged at 4°C and 15,000 rpm (20,400 g) for 5 minutes, and the supernatant was discarded. 0.24 mL of a masking buffer (buffer obtained by incorporating 0.1% Tween 20 into 1 M Tris having a pH of 8.0) was added to the residue, and the particles were dispersed with an ultrasonic wave. The dispersion was stirred at room temperature for 1 hour, and was then left at rest at 4°C overnight to bond trishydroxymethylaminomethane (Tris) to the remaining activated carboxy groups. Next, the resultant was centrifuged at 4°C and 15,000 rpm (20,400 g) for 5 minutes, and the supernatant was discarded. 0.2 mL of a washing buffer (10 mM hydroxyethylpiperazineethanesulfonic acid (HEPES), pH: 7.9) was added to the residue, and the particles were dispersed with an ultrasonic wave. The washing operation (the centrifugation and the re-dispersion) with the washing buffer (10 mM HEPES, pH: 7.9) was repeated once. A washing operation was performed with 0.2 mL of a storage buffer (10 mM HEPES, pH: 7.9, containing 0.01% Tween 20) once. 1.0 mL of the storage buffer was added to the washed product, and the particles were dispersed with an ultrasonic wave. The particle concentration of the dispersion finally became 0.1 mass% (1 mg/mL). The resultant dispersion of the

particles was stored in a refrigerator.

The affinity particles obtained in this Example are named as described below. When the particles used are the particles 2-1, the affinity particles are represented as "affinity particles 2-1". In addition, when the particles used are the particles 2-2, the affinity particles are represented as "affinity particles 2-2". When the particles used are the particles 2-3, the affinity particles are represented as "affinity particles 2-3". When the particles used are the particles 2-4, the affinity particles are represented as "affinity particles 2-4". When the particles used are the particles 2-5, the affinity particles are represented as "affinity particles 2-5". When the particles used are the particles 2-6, the affinity particles are represented as "affinity particles 2-6". Table 2-1 shows a summary of the particle physical properties of the affinity particles 2-1 to 2-6.

(Example 2-13: Production of Affinity Particles 2-7 to 2-12 by Antibody Sensitization to Particles)

The particles used were changed to the particles 2-5, and the loading amount of the antibody of Example 2-12 was changed from 25 μg/mg to 48 μg/mg, 41 μg/mg, 31 μg/mg, 25 μg/mg, 18 μg/mg, and 11 μg/mg, respectively. In the same manner as in Example 2-12 except for the foregoing, 0.1 mass% aqueous dispersions of affinity particles 2-7, affinity particles 2-8, affinity particles 2-9, affinity particles 2-10, affinity particles 2-11, and affinity particles 2-12 were obtained. Table 2-1 shows a summary of the particle physical properties of the affinity particles 2-7 to 2-12.

(Example 2-14: Production of Affinity Particles 2-13 by Antibody Sensitization to Particles)

A 0.1 mass% aqueous dispersion of affinity particles 2-13 was obtained in the same manner as in Example 2-12 except that the particles used were changed to the particles 2-7, and the loading amount of the antibody of Example 2-12 was changed from 25 μg/mg to 54 μg/mg. Table 2-1 shows a summary of the particle physical properties of the affinity particles 2-13.

(Example 2-15: Production of Affinity Particles 2-14 by Antibody Sensitization to Particles)

The particles used were changed to the particles 2-5, the antibody of Example 2-12 was changed from the anti-CRP antibody to an anti-prostate-specific antigen (PSA) antibody, and the loading amount of the antibody of Example 2-12 was changed from 25 μg/mg to 20 μg/mg. In the same manner as in Example 2-12 except for the foregoing, a 0.1 mass% aqueous dispersion of affinity particles 2-14 was obtained. Table 2-1 shows a summary of the particle physical properties of the affinity particles 2-14.

(Example 2-16: Production of Affinity Particles 2-15 by Antibody Sensitization to Particles)

The particles used were changed to the particles 2-5, the antibody of Example 2-12 was changed from the anti-CRP antibody to an anti-bovine serum albumin (BSA) antibody, and the loading amount of the antibody of Example 2-12 was changed from 25 μg/mg to 23 μg/mg. In the same manner as in Example 2-12 except for the foregoing, a 0.1 mass% aqueous dispersion of affinity particles 2-15 was obtained. Table 2-1 shows a summary of the particle physical properties of the affinity particles 2-15.

(Comparative Example 2-1: Production of Affinity Particles 2-16 to 2-18 by Antibody Sensitization to Particles)

0.1 mass% aqueous dispersions of affinity particles 2-16, affinity particles 2-17, and affinity particles 2-18 were obtained in the same manner as in Example 2-12 except that the particles used of Example 2-12 were changed to the particles 2-8, the particles 2-9, and the particles 2-10, respectively. Table 2-1 shows a summary of the particle physical properties of the affinity particles 2-16 to 2-18.

(Comparative Example 2-2: Production of Affinity Particles 2-19 by Antibody Sensitization to Particles)

A 0.1 mass% aqueous dispersion of affinity particles 2-19 was obtained in the same manner as in Example 2-12 except that the particles used were changed to the particles 2-5, and the loading amount of the antibody of Example 2-12 was changed from 25 μg/mg to 5 μg/mg. Table 2-1 shows a summary of the particle physical properties of the affinity particles 2-19.

(Comparative Example 2-3: Production of Affinity Particles 2-20 and 2-21 by Antibody Sensitization to Particles)

0.1 mass% aqueous dispersions of affinity particles 2-20 and 2-21 were obtained in the same manner as in Example 2-12 except that the particles used were changed to the particles 2-5, and the loading amount of the antibody of

Example 2-12 was changed from 25 μg/mg to 64 μg/mg and 56 μg/mg, respectively. Table 2-1 shows a summary of the particle physical properties of the affinity particles 2-20 and 2-21.

(Example 2-17: Evaluation of Antibody Sensitization Ratio of Affinity Particles)

The antibody sensitization ratios (%) of the affinity particles 2-1 to 2-21 produced in Examples 2-12 to 2-16 and Comparative Examples 2-1 to 2-3 were determined by protein determination. Herein, the term "antibody sensitization ratio (%)" means the ratio of the amount of the antibody bonded to the particles to the amount of the antibody used in the reaction with the particles (antibody loading amount). An evaluation example of the protein determination is described below.

First, 7 mL of the liquid A of PROTEIN ASSAY BCA KIT (manufactured by FUJIFILM Wako Pure Chemical Corporation) and 140 μL of the liquid B were mixed, and the prepared liquid was adopted as a liquid AB. Next, 25 μL (particle amount: 25 μg) of the dispersion (0.1% solution) of the affinity particles was taken, and was loaded into a 1.5 mL tube. Next, 200 μL of the liquid AB was added to the dispersion (25 μL), and the mixture was incubated at 60°C for 30 minutes. The resultant solution was centrifuged at 4°C and 15,000 rpm (20,400 g) for 5 minutes, and 200 μL of the supernatant was loaded into a 96-well microwell with a pipetter. The absorbance of the supernatant at a wavelength of 562 nm was measured with a microplate reader together with standard samples (several samples were obtained by diluting the antibody with 10 mM HEPES so that its concentration fell within the range of from 0 μg/mL to 200 μg/mL). The amount of the antibody was calculated from a standard curve. The amount of the antibody sensitized to the particles (the amount of the bonded antibody per weight of the particles (μg/mg)) was determined by dividing the calculated antibody amount by the weight of the particles (herein, 0.025 mg). Finally, the sensitization ratio was calculated. In the case where the antibody loading amount is 25 μg per 1 mg of the particles, when the antibody sensitization amount is 12.5 μg/mg, the sensitization ratio is 50%. The results are summarized in Table 2-1.

(Example 2-18: Evaluation of Nonspecific Adsorption to Affinity Particles using Chyle Liquid)

The affinity particles 2-1 to 2-21 were each dispersed in a phosphate buffer at 0.1 mass% to prepare a dispersion (liquid A). Next, 60 μL of a chyle liquid (liquid B) formed of triolein, lecithin, free fatty acids, bovine albumin, and a Tris buffer was added to 30 μL of each dispersion, and the absorbance of the mixed liquid immediately after its stirring at a wavelength of 572 nm was measured. A spectrophotometer GeneQuant 1300 manufactured by Biochrom was used in the absorbance measurement. Then, each mixed liquid was left at rest at 37°C for 5 minutes, and then its absorbance at a wavelength of 572 nm was measured again, followed by the calculation of the value "variation ΔABS in absorbance×10,000". The results are summarized in Table 2-1.

It is understood that, as the value becomes larger, nonspecific adsorption occurs to a larger extent. However, osmotic pressure agglutination may have been detected instead of agglutination resulting from nonspecific adsorption. In any case, when affinity particles having a large value of the "variation ΔABS in absorbance×10,000" in this evaluation are used as particles for the latex agglutination method in the specimen test, concern is raised in that a normal specimen is interpreted as being false positive owing to noise. The threshold values for excellent, good, fair, and bad in Table 2-1 are criteria determined from noise risks in the detection of a target substance having a low concentration by the latex agglutination method.

(Example 2-19: Evaluation of Dispersion Stability of Affinity Particles)

The affinity particles 2-1 to 2-21 were each sufficiently dispersed with an ultrasonic wave, and then stored at 4°C. The settlement of the particles was visually observed over time. The results are summarized in Table 2-1. As described in the embodiments, particles having a small zeta potential difference, or affinity particles falling within an appropriate range in terms of occupied area ratio of the antibody had satisfactory dispersion stability. The threshold values for excellent, good, fair, and bad in Table 2-1 are criteria determined from the storage period and particle size of a reagent to be used in the latex agglutination method (because, with the solution composition of this Example, the particles inevitably undergo natural settlement, though slowly).

(Example 2-20: Evaluation 1 of Latex Agglutination Sensitivity of Affinity Particles)

1 μL of human CRP (manufactured by Denka Seiken Co., Ltd., derived from human plasma, 40 μg/ml) and 50 μL of a buffer (phosphate buffered saline (PBS) containing 0.01% Tween 20) were mixed to prepare a mixed liquid (hereinafter represented as "R1+"), and its temperature was kept at 37°C. In addition, 1 μL of physiological saline and 50 μL of the buffer (PBS containing 0.01% Tween 20) were mixed to prepare a mixed liquid (hereinafter represented as "R1-") as a control, and its temperature was similarly kept at 37°C. Next, 50 μL of each of the solutions containing the affinity particles 2-1 to 2-13 and the affinity particles 2-16 to 2-21 prepared in Examples and Comparative Examples (particle concentration:

0.1 mass%, referred to as "R2") was mixed with R1+ or R1-, and the mixture was stirred. The absorbance of the mixed liquid (volume: 101 μL) immediately after the mixing and stirring at a wavelength of 572 nm was measured. A spectrophotometer GeneQuant 1300 manufactured by Biochrom was used in the absorbance measurement. Then, the mixed liquid was left at rest at 37°C for 5 minutes, and then its absorbance at a wavelength of 572 nm was measured again, followed by the calculation of the value "variation ΔABS in absorbance×10,000". This series of evaluations was performed for each of: R2 immediately after its preparation in each of Examples and Comparative Examples; R2 after a lapse of 24 hours from the preparation; and R2 after a lapse of 72 hours from the preparation. The results are summarized in Table 2-1. A larger value of the R- in Table 2-1 means that agglutination resulting from nonspecific adsorption or osmotic pressure agglutination occurs in the affinity particles to a larger extent. Accordingly, when the particles are used as particles for the latex agglutination method in the specimen test, concern is raised in that a normal specimen is interpreted as being false positive owing to noise. Meanwhile, affinity particles having a larger value of the R+ in Table 2-1 are expected to be capable of detecting a target substance with higher sensitivity when used as affinity particles for the latex agglutination method in the specimen test. The threshold values for excellent, good, fair, and bad in R- in Table 2-1 are criteria determined from noise risks in the detection of a target substance having a low concentration by the latex agglutination method. In addition, the threshold values for excellent, good, fair, and bad in R+ are criteria determined with reference to the following values obtained by using CRP-L Auto "TBA" as a kit for CRP detection and a CRP standard solution "TBA" for Latex, which are manufactured by Denka Seiken Co., Ltd.

ΔABS×10,000 of 0 μg/ml (physiological saline): -80
ΔABS×10,000 of 5 μg/ml: 1,410
ΔABS×10,000 of 20 μg/ml: 3,530
ΔABS×10,000 of 40 μg/ml: 5,130
ΔABS×10,000 of 160 μg/ml: 9,750
ΔABS×10,000 of 320 μg/ml: 12,150

(Example 2-21: Evaluation 2 of Latex Agglutination Sensitivity of Affinity Particles)

The latex agglutination sensitivity of the affinity particles 2-14 was evaluated in the same manner as in Example 2-20 except that the human CRP of Example 2-20 was changed to PSA, and the affinity particles were changed to the affinity particles 2-14. The results are shown in Table 2-1. It was found that the affinity particles 2-14 were affinity particles having high sensitivity to PSA like the affinity particles 2-1 to 2-13 having high sensitivity to CRP.

(Example 2-22: Evaluation 3 of Latex Agglutination Sensitivity of Affinity Particles)

The latex agglutination sensitivity of the affinity particles 2-15 was evaluated in the same manner as in Example 2-20 except that the human CRP of Example 2-20 was changed to BSA, and the affinity particles were changed to the affinity particles 2-15. The results are shown in Table 2-1. It was found that the affinity particles 2-15 were affinity particles having high sensitivity to BSA like the affinity particles 2-1 to 2-13 having high sensitivity to CRP.

[Table 2-1]

Table 2-1. Summary of Dispersion Stabilities, Nonspecific Adsorptivities, and Latex Agglutination Sensitivities of Affinity Particles

| | Example | | | | | | | | | | | | | | |
|---|---|---|---|---|---|---|---|---|---|---|---|---|---|---|---|
| | Affinity particles 2-1 | Affinity particles 2-2 | Affinity particles 2-3 | Affinity particles 2-4 | Affinity particles 2-5 | Affinity particles 2-6 | Affinity particles 2-7 | Affinity particles 2-8 | Affinity particles 2-9 | Affinity particles 2-10 | Affinity particles 2-11 | Affinity particles 2-12 | Affinity particles 2-13 | Affinity particles 2-14 | Affinity particles 2-15 |
| Particles used | Particles 2-1 | Particles 2-2 | Particles 2-3 | Particles 2-4 | Particles 2-5 | Particles 2-6 | Particles 2-5 | Particles 2-5 | Particles 2-5 | Particles 2-5 | Particles 2-5 | Particles 2-5 | Particles 2-7 | Particles 2-5 | Particles 2-5 |
| Antibody used | CRP | CRP | CRP | CRP | CRP | CRP | CRP | CRP | CRP | CRP | CRP | CRP | CRP | PSA | BSA |
| (1) Particle diameter in water (nm) of particulate copolymer | 196.6 | 196.6 | 196.6 | 196.6 | 196.6 | 196.6 | 196.6 | 196.6 | 196.6 | 196.6 | 196.6 | 196.6 | 196.6 | 196.6 | 196.6 |
| (2) Dry particle diameter (nm) of particulate copolymer | 206.9 | 206.9 | 206.9 | 206.9 | 206.9 | 206.9 | 206.9 | 206.9 | 206.9 | 206.9 | 206.9 | 206.9 | 206.9 | 206.9 | 206.9 |
| (3) Dry particle diameter (nm) of particles | 207.2 | 206.2 | 205.3 | 205.8 | 205.6 | 205.7 | 205.6 | 205.6 | 205.6 | 205.6 | 205.6 | 205.6 | 198 | 205.6 | 205.6 |
| (4) Particle diameter in water (nm) of particles | 256.9 | 237.1 | 242.3 | 247 | 257 | 279.8 | 257 | 257 | 257 | 257 | 257 | 257 | 213.8 | 257 | 257 |
| (4)-(2) (nm) | 50 | 30.2 | 35.4 | 40.1 | 50.1 | 72.9 | 50.1 | 50.1 | 50.1 | 50.1 | 50.1 | 50.1 | 6.9 | 50.1 | 50.1 |
| (4)-(3) (nm) | 49.7 | 30.9 | 37 | 41.2 | 51.4 | 74.1 | 51.4 | 51.4 | 51.4 | 51.4 | 51.4 | 51.4 | 15.8 | 51.4 | 51.4 |
| (4)/(3) | 1.24 | 1.15 | 1.18 | 1.2 | 1.25 | 1.36 | 1.25 | 1.25 | 1.25 | 1.25 | 1.25 | 1.25 | 1.08 | 1.25 | 1.25 |
| Zeta potential (mV) of particles | -29 | -15 | -14 | -17 | -16 | -18 | -16 | -16 | -16 | -16 | -16 | -16 | -29 | -16 | -16 |
| Zeta potential (mV) of antibody | -9.0 | -9.0 | -9.0 | -9.0 | -9.0 | -9.0 | -9.0 | -9.0 | -9.0 | -9.0 | -9.0 | -9.0 | -9 | -12.0 | -11.0 |
| Zeta potential difference (mV) | 20 | 6 | 5 | 8 | 7 | 9 | 7 | 7 | 7 | 7 | 7 | 7 | 20 | 4 | 5 |
| Particle diameter in water (nm) of affinity particles | 272 | 252 | 257 | 262 | 272 | 295 | 279 | 277 | 273 | 272 | 272 | 268 | 232.8 | 297 | 291 |
| Antibody sensitization ratio (%) | 99 | 91 | 95 | 100 | 97 | 99 | 90 | 95 | 97 | 97 | 100 | 100 | 59 | 100 | 100 |
| Occupied area ratio (%) | 22.5 | 20.6 | 21.5 | 22.7 | 22.0 | 22.5 | 39 | 35 | 27 | 22.0 | 16 | 10 | 20.7 | 19 | 21 |
| Dispersion stability | good | excellent | excellent | excellent | excellent | excellent | excellent | excellent | excellent | excellent | excellent | fair | fair | excellent | excellent |
| Chyle (ΔABS×10,000) | -11 | 17 | 12 | 17 | -9 | 19 | 468 | 97 | 23 | 12 | 9 | -15 | 1,923 | 16 | 22 |

(continued)

Table 2-1. Summary of Dispersion Stabilities, Nonspecific Adsorptivities, and Latex Agglutination Sensitivities of Affinity Particles

| | | | Example | | | | | | | | | | | | | | |
|---|---|---|---|---|---|---|---|---|---|---|---|---|---|---|---|---|---|
| | | | Affinity particles 2-1 | Affinity particles 2-2 | Affinity particles 2-3 | Affinity particles 2-4 | Affinity particles 2-5 | Affinity particles 2-6 | Affinity particles 2-7 | Affinity particles 2-8 | Affinity particles 2-9 | Affinity particles 2-10 | Affinity particles 2-11 | Affinity particles 2-12 | Affinity particles 2-13 | Affinity particles 2-14 | Affinity particles 2-15 |
| Latex agglutinati on reactivity (ΔABS ×10,000) | Immediately after sensitization | R1- | 8 | 9 | 7 | 6 | 12 | 26 | 102 | 65 | 12 | 12 | 12 | 12 | 11 | 12 | 7 |
| | | R+ | 11,528 | 8,342 | 10,162 | 12,102 | 13,524 | 13,852 | 8,593 | 9,678 | 11,582 | 13,524 | 12,932 | 10,006 | 6,782 | 8,120 | 8,006 |
| | 24 hours after sensitization | R1- | 15 | 11 | -15 | -10 | 6 | 35 | 124 | 72 | 6 | 6 | 6 | 6 | 98 | 6 | 39 |
| | | R1+ | 11,593 | 8,523 | 10,098 | 12,238 | 13,259 | 13,569 | 8,829 | 9,352 | 11,185 | 13,259 | 12,611 | 10,118 | 6,238 | 8,251 | 8,102 |
| | 72 hours after sensitization | R1- | -19 | 7 | -9 | 7 | -11 | 42 | 111 | 51 | -11 | -11 | -11 | -11 | 301 | 9 | 11 |
| | | R1+ | 11,479 | 8,426 | 10,128 | 12,185 | 13,622 | 13,294 | 8,637 | 9,421 | 11,321 | 13,622 | 12,730 | 9,469 | 5,725 | 8,243 | 8,037 |

| | | | Comparative Example | | | | | |
|---|---|---|---|---|---|---|---|---|
| | | | Affinity particles 2-16 | Affinity particles 2-17 | Affinity particles 2-18 | Affinity particles 2-19 | Affinity particles 2-20 | Affinity particles 2-21 |
| Particles used | | | Particles 2-8 | Particles 2-9 | Particles 2-10 | Particles 2-5 | Particles 2-5 | Particles 2-5 |
| Antibody used | | | CRP | CRP | CRP | CRP | CRP | CRP |
| (1) Particle diameter in water (nm) of particulate copolymer | | | 196.6 | 196.6 | 196.6 | 196.6 | 196.6 | 196.6 |
| (2) Dry particle diameter (nm) of particulate copolymer | | | 206.9 | 206.9 | 206.9 | 206.9 | 206.9 | 206.9 |
| (3) Dry particle diameter (nm) of particles | | | 207.6 | 205.9 | 206.1 | 205.6 | 205.6 | 205.6 |
| (4) Particle diameter in water (nm) of particles | | | 259.5 | 259.434 | 257.625 | 257 | 257 | 257 |
| (4)-(2) (nm) | | | 52.6 | 52.534 | 50.725 | 50.1 | 50.1 | 50.1 |
| (4)-(3) (nm) | | | 51.9 | 53.534 | 51.525 | 51.4 | 51.4 | 51.4 |
| (4)/(3) | | | 1.25 | 1.26 | 1.25 | 1.25 | 1.25 | 1.25 |
| Zeta potential (mV) of particles | | | -43 | -37 | -35 | -16 | -16 | -16 |
| Zeta potential (mV) of antibody | | | -9.0 | -9.0 | -9.0 | -9.0 | -9.0 | -9.0 |
| Zeta potential difference (mV) | | | 34 | 28 | 26 | 7 | 7 | 7 |
| Particle diameter in water (nm) of affinity particles | | | 287 | 277 | 272 | 264 | 287 | 280 |
| Antibody sensitization ratio (%) | | | 95 | 99 | 100 | 100 | 85 | 87 |
| Occupied area ratio (%) | | | 21.5 | 22.5 | 22.7 | 5 | 49 | 44 |
| Dispersion stability | | | bad | bad | bad | bad | excellent | excellent |
| Chyle ($\Delta$ABS$\times$10,000) | | | 56 | 42 | 37 | -2 | 2,432 | 1,227 |
| Latex agglutination reactivity ($\Delta$ABS $\times$10,000) | Immediately after sensitization | R1- | 12 | 5 | 12 | 12 | 482 | 326 |
| | | R+ | 9,541 | 10,261 | 12,102 | 7,001 | 6,968 | 7,120 |
| | 24 hours after sensitization | R1- | 38 | -19 | -15 | 6 | 491 | 292 |
| | | R1+ | 9,657 | 10,198 | 12,238 | 6,920 | 6,793 | 7,529 |
| | 72 hours after sensitization | R1- | 21 | -12 | 17 | -11 | 456 | 301 |
| | | R1+ | 9,429 | 10,120 | 12,185 | 6,459 | 6,899 | 7,637 |

| | | |
|---|---|---|
| Dispersion stability: | Visual observation finds no settlement in 1 week: | excellent |
| | Visual observation finds no settlement in 3 days: | good |
| | Visual observation finds no settlement in 24 hours: | fair |
| | Visual observation finds settlement within 24 hours: | bad |
| Chyle: | Less than 50: | excellent |
| *Nonspecific adsorption standards | 50 or more and less than 100: | good |
| | 100 or more and less than 500: | fair |
| | 500 or more: | bad |
| R1-: | Less than 50: | excellent |
| *Nonspecific adsorption standards | 50 or more and less than 100: | good |

(continued)

| | | |
|---|---|---|
| | 100 or more and less than 500: | fair |
| | 500 or more: | bad |
| R1+: | 10,000 or more: | excellent |
| | 8,000 or more and less than 10,000: | good |
| | 5,000 or more and less than 8,000: | fair |
| | Less than 5,000: | bad |

(Example 2-23: Synthesis of Particles 2-23)

An aqueous dispersion of a particulate copolymer 2-2 was obtained in the same manner as in Example 2-1 except that, in Example 2-1, 22.7 g of St was changed to 12.0 g thereof, 33.9 g of GMA was changed to 17.9 g thereof, 0.86 g of divinylbenzene was changed to 0.45 g thereof, and the amount of GMA to be added to the three-necked separable flask 2 hours after the initiation of the polymerization was changed from 5.8 g to 3.1 g. After the dispersion had been gradually cooled to room temperature, part of the dispersion was collected, and its polymerization conversion ratio was evaluated by using proton NMR, gas chromatography, and gel permeation chromatography. As a result, it was recognized that the polymerization conversion ratio was substantially 100%. The particulate copolymer 2-2 had a dry particle diameter of 151.4 nm and a particle diameter in water of 160.2 nm. The particulate copolymer 2-2 was subjected to ultrafiltration concentration, or was diluted by the addition of ion-exchanged water, so as to be a 2.5 wt% aqueous dispersion, and the dispersion was stored under a light-shielding condition at 4°C.

A 1.0 wt% aqueous dispersion of particles 2-23 was obtained in the same manner as in Example 2-6 except that the particulate copolymer 2-1 of Example 2-6 was changed to the particulate copolymer 2-2. The particles 2-23 had a dry particle diameter of 161.6 nm, a particle diameter in water of 202 nm, and a zeta potential of -16 mV.

(Example 2-24: Synthesis of Particles 2-24)

An aqueous dispersion of a particulate copolymer 2-3 was obtained in the same manner as in Example 2-1 except that, in Example 2-1, 22.7 g of St was changed to 5.0 g thereof, 33.9 g of GMA was changed to 7.5 g thereof, 0.86 g of divinylbenzene was changed to 0.19 g thereof, and the amount of GMA to be added to the three-necked separable flask 2 hours after the initiation of the polymerization was changed from 5.8 g to 1.3 g. After the dispersion had been gradually cooled to room temperature, part of the dispersion was collected, and its polymerization conversion ratio was evaluated by using proton NMR, gas chromatography, and gel permeation chromatography. As a result, it was recognized that the polymerization conversion ratio was substantially 100%. The particulate copolymer 2-3 had a dry particle diameter of 96.8 nm and a particle diameter in water of 105.2 nm. The particulate copolymer 2-3 was subjected to ultrafiltration concentration, or was diluted by the addition of ion-exchanged water, so as to be a 2.5 wt% aqueous dispersion, and the dispersion was stored under a light-shielding condition at 4°C.

A 1.0 wt% aqueous dispersion of particles 2-24 was obtained in the same manner as in Example 2-6 except that the particulate copolymer 2-1 of Example 2-6 was changed to the particulate copolymer 2-3. The particles 2-24 had a dry particle diameter of 119 nm, a particle diameter in water of 144 nm, and a zeta potential of -15 mV.

(Example 2-25: Production of Affinity Particles 2-5K to 2-7K by Antibody Sensitization of Anti-KL-6 Antibody to Particles)

An anti-KL-6 antibody was sensitized to the particles 2-5, the particles 2-23, and the particles 2-24 in the same manner as in Example 2-12 except that the particles used of Example 2-12 were changed to the particles 2-5, the particles 2-23, and the particles 2-24, respectively, and the antibody of Example 2-12 was changed from the anti-CRP antibody to the anti-KL-6 antibody. The resultant affinity particles for KL-6 are referred to as "affinity particles 2-5K", "affinity particles 2-6K", and "affinity particles 2-7K", respectively. The antibody sensitization ratios of the affinity particles 2-5K, the affinity particles 2-6K, and the affinity particles 2-7K were evaluated in the same manner as in Example 2-17. As a result, the antibody sensitization ratios were found to be 93%, 100%, and 94%, respectively. The occupied area ratios of the antibody were 20.3%, 17.3%, and 12.4%, respectively. The zeta potential of the anti-KL-6 antibody was -14 mV. Accordingly, the zeta potential differences (mV) of the affinity particles 2-5K, the affinity particles 2-6K, and the affinity particles 2-7K were 2, 2, and 1, respectively.

(Example 2-26: Evaluation of Latex Agglutination Sensitivity of Affinity Particles to KL-6)

The latex agglutination sensitivities of the affinity particles 2-5K, the affinity particles 2-6K, and the affinity

particles 2-7K were evaluated in the same manner as in Example 2-20 except that the human CRP of Example 2-20 was changed to human KL-6.

Absorbance variations (ΔABS×10,000) obtained with the affinity particles 2-5K are as shown below.

0 U/mL (physiological saline): -140
500 U/mL: 410
1,000 U/mL: 1,380
2,000 U/mL: 4,840
5,000 U/mL: 7,520

Absorbance variations (ΔABS×10,000) obtained with the affinity particles 2-6K are as shown below.

0 U/mL (physiological saline): -240
500 U/mL: -90
1,000 U/mL: 480
2,000 U/mL: 3,320
5,000 U/mL: 6,160

Absorbance variations (ΔABS×10,000) obtained with the affinity particles 2-7K are as shown below.

0 U/mL (physiological saline): -320
500 U/mL: -50
1,000 U/mL: -340
2,000 U/mL: 550
5,000 U/mL: 1,200

All the particles gave linear absorbance variations with respect to the concentration of KL-6. It was found that, when the concentration of KL-6 was constant, as the particle diameter became larger, the absorbance variation increased. The latex agglutination sensitivity can be controlled by controlling the particle diameter.

(Example 2-27: Effect of Sensitizer on Latex Agglutination Sensitivity)

The latex agglutination sensitivities of the affinity particles 2-5K and the affinity particles 2-6K were evaluated in the same manner as in Example 2-20 except that the human CRP of Example 2-20 was changed to human KL-6, and the buffer (PBS containing 0.01% Tween 20) was changed to a buffer containing a sensitizer (PBS containing 0.58% PVP K90 or 0.68% sodium alginate 80-120 and containing 0.01% Tween 20).

Absorbance variations (ΔABS×10,000) obtained with the affinity particles 2-5K in the case of using alginic acid as the sensitizer are as shown below.

0 U/mL (physiological saline): -240
500 U/mL: 1,090
1,000 U/mL: 2,780
2,000 U/mL: 9,960
5,000 U/mL: 11,520

Absorbance variations (ΔABS×10,000) obtained with the affinity particles 2-6K in the case of using alginic acid as the sensitizer are as shown below.

0 U/mL (physiological saline): -50
500 U/mL: 420
1,000 U/mL: 1,260
2,000 U/mL: 6,590
5,000 U/mL: 8,720

Absorbance variations (ΔABS×10,000) obtained with the affinity particles 2-5K in the case of using PVP as the sensitizer are as shown below.

0 U/mL (physiological saline): -70

500 U/mL: 480
1,000 U/mL: 1,020
2,000 U/mL: 3,860
5,000 U/mL: 5,970

Absorbance variations (ΔABS×10,000) obtained with the affinity particles 2-6K in the case of using PVP as the sensitizer are as shown below.

0 U/mL (physiological saline): -280
500 U/mL: -60
1,000 U/mL: 670
2,000 U/mL: 4,080
5,000 U/mL: 6,820

The sensitizing effects of PVP and alginic acid were recognized for the affinity particles 2-5K and the affinity particles 2-6K. In particular, alginic acid was found to have a high sensitizing effect.

(Example 2-28: Production of Affinity Particles 2-5I by Antibody Sensitization of Anti-IgE Antibody to Particles)

An anti-IgE antibody was sensitized to the particles 2-5 in the same manner as in Example 2-12 except that the antibody of Example 2-12 was changed from the anti-CRP antibody to the anti-IgE antibody. The resultant affinity particles for IgE are referred to as "affinity particles 2-5I".

The antibody sensitization ratio of the affinity particles 2-5I was evaluated in the same manner as in Example 2-17. As a result, the antibody sensitization ratio was found to be 80%. The occupied area ratio of the antibody was 18%. The zeta potential of the anti-IgE antibody was -6 mV. Accordingly, the zeta potential difference (mV) of the affinity particles 2-5I was 10.

(Example 2-29: Evaluation of Latex Agglutination Sensitivity of Affinity Particles to IgE)

The latex agglutination sensitivity of the affinity particles 2-5I was evaluated in the same manner as in Example 2-20 except that the human CRP of Example 2-20 was changed to human IgE. The amount of the specimen was set to 1.5 μL, the amount of the specimen diluent (Good's buffer, LT Auto Wako IgE, Wako Pure Chemical Industries, Ltd.) was set to 75 μL, and the amount of the affinity particle dispersion was set to 25 μL.

Absorbance variations (ΔABS×10,000) obtained with the affinity particles 2-5I are as shown below.

0 IU/mL (physiological saline): -60
100 IU/mL: -50
300 IU/mL: 100
1,000 IU/mL: 200
2,000 IU/mL: 510
3,000 IU/mL: 1,790

The affinity particles 2-5I gave a linear absorbance variation with respect to the concentration of IgE.

Further, the latex agglutination sensitivity of the affinity particles 2-5I was evaluated with a buffer containing a sensitizer (using a Good's buffer containing 0.045% alginic acid as the specimen diluent) in the same manner as in Example 2-27.

Absorbance variations (ΔABS×10,000) obtained with the affinity particles 2-5K in the case of using alginic acid as the sensitizer are as shown below.

0 IU/mL (physiological saline): 30
100 IU/mL: 140
300 IU/mL: 300
1,000 IU/mL: 1,130
2,000 IU/mL: 3,870
3,000 IU/mL: 5,910

The sensitizing effect of alginic acid was recognized for the affinity particles 2-5I.

(Example 2-30: Production of Affinity Particles 2-22 by Antibody Sensitization to Particles and Evaluation thereof)

The anti-CRP antibody was sensitized to the particles 2-5 in the same manner as in Example 2-12 except that 1 M Tris in the masking buffer of Example 2-12 was changed to 1 M ethanolamine. The resultant affinity particles for CRP are referred to as "affinity particles 2-22". The difference from the affinity particles 2-5 is that part of the carboxyl groups of the repeating unit A are Tris in the affinity particles 2-5, but are ethanolamine in the affinity particles 2-22. Accordingly, the affinity particles 2-22 are identical to the affinity particles 2-5 in antibody sensitization ratio and occupied area ratio of the antibody.

The latex agglutination sensitivity of the affinity particles 2-22 was evaluated in the same manner as in Example 2-20.

Absorbance variations (ΔABS×10,000) obtained with the affinity particles 2-22 in terms of values relative to those of the affinity particles 2-5 are as shown below.

5 μg/mL: 1.11
20 μg/mL: 1.17
40 μg/mL: 1.13
160 μg/mL: 1.09
320 μg/mL: 1.10

It was found that the affinity particles 2-22 increased the absorbance variation as compared to the affinity particles 2-5. The latex agglutination sensitivity to CRP was able to be controlled by changing the chemical structure of masking to ethanolamine.

Now, the present invention is described in detail by way of Examples corresponding to the third embodiment. However, the present invention is not limited to these Examples.

(Example 3-1: Synthesis of Particles 3-1)

1.2 g of styrene (Kishida Chemical Co., Ltd.), 1.8 g of glycidyl methacrylate (Kishida Chemical Co., Ltd.), 0.04 g of divinylbenzene (Kishida Chemical Co., Ltd.), and 100 g of ion-exchanged water were weighed in a 200 ml flask to provide a mixed liquid. After that, the mixed liquid was held at 40°C while being stirred at 200 rpm, and nitrogen bubbling was performed for 30 minutes. Next, the nitrogen bubbling was replaced with a nitrogen flow. Then, a separately prepared dissolved liquid, which had been obtained by dissolving 0.06 g of V-50 (Wako Pure Chemical Industries, Ltd.) in 3 g of ion-exchanged water, was added to the mixed liquid to initiate radical polymerization (soap-free emulsion polymerization). Two hours after the initiation of the polymerization, 0.3 g of glycidyl methacrylate was added to the radical polymerization reaction field, and the mixture was held at 70°C while being stirred for 8 hours at 200 rpm, followed by gradual cooling to room temperature to provide a particle dispersion (step 1). At this time, the contents of the 200 ml flask were sampled, and their radical polymerization conversion ratio was evaluated by using proton NMR, gas chromatography, and gel permeation chromatography. As a result, it was recognized that the radical polymerization conversion ratio was substantially 100%. While the particle dispersion obtained in the step 1 was cooled and stirred in an ice bath, an aqueous solution obtained by adding 2.2 g of mercaptosuccinic acid (Wako Pure Chemical Industries, Ltd.: The total number of moles of mercaptosuccinic acid was equal to the number of moles of the glycidyl methacrylate) and 3.0 g of triethylamine to 40 g of ion-exchanged water was prepared and added dropwise to the particle dispersion. After the completion of the dropwise addition, the pH of the reaction liquid was adjusted to 10.3 by using triethylamine and a 2 N hydrochloric acid aqueous solution. After that, the resultant was increased in temperature to 70°C and held for 4 hours while being stirred to subject glycidyl methacrylate-derived epoxy groups and mercaptosuccinic acid-derived thiol groups to a chemical reaction to provide particles 3-1 having carboxy groups as reactive functional groups (step 2). No agglutinated mass or the like occurred during the chemical reaction. The particles 3-1 were purified by a centrifugal operation, and the dispersion medium was replaced with pure water before storage (the replacement of the dispersion medium was also performed by a centrifugal operation). The evaluation of the particles 3-1 through use of dynamic light scattering (DLS-8000: Otsuka Electronics Co., Ltd.) found that their number-average particle diameter was 214 nm. In addition, their carboxy group amount per unit mass was determined to be 130 [nmol/mg].

(Example 3-2: Synthesis of Particles 3-2)

Particles 3-2 having carboxy groups as reactive functional groups were obtained by the same experimental operation as in Example 3-1 except that the pH of the reaction liquid in the step 2 of Example 3-1 was changed from 10.3 to 10.7. Purification and storage methods are also the same. The evaluation of the particle diameter of the particles 3-2 through use of a dynamic light scattering method (DLS-8000: Otsuka Electronics Co., Ltd.) found that their number-average particle diameter was 226 nm. In addition, their carboxy group amount per unit mass was determined to be 200 [nmol/mg].

(Example 3-3: Synthesis of Particles 3-3)

Particles 3-3 having carboxy groups as reactive functional groups were obtained by the same experimental operation as in Example 3-1 except that 2.2 g of mercaptosuccinic acid in the step 2 of Example 3-1 was changed to 1.6 g of mercaptopropionic acid (Wako Pure Chemical Industries, Ltd.: The number of moles of mercaptopropionic acid was equal to the number of moles of the glycidyl methacrylate), and the amount of triethylamine to be added to the aqueous solution was changed to 1.6 g. As in Example 3-1, no agglutinated mass or the like occurred during the chemical reaction. Purification and storage methods are also the same. The evaluation of the particle diameter of the particles 3-3 through use of a dynamic light scattering method (DLS-8000: Otsuka Electronics Co., Ltd.) found that their number-average particle diameter was 211 nm. In addition, their carboxy group amount per unit mass was determined to be 120 [nmol/mg].

(Example 3-4: Synthesis of Particles 3-4)

Particles 3-4 having carboxy groups as reactive functional groups were obtained by the same experimental operation as in Example 3-1 except that, in the step 2 of Example 3-1, the reaction was performed without the addition of triethylamine. Purification and storage methods are also the same. The evaluation of the particle diameter of the particles 3-4 through use of a dynamic light scattering method (DLS-8000: Otsuka Electronics Co., Ltd.) found that their number-average particle diameter was 209 nm. In addition, their carboxy group amount per unit mass was determined to be 20 [nmol/mg].

(Example 3-5: Synthesis of Particles 3-5)

Particles 3-5 having carboxy groups as reactive functional groups were obtained by the same experimental operation as in Example 3-1 except that the pH of the reaction liquid in the step 2 of Example 3-1 was changed from 10.3 to 9.9. Purification and storage methods are also the same. The evaluation of the particle diameter of the particles 3-5 through use of a dynamic light scattering method (DLS-8000: Otsuka Electronics Co., Ltd.) found that their number-average particle diameter was 216 nm. In addition, their carboxy group amount per unit mass was determined to be 80 [nmol/mg].

(Example 3-6: Synthesis of Particles 3-6)

While the particles 3-5 obtained in Example 3-5 were cooled and stirred in an ice bath, an aqueous solution obtained by adding 0.9 g of aminoethanol (Wako Pure Chemical Industries, Ltd.: The total number of moles of aminoethanol was equal to the number of moles of the glycidyl methacrylate) and 0.6 g of sodium hydroxide (Kishida Chemical Co., Ltd.: in an amount equal to the number of moles of aminoethanol) to 40 g of ion-exchanged water was prepared and added dropwise to the particle dispersion. After the completion of the dropwise addition, the pH of the reaction liquid was adjusted to 10.0 by using sodium hydroxide and 1 N hydrochloric acid. After that, the resultant was increased in temperature to 70°C and held for 4 hours while being stirred to subject glycidyl methacrylate-derived epoxy groups and aminoethanol-derived amino groups to a chemical reaction to provide particles 3-6 whose epoxy groups had been ring-opened. No agglutinated mass or the like occurred during the chemical reaction. The particles 3-6 were purified by a centrifugal operation, and the dispersion medium was replaced with pure water before storage (the replacement of the dispersion medium was also performed by a centrifugal operation). The evaluation of the particles 3-6 through use of dynamic light scattering (DLS-8000: Otsuka Electronics Co., Ltd.) found that their number-average particle diameter was 214 nm. In addition, their carboxy group amount per unit mass was determined to be 80 [nmol/mg].

(Example 3-7: Synthesis of Particles 3-7)

While the particles 3-5 obtained in Example 3-5 were cooled and stirred in an ice bath, an aqueous solution obtained by adding 1.4 g of 3-mercapto-1-propanol (Wako Pure Chemical Industries, Ltd.: The total number of moles of mercaptopropanol was equal to the number of moles of the glycidyl methacrylate) and 1.5 g of triethylamine (Kishida Chemical Co., Ltd.: in an amount equal to the number of moles of 3-mercapto-1-propanol) to 40 g of ion-exchanged water was prepared and added dropwise to the particle dispersion. After the completion of the dropwise addition, the pH of the reaction liquid was adjusted to 10.0 by using triethylamine and 1 N hydrochloric acid. After that, the resultant was increased in temperature to 70°C and held for 4 hours while being stirred to subject glycidyl methacrylate-derived epoxy groups and mercaptopropanol-derived mercapto groups to a chemical reaction to provide particles 3-7 whose epoxy groups had been ring-opened. No agglutinated mass or the like occurred during the chemical reaction. The particles 3-7 were purified by a

centrifugal operation, and the dispersion medium was replaced with pure water before storage (the replacement of the dispersion medium was also performed by a centrifugal operation). The evaluation of the particles 3-7 through use of dynamic light scattering (DLS-8000: Otsuka Electronics Co., Ltd.) found that their number-average particle diameter was 220 nm. In addition, their carboxy group amount per unit mass was determined to be 80 [nmol/mg].

(Comparative Example 3-1: Synthesis of Modified SG Particles 3-1)

Modified SG particles 3-1 having carboxy groups as reactive functional groups were obtained by the same experimental operation as in Example 3-1 except that 2.2 g of mercaptosuccinic acid in the step 2 of Example 3-1 was changed to 1.1 g of glycine (Wako Pure Chemical Industries, Ltd.: The number of moles of glycine was equal to the number of moles of the glycidyl methacrylate), and the amount of triethylamine to be added to the aqueous solution was changed to 1.6 g. As in Example 3-1, no agglutinated mass or the like occurred during the chemical reaction. Purification and storage methods are also the same. The evaluation of the particle diameter of the modified SG particles 3-1 through use of a dynamic light scattering method (DLS-8000: Otsuka Electronics Co., Ltd.) found that their number-average particle diameter was 211 nm. In addition, their carboxy group amount per unit mass was determined to be 120 [nmol/mg].

(Synthesis of Affinity Particles: Bonding of Antibody)

The particles 3-1 to 3-7 and the modified SG particles 3-1 were each dispersed in a MES buffer at 1.0 wt% to prepare 1 μl each of dispersions. A dissolved liquid, which had been obtained by dissolving 0.055 mg of 1-[3-(dimethylaminopropyl)-3-ethylcarbodiimide] (Wako Pure Chemical Industries, Ltd.) in 10 μl of a phosphate buffer, was added to each of those dispersions. After that, 5 μl of a 4.9 mg/ml dispersion of clone C5 (Funakoshi Co., Ltd.) of a monoclonal mouse anti-human C-reactive protein (hereinafter referred to as "CRP antibody") and 5 μl of a 5.8 mg/ml dispersion of clone C6 (Funakoshi Co., Ltd.) thereof were added to the mixture. Then, the mixture was shaken at room temperature for 180 hours to synthesize affinity particles. Next, the affinity particles were purified by performing centrifugal purification (15,000 rpm) three times, and were finally stored in the state of being dispersed in 1 ml of a phosphate buffer. The affinity particles obtained from the particles 3-1 to 3-7 are hereinafter referred to as "affinity particles 3-1 to 3-7", respectively. In addition, the affinity particles obtained from the modified SG particles 3-1 are referred to as "CRP-immobilized modified SG particles 3-1".

The sensitization ratios of the affinity particles 3-1 to 3-7 and the CRP-immobilized modified SG particles 3-1 were evaluated based on the following standards. The results are shown in Table 3-1.

A: 80% or more
B: 60% or more and less than 80%
C: less than 60%

(Evaluation of Antigen-Antibody Reactivity to Human CRP Antigen)

1 μl of human CRP (C4063 manufactured by Sigma-Aldrich, C-reactive protein, derived from human plasma, 32 mg/dl) and 50 μl of a buffer (buffer (R-1) of CRP-L Auto "TBA", Denka Seiken Co., Ltd.) were mixed to give a mixed liquid, and the mixed liquid was warmed at 37°C for 5 minutes. Next, 50 μl of each of the dispersions of the affinity particles obtained in Examples 3-1 to 3-7 and Comparative Example 3-1 was mixed with the mixed liquid, and the absorbance of the dispersion immediately after its stirring at a wavelength of 572 nm was measured. A spectrophotometer GeneQuant 1300 manufactured by Biochrom was used in the absorbance measurement. Then, the dispersion was left at rest at 37°C for 5 minutes, and then its absorbance at a wavelength of 572 nm was measured again, followed by the calculation of a variation ΔABS in absorbance×10,000. The affinity particles 3-1 to 3-7 and the CRP-immobilized modified SG particles 3-1 were each evaluated for the value "variation ΔABS in absorbance×10,000" based on the following standards. The results are shown in Table 3-1.

A: The variation is 10,000 or more.
B: The variation is 5,000 or more and less than 10,000.
C: The variation is less than 5,000.

(Nonspecific Adsorptivity Evaluation)

In addition, nonspecific adsorptivity was evaluated by performing evaluation in the same manner except for using physiological saline instead of adding 1 μl of human CRP (C4063 manufactured by Sigma-Aldrich, C-reactive protein, derived from human plasma, 32 mg/dl), and a variation ΔABS in absorbance×10,000 was calculated. The affinity particles

3-1 to 3-7 and the CRP-immobilized modified SG particles 3-1 were each evaluated for the value "variation ΔABS in absorbance×10,000" based on the following standards. The results are shown in Table 3-1.

A: The variation is less than 1,000.
B: The variation is 1,000 or more.

[Table 3-1]

Table 3-1

| | Example 3-1 | Example 3-2 | Example 3-3 | Example 3-4 | Example 3-5 | Example 3-6 | Example 3-7 | Comparative Example 3-1 |
|---|---|---|---|---|---|---|---|---|
| Carboxylic acid amount [nmol/mg] | 130 | 200 | 120 | 20 | 80 | 80 | 80 | 120 |
| Sensitization ratio | A | A | A | B | B | B | B | C |
| CRP antigen-antibody reactivity | A | A | A | A | A | B | B | C |
| Nonspecific adsorptivity | A | A | A | A | A | A | A | B |

As described above, there can be provided particles that, by virtue of having a structure having a carboxy group in a side chain via a sulfide group, are excellent in ability to suppress nonspecific adsorption, have such a sensitization property as to be highly sensitive in the latex immunoagglutination method, and allow a ligand to be chemically bonded to the surfaces of the particles in high yield.

(Example 3-8: Synthesis of Particles 3-8 having Small Particle Diameter)

A particle dispersion was obtained in the same manner as in the step 1 of Example 3-1 except that, in Example 3-1, the amount of styrene was changed to 0.5 g, the amount of glycidyl methacrylate was changed to 0.8 g, the amount of divinylbenzene was changed to 0.02 g, and the amount of GMA to be added to the three-necked separable flask 2 hours after the initiation of the polymerization was changed to 0.13 g. Particles 3-8 having carboxy groups as reactive functional groups were obtained by the same experimental operation as in the step 2 of Example 3-1 except that the pH of the reaction liquid in the step 2 of Example 3-1 was changed from 10.3 to 10.7. The particles were subjected to centrifugal purification, and then the dispersion medium was replaced with pure water before storage. The evaluation of the particles 3-8 through use of dynamic light scattering (DLS-8000: Otsuka Electronics Co., Ltd.) found that their number-average particle diameter was 184 nm. In addition, their carboxy group amount per unit mass was determined to be 215 [nmol/mg].

(Example 3-9: Synthesis of Particles 3-9 having Small Particle Diameter with Different Carboxy Group Amount)

Particles 3-9 having carboxy groups as reactive functional groups were obtained by the same experimental operation as in Example 3-8 except that the pH of the reaction liquid in the step 2 of Example 3-8 was changed from 10.3 to 9.98. Purification and storage methods are also the same. The evaluation of the particle diameter of the particles 3-9 through use of a dynamic light scattering method (DLS-8000: Otsuka Electronics Co., Ltd.) found that their number-average particle diameter was 153 nm. In addition, their carboxy group amount per unit mass was determined to be 96 [nmol/mg].

(Example 3-10: Synthesis of Particles 3-10 having Small Particle Diameter with Different Carboxy Group Amount)

Particles 3-10 having carboxy groups as reactive functional groups were obtained by the same experimental operation as in Example 3-8 except that the pH of the reaction liquid in the step 2 of Example 3-8 was changed from 10.3 to 2.3 (triethylamine was not used). Purification and storage methods are also the same. The evaluation of the particle

diameter of the particles 3-10 through use of a dynamic light scattering method (DLS-8000: Otsuka Electronics Co., Ltd.) found that their number-average particle diameter was 149 nm. In addition, their carboxy group amount per unit mass was determined to be 22 [nmol/mg].

(Example 3-11: Synthesis of Particles 3-11 having Large Particle Diameter)

A particle dispersion was obtained in the same manner as in the step 1 of Example 3-1 except that, in Example 3-1, the amount of styrene was changed to 2.27 g, the amount of glycidyl methacrylate was changed to 3.4 g, the amount of divinylbenzene was changed to 0.08 g, and the amount of GMA to be added to the three-necked separable flask 2 hours after the initiation of the polymerization was changed to 0.56 g. Particles 3-11 having carboxy groups as reactive functional groups were obtained by the same experimental operation as in the step 2 of Example 3-1 except that the pH of the reaction liquid in the step 2 of Example 3-1 was changed from 10.3 to 11.3. The particles were subjected to centrifugal purification, and then the dispersion medium was replaced with pure water before storage. The evaluation of the particles 3-11 through use of dynamic light scattering (DLS-8000: Otsuka Electronics Co., Ltd.) found that their number-average particle diameter was 309 nm. In addition, their carboxy group amount per unit mass was determined to be 266 [nmol/mg].

(Example 3-12: Synthesis of Particles 3-12 having Large Particle Diameter with Different Carboxy Group Amount)

Particles 3-12 having carboxy groups as reactive functional groups were obtained by the same experimental operation as in Example 3-11 except that the pH of the reaction liquid in the step 2 of Example 3-11 was changed from 11.3 to 11.0. Purification and storage methods are also the same. The evaluation of the particle diameter of the particles 3-12 through use of a dynamic light scattering method (DLS-8000: Otsuka Electronics Co., Ltd.) found that their number-average particle diameter was 280 nm. In addition, their carboxy group amount per unit mass was determined to be 239 [nmol/mg].

(Example 3-13: Synthesis of Particles 3-13 having Large Particle Diameter with Different Carboxy Group Amount)

Particles 3-13 having carboxy groups as reactive functional groups were obtained by the same experimental operation as in Example 3-11 except that the pH of the reaction liquid in the step 2 of Example 3-11 was changed from 10.3 to 2.4 (triethylamine was not used). Purification and storage methods are also the same. The evaluation of the particle diameter of the particles 3-13 through use of a dynamic light scattering method (DLS-8000: Otsuka Electronics Co., Ltd.) found that their number-average particle diameter was 247 nm. In addition, their carboxy group amount per unit mass was determined to be 16 [nmol/mg].

(Example 3-14: Synthesis of Affinity Particles)

0.1 mL (1 mg in terms of particles) of the particle dispersion (solution having a concentration of 1.0 wt%, 10 mg/mL) of each of the particles 3-8 to 3-13 was transferred to a microtube (volume: 1.5 mL), 0.12 mL of an activation buffer (25 mM MES, pH: 6.0) was added thereto, and the mixture was centrifuged at 4°C and 15,000 rpm (20,400 g) for 5 minutes. After the centrifugation, the supernatant was discarded. 0.12 mL of an activation buffer (25 mM MES, pH: 6.0) was added to the residue, and the particles were re-dispersed with an ultrasonic wave. The centrifugation and the re-dispersion were repeated once.

Next, 60 μL each of a WSC solution (solution obtained by dissolving 50 mg of WSC in 1 mL of an activation buffer, the term "WSC" means 1-[3-(dimethylaminopropyl)-3-ethylcarbodiimide] hydrochloride) and a Sulfo NHS solution (solution obtained by dissolving 50 mg of Sulfo NHS in 1 mL of an activation buffer, the term "Sulfo NHS" means sulfo-N-hydroxysuccinimide) were added to the resultant, and were dispersed therein with an ultrasonic wave. The dispersion was stirred at room temperature for 30 minutes to transform the carboxy groups of its particles into active esters. The resultant was centrifuged at 4°C and 15,000 rpm (20,400 g) for 5 minutes, and the supernatant was discarded. 0.2 mL of an immobilization buffer (25 mM MES, pH: 5.0) was added to the residue, and the particles were dispersed with an ultrasonic wave. The dispersion was centrifuged at 4°C and 15,000 rpm (20,400 g) for 5 minutes, and the supernatant was discarded. 50 μL of the immobilization buffer was added to the residue, and the particles whose carboxy groups had been activated were dispersed with an ultrasonic wave.

50 μL of an antibody solution (solution obtained by diluting an anti-CRP antibody with the immobilization buffer so that its concentration became 25 μg/50 μL) was added to 50 μL of the solution of the particles whose carboxy groups had been activated, and the particles were dispersed with an ultrasonic wave. The loading amount of the antibody is 25 μg per 1 mg of the particles (25 μg/mg). An antibody final concentration is 0.25 mg/mL, and a particle final concentration is 10 mg/mL. The contents in the microtube were stirred at room temperature for 60 minutes to bond the antibody to the carboxy groups of the particles. Next, the resultant was centrifuged at 4°C and 15,000 rpm (20,400 g) for 5 minutes, and the

supernatant was discarded. 0.24 mL of a masking buffer (buffer obtained by incorporating 0.1% Tween 20 into 1 M Tris having a pH of 8.0) was added to the residue, and the particles were dispersed with an ultrasonic wave. The dispersion was stirred at room temperature for 1 hour, and was then left at rest at 4°C overnight to bond Tris to the remaining activated carboxy groups. Next, the resultant was centrifuged at 4°C and 15,000 rpm (20,400 g) for 5 minutes, and the supernatant was discarded. 0.2 mL of a washing buffer (10 mM HEPES, pH: 7.9) was added to the residue, and the particles were dispersed with an ultrasonic wave. The washing operation (the centrifugation and the re-dispersion) with the washing buffer (10 mM HEPES, pH: 7.9) was repeated once. A washing operation was performed with 0.2 mL of a storage buffer (10 mM HEPES, pH: 7.9, containing 0.01% Tween 20) once. 1.0 mL of the storage buffer was added to the washed product, and the particles were dispersed with an ultrasonic wave. The particle concentration of the dispersion finally became 0.1 wt% (1 mg/mL). The dispersion was stored in a refrigerator. The affinity particles obtained from the particles 3-8 to 3-13 are hereinafter referred to as "affinity particles 3-8 to 3-13", respectively.

(Example 3-15: Antigen-Antibody Reactivity Evaluation of Affinity Particles 3-8 to 3-13)

Antigen-antibody reactivity to a human CRP antigen was evaluated in the same manner as in Example described above. The values "variation ΔABS in absorbance×10,000" of the affinity particles 3-8 to 3-13 with respect to human CRP (32 mg/dl) were 3,430, 4,330, 6,930, 2,250, 6,750, and 12,400, respectively. Antigen-antibody reactivity to the human CRP antigen was recognized in all of the particles.

(Example 3-16: Nonspecific Adsorptivity Evaluation of Affinity Particles 3-8 to 3-13)

The evaluation of nonspecific adsorptivity using physiological saline was performed in the same manner as in Examples described above. The values "variation ΔABS in absorbance×10,000" of the affinity particles 3-8 to 3-13 were 40, 80, 120, -240, 0, and 260, respectively. Nonspecific adsorptivity was not found in any of the particles.

(Example 3-17: Synthesis of Affinity Particles having Increased Amount of Bonded Antibody)

Affinity particles 3-14 having an increased amount of a bonded antibody were synthesized by bonding the anti-CRP antibody to the particles 3-8 in the same manner as in Example 3-14 except that the loading amount of the antibody was changed to 200 μg per 1 mg of the particles (200 μg/mg). The antigen-antibody reactivity of the resultant affinity particles 3-14 to the human CRP antigen was evaluated. As a result, the value "variation ΔABS in absorbance×10,000" of the affinity particles 3-14 with respect to human CRP (32 mg/dl) was found to be 10,440. The evaluation of nonspecific adsorptivity using physiological saline was performed. As a result, the value "variation ΔABS in absorbance×10,000" of the affinity particles 3-14 was found to be 600. It was found that the variation in absorbance, that is, sensitivity was able to be enhanced by increasing the amount of the antibody bonded to the particles.

(Example 3-18: Evaluation of Nonspecific Adsorption of Human Serum Specimen to Particles)

51 μL of a diluted specimen liquid formed of a human serum specimen (1 μL, NHS-9, Tennessee Blood Services) and a phosphate buffer (50 μL) was added to 50 μL of the dispersion of each of the particles 3-8 to the particles 3-12, and the absorbance of the mixed liquid immediately after its stirring at a wavelength of 572 nm was measured. A spectrophotometer GeneQuant 1300 manufactured by Biochrom was used in the absorbance measurement. Then, each mixed liquid was left at rest at 37°C for 5 minutes, and then its absorbance at a wavelength of 572 nm was measured again, followed by the calculation of the value "variation ΔABS in absorbance×10,000". It was understood that, as the value became larger, nonspecific adsorption occurred to a larger extent. The values "variation ΔABS in absorbance×10,000" of the particles 3-8 to 3-12 were -70, -20, 60, -50, and 40, respectively. Nonspecific adsorptivity for the human serum specimen was not found in any of the particles.

## Claims

**1.** A particle comprising, in a surface layer thereof, a copolymer having a repeating unit A and a repeating unit B,

wherein the repeating unit A has a side chain A, and the side chain A has, at a terminal thereof, a carboxy group to be bonded to a ligand,
wherein the repeating unit B has a side chain B, and the side chain B has a hydroxy group at a terminal thereof,
wherein the particle is configured such that, when the particle is dispersed in ion-exchanged water, the surface layer of the particle is hydrated to form a swollen layer,

wherein a density of the carboxy groups, determined by the method mentioned in the specification, to be incorporated into the swollen layer satisfies the formula (1-1), and
wherein a dry particle diameter to be measured when the particle is dried and a particle diameter in water, both determined by the method mentioned in the specification, to be measured when the particle is dispersed in ion-exchanged water satisfy the formula (1-2).

$$0.04 \leq [\text{Carboxy group density (group/nm}^3)] \leq 0.15 \cdots \text{Formula (1-1)}$$

$$1.10 \leq [\text{Particle diameter in water/dry particle diameter}] \leq 1.40 \cdots \text{Formula (1-2)}$$

**2.** The particle according to claim 1, wherein a zeta potential of the particle, determined by the method mentioned in the specification, satisfies the formula (1-3).

$$-30 \leq [\text{Zeta potential (mV)}] \leq -10 \cdots \text{Formula (1-3)}$$

**3.** The particle according to claim 1 or 2, wherein the number of moles of the repeating unit A and the number of moles of the repeating unit B satisfy the formula (1-4).

$$0.05 \leq [\text{Number of moles of repeating unit A}]/[\text{number of moles of repeating unit B}] \leq 1.00 \qquad \text{Formula (1-4)}$$

…

**4.** The particle according to any one of claims 1 to 3, wherein the repeating unit A is represented by the formula (1-5):

[Chem. 1]

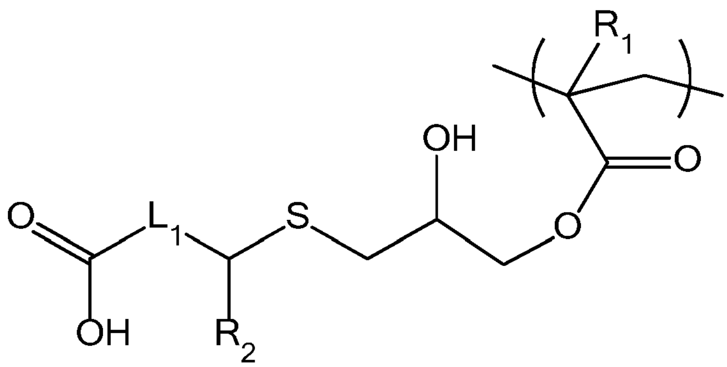

(1-5)

where $R_1$ represents a methyl group or a hydrogen atom, $R_2$ represents a carboxy group or a hydrogen atom, and $L_1$ represents an alkylene group or oxyalkylene group having 1 to 15 carbon atoms that may be substituted.

**5.** The particle according to claim 4, wherein the repeating unit B is represented by the formula (1-6):

[Chem. 2]

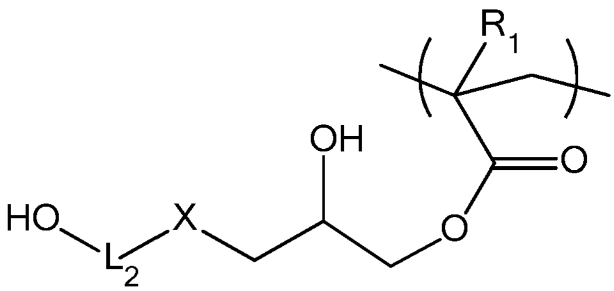

(1-6)

where $R_1$ represents a methyl group or a hydrogen atom, $L_2$ represents an alkylene group or oxyalkylene group having 2 to 15 carbon atoms that may be substituted, and has a relationship of [number of carbon atoms of $L_1$]+2≥[number of carbon atoms of $L_2$], and X represents a sulfur atom or a nitrogen atom that may be substituted.

**6.** The particle according to claim 5, wherein $L_1$ in the formula (1-5) represents an alkylene group having 1 carbon atom, wherein $L_2$ in the formula (1-6) represents an alkylene group having 2 carbon atoms, and wherein in the formula (1-6), X represents a sulfur atom, $L_2$ represents an alkylene group having 3 carbon atoms, and one hydrogen atom of the alkylene group is substituted with a hydroxy group.

**7.** The particle according to any one of claims 1 to 6, wherein the particle has, as a repeating unit C, at least one kind selected from the group consisting of styrenes and (meth)acrylates.

**8.** An affinity particle comprising:

the particle of any one of claims 1 to 7; and
a ligand bonded to the particle.

**9.** The affinity particle according to claim 8, wherein the ligand is an antibody or an antigen.

**10.** The affinity particle according to claim 8 or 9, wherein the ligand is anti-CRP antibody, anti-KL-6 antibody or anti-IgE antibody.

**11.** An affinity particle comprising:

a particle; and
a ligand on a surface of the particle,
wherein a ratio of an area occupied by the ligand to the surface of the particle, determined by the method mentioned in the specification, satisfies a relationship of the formula (2-1),
wherein zeta potentials of the particle and the ligand, both determined by the method mentioned in the specification, satisfy a relationship of the formula (2-2), and
wherein the particle has a repeating unit A represented by the formula (2-3):

$$10 \leq [\text{Occupied area ratio (\%)}] \leq 40 \cdots \text{Formula (2-1)}$$

$$0 \leq [||\text{Zeta potential (mV) of particle}|-|\text{zeta potential (mV) of ligand}||] \leq 20 \quad \text{Formula (2-2)}$$

[Chem. 3]

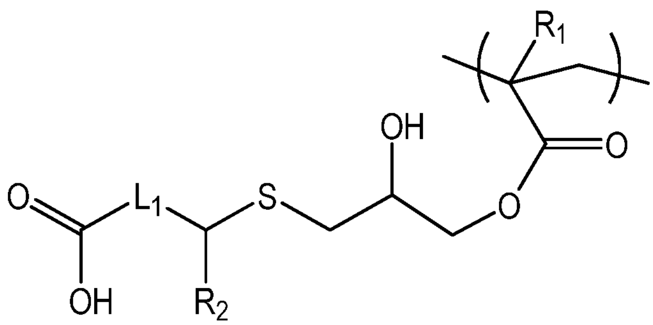

(2-3)

in the formula (2-3), $R_1$ represents a methyl group or a hydrogen atom, $R_2$ represents a carboxy group or a hydrogen atom, and $L_1$ represents an alkylene group having 1 to 15 carbon atoms that may have a substituent, or an oxyalkylene group having 1 to 15 carbon atoms that may have a substituent.

**12.** The affinity particle according to claim 11, wherein the particle has a repeating unit B having a hydroxy group at a terminal of a side chain thereof.

**13.** The affinity particle according to claim 11 or 12, wherein $L_1$ in the formula (2-3) represents a methylene group, and wherein $R_2$ in the formula (2-3) represents a carboxy group.

**14.** The affinity particle according to claim 12, wherein the repeating unit B is represented by the formula (2-4):

[Chem. 4]

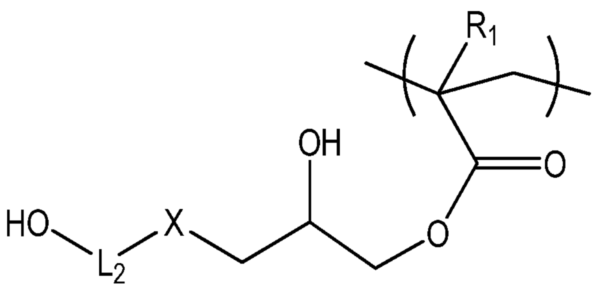

(2-4)

in the formula (2-4), $R_1$ represents a methyl group or a hydrogen atom, $L_2$ represents an alkylene group having 2 to 15 carbon atoms that may have a substituent, or an oxyalkylene group having 2 to 15 carbon atoms that may have a substituent, X represents a sulfur atom or a nitrogen atom that may have a substituent, and $L_1$ in the formula (2-3) and $L_2$ in the formula (2-4) satisfy a relationship of [number of carbon atoms of $L_1$]+2≥[number of carbon atoms of $L_2$].

**15.** The affinity particle according to claim 14, wherein $L_2$ in the formula (2-4) represents an alkylene group having 2 carbon atoms, and wherein X in the formula (2-4) represents a sulfur atom, $L_2$ in the formula (2-4) represents an alkylene group having 3 carbon atoms, and one hydrogen atom of the alkylene group represented by $L_2$ is substituted with a hydroxy group.

**16.** The affinity particle according to claim 12, wherein a surface layer of the particle contains a copolymer having the repeating unit A and the repeating unit B, and when the particle is dispersed in an aqueous solution, the surface layer is hydrated to form a swollen layer.

**17.** The affinity particle according to any one of claims 11 to 16, wherein a dry particle diameter of the particle to be measured when the particle is dried and a particle diameter in water of the particle to be measured when the particle is dispersed in ion-exchanged water, both determined by the method mentioned in the specification, satisfy the formula (2-5).

$$1.1 \leq [\text{Particle diameter in water/dry particle diameter}] \leq 1.4 \cdots \text{Formula (2-5)}$$

…

**18.** The affinity particle according to any one of claims 11 to 17, wherein the affinity particle has a chemical structure represented by the formula (2-6), the chemical structure being obtained by transforming a part of the carboxy group of the repeating unit A through use of a chemical reaction:

[Chem. 5]

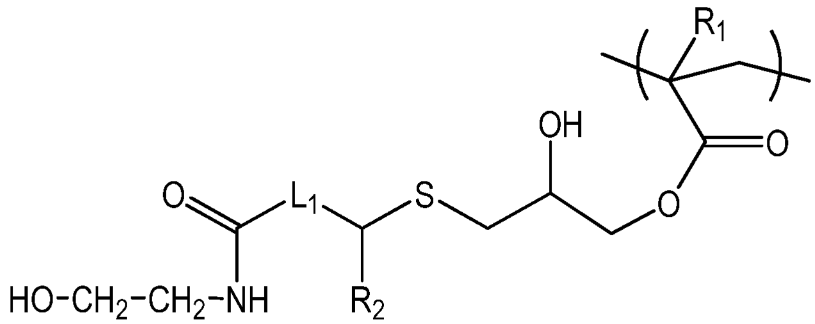

(2-6)

in the formula (2-6), $R_1$ represents a methyl group or a hydrogen atom, $R_2$ represents a carboxy group or a hydrogen atom, and $L_1$ represents an alkylene group having 1 to 15 carbon atoms that may have a substituent, or an oxyalkylene group having 1 to 15 carbon atoms that may have a substituent.

**19.** The affinity particle according to any one of claims 11 to 18, wherein the particle has, as a repeating unit C, at least one kind selected from the group consisting of styrenes and (meth)acrylates.

**20.** The affinity particle according to any one of claims 11 to 19, wherein the ligand is an antibody or an antigen.

**21.** The affinity particle according to any one of claims 11 to 20, wherein the ligand is anti-CRP antibody, anti-PSA antibody, anti-BSA antibody,anti-KL-6 antibody or anti-IgE antibody.

**22.** A reagent for use in detection of a target substance in a specimen by in vitro diagnosis, the reagent comprising the affinity particle of any one of claims 8 to 21.

**23.** The reagent according to claim 22, wherein the reagent is for use in detection of the target substance in the specimen by an agglutination method.

**24.** A kit for use in detection of a target substance in a specimen by in vitro diagnosis, the kit comprising at least the reagent of claim 22 or 23.

## Patentansprüche

**1.** Teilchen, umfassend, in einer Oberflächenschicht davon, ein Copolymer mit einer Wiederholungseinheit A und einer Wiederholungseinheit B,

wobei die Wiederholungseinheit A eine Seitenkette A aufweist, und die Seitenkette A an einem Ende davon eine Carboxygruppe aufweist, die an einen Liganden zu binden ist,
wobei die Wiederholungseinheit B eine Seitenkette B aufweist, und die Seitenkette B an einem Ende davon eine Hydroxygruppe aufweist,
wobei das Teilchen so konfiguriert ist, dass, wenn das Teilchen in ionenausgetauschtem Wasser dispergiert wird, die Oberflächenschicht des Teilchens hydratisiert wird, um eine gequollene Schicht zu bilden,
wobei eine durch die in der Beschreibung benannte Methode bestimmte Dichte der in die gequollene Schicht einzubauenden Carboxygruppen die Formel (1-1) erfüllt, und
wobei ein Trockenteilchendurchmesser, der zu messen ist, wenn das Teilchen getrocknet ist, und ein Teilchendurchmesser in Wasser, der zu messen ist, wenn das Teilchen in ionenausgetauschtem Wasser dispergiert ist, die beide durch die in der Beschreibung benannte Methode bestimmt sind, die Formel (1-2) erfüllen.

$$0{,}04 \leq [\text{Carboxygruppendichte (Gruppe/nm}^3)] \leq 0{,}15 \cdots \text{Formel (1-1)}$$

$$1{,}10 \leq [\text{Teilchendurchmesser in Wasser/Trockenteilchendurchmesser}] \leq 1{,}40 \cdot\cdot \text{Formel(1-2)}$$

2. Teilchen nach Anspruch 1, wobei ein durch die in der Beschreibung benannte Methode bestimmtes Zetapotenzial des Teilchens die Formel (1-3) erfüllt

$$-30 \leq [\text{Zetapotential (mV)}] \leq -10 \cdots \text{Formel (1-3)}$$

3. Teilchen nach Anspruch 1 oder 2, wobei die Anzahl der Mole der Wiederholungseinheit A und die Anzahl der Mole der Wiederholungseinheit B die Formel (1-4) erfüllen.

0,05≤[Anzahl der Mole der Wiederholungseinheit A]/[Anzahl der Mole der Wiederholungseinheit B]≤1,00 Formel (1-4)

4. Teilchen nach einem der Ansprüche 1 bis 3, wobei die Wiederholungseinheit A durch die Formel (1-5) dargestellt wird:

[Chem. 1]

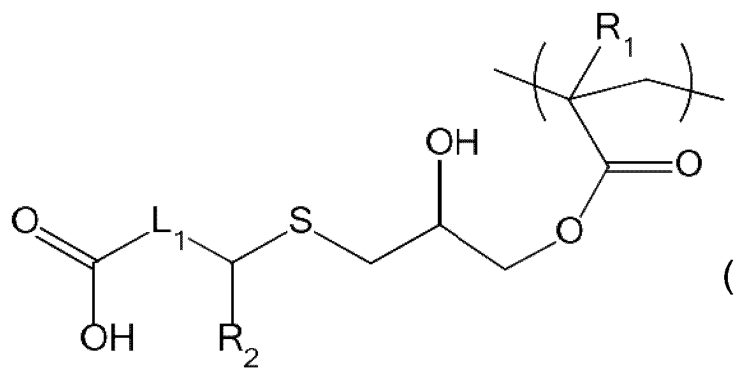

(1-5)

wobei $R_1$ eine Methylgruppe oder ein Wasserstoffatom darstellt, $R_2$ eine Carboxygruppe oder ein Wasserstoffatom darstellt und $L_1$ eine Alkylengruppe oder Oxyalkylengruppe mit 1 bis 15 Kohlenstoffatomen darstellt, die substituiert sein kann.

5. Teilchen nach Anspruch 4, wobei die Wiederholungseinheit B durch die Formel (1-6) dargestellt wird:

[Chem. 2]

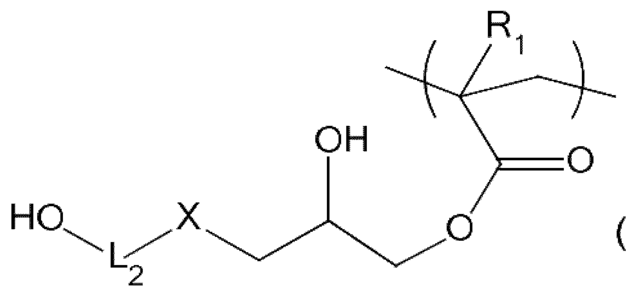

(1-6)

wobei $R_1$ eine Methylgruppe oder ein Wasserstoffatom darstellt, $L_2$ eine Alkylengruppe oder Oxyalkylengruppe mit 2 bis 15 Kohlenstoffatomen, die substituiert sein kann, darstellt und ein Verhältnis von [Anzahl der Kohlenstoffatome von $L_1$]+2≥[Anzahl der Kohlenstoffatome von $L_2$] aufweist, und X ein Schwefelatom oder ein Stickstoffatom darstellt, das substituiert sein kann.

6. Teilchen nach Anspruch 5, wobei $L_1$ in der Formel (1-5) eine Alkylengruppe mit 1 Kohlenstoffatom darstellt, wobei $L_2$ in der Formel (1-6) eine Alkylengruppe mit 2 Kohlenstoffatomen darstellt, und wobei in der Formel (1-6) X ein Schwefelatom darstellt, $L_2$ eine Alkylengruppe mit 3 Kohlenstoffatomen darstellt, und ein Wasserstoffatom der

Alkylengruppe durch einer Hydroxygruppe substituiert ist.

**7.** Teilchen nach einem der Ansprüche 1 bis 6, wobei das Teilchen als Wiederholungseinheit C mindestens eine Art ausgewählt aus der Gruppe bestehend aus Styrolen und (Meth)acrylaten aufweist.

**8.** Affinitätsteilchen umfassend:

das Teilchen nach einem der Ansprüche 1 bis 7; und
einen Liganden, der an das Teilchen gebunden ist.

**9.** Affinitätsteilchen nach Anspruch 8, wobei der Ligand ein Antikörper oder ein Antigen ist.

**10.** Affinitätsteilchen nach Anspruch 8 oder 9, wobei der Ligand Anti-CRP-Antikörper, Anti-KL-6-Antikörper oder Anti-IgE-Antikörper ist.

**11.** Ein Affinitätsteilchen, umfassend:

ein Teilchen; und
einen Liganden auf einer Oberfläche des Teilchens,
wobei ein durch die in der Beschreibung benannte Methode bestimmtes Verhältnis einer von dem Liganden eingenommenen Fläche zu der Oberfläche des Teilchens eine Beziehung der Formel (2-1) erfüllt,
wobei die Zetapotentiale des Teilchens und des Liganden, die beide durch die in der Beschreibung benannte Methode bestimmt sind, eine Beziehung der Formel (2-2) erfüllen, und
wobei das Teilchen eine Wiederholungseinheit A aufweist, die durch die Formel (2-3) dargestellt wird:

$$10 \leq [\text{Eingenommenes Flächenverhältnis (\%)}] \leq 40 \cdots \text{Formel (2-1)}$$

$$0 \leq [||\text{Zetapotential (mV) des Teilchens}|-|\text{Zetapotential (mV) des Liganden}||] \leq 20 \qquad \text{Formel (2-2)}$$

[Chem. 3]

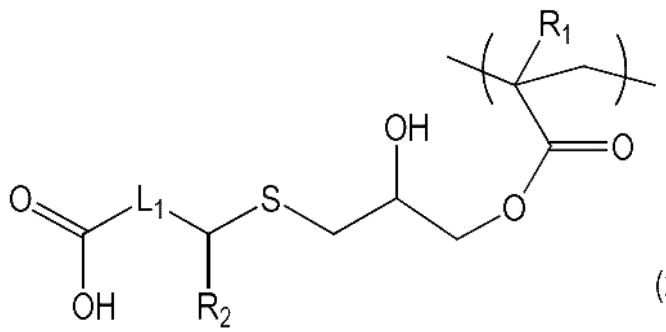

(2-3)

in der Formel (2-3), stellen $R_1$ eine Methylgruppe oder ein Wasserstoffatom dar, $R_2$ stellt eine Carboxygruppe oder ein Wasserstoffatom dar, und $L_1$ stellt eine Alkylengruppe mit 1 bis 15 Kohlenstoffatomen, die einen Substituenten aufweisen kann, oder eine Oxyalkylengruppe mit 1 bis 15 Kohlenstoffatomen, die einen Substituenten aufweisen kann, dar.

**12.** Affinitätsteilchen nach Anspruch 11, wobei das Teilchen eine Wiederholungseinheit B mit einer Hydroxygruppe an einem Ende einer Seitenkette davon aufweist.

**13.** Affinitätsteilchen nach Anspruch 11 oder 12, wobei $L_1$ in der Formel (2-3) eine Methylengruppe darstellt, und wobei $R_2$ in der Formel (2-3) eine Carboxygruppe darstellt.

**14.** Affinitätsteilchen nach Anspruch 13, wobei die Wiederholungseinheit B durch die Formel (2-4) dargestellt wird:

[Chem. 4]

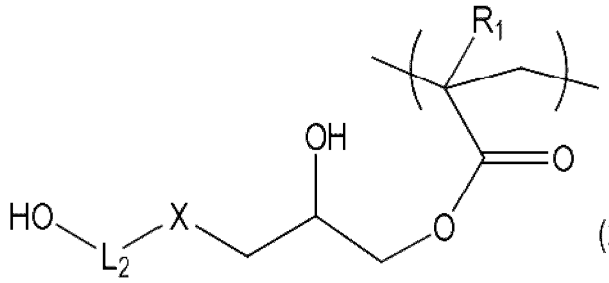

(2-4)

wobei in der Formel (2-4) $R_1$ eine Methylgruppe oder ein Wasserstoffatom darstellt, $L_2$ eine Alkylengruppe mit 2 bis 15 Kohlenstoffatomen, die einen Substituenten haben kann, oder eine Oxyalkylengruppe mit 2 bis 15 Kohlenstoffatomen, die einen Substituenten haben kann, darstellt, X ein Schwefelatom oder ein Stickstoffatom, welche einen Substituenten haben können, darstellt; und

$L_1$ in der Formel (2-3) und $L_2$ in der Formel (2-4) erfüllen eine Beziehung von [Anzahl der Kohlenstoffatome von $L_1$] +2≥[Anzahl der Kohlenstoffatome von $L_2$].

**15.** Affinitätsteilchen nach Anspruch 14, wobei $L_2$ in der Formel (2- 4) eine Alkylengruppe mit 2 Kohlenstoffatomen darstellt, und wobei X in der Formel (2-4) ein Schwefelatom darstellt, $L_2$ in der Formel (2-4) eine Alkylengruppe mit 3 Kohlenstoffatomen darstellt, und ein Wasserstoffatom der durch $L_2$ dargestellten Alkylengruppe durch einer Hydroxygruppe substituiert ist.

**16.** Affinitätsteilchen nach Anspruch 12, wobei eine Oberflächenschicht des Teilchens ein Copolymer mit der Wiederholungseinheit A und der Wiederholungseinheit B enthält, und, wenn das Teilchen in einer wässrigen Lösung dispergiert wird, die Oberflächenschicht hydratisiert wird, um eine gequollene Schicht zu bilden.

**17.** Affinitätsteilchen nach einem der Ansprüche 11 bis 16, wobei ein Trockenteilchendurchmesser des Teilchens, der wenn das Teilchen getrocknet ist zu messen ist, und ein Teilchendurchmesser in Wasser des Teilchens, der wenn das Teilchen in ionenausgetauschtem Wasser dispergiert ist zu messen ist, die beide durch die in der Beschreibung benannte Methode bestimmt sind, die Formel (2-5) erfüllen.

$$1{,}1 \leq [\text{Teilchendurchmesser in Wasser/Trockenteilchendurchmesser}] \leq 1{,}4 \cdots \text{Formel (2-5)}$$

**18.** Affinitätsteilchen nach einem der Ansprüche 11 bis 17, wobei das Affinitätsteilchen eine durch die Formel (2-6) dargestellte chemische Struktur aufweist, wobei die chemische Struktur durch Umwandlung eines Teils der Carboxygruppe der Wiederholungseinheit A durch Verwendung einer chemischen Reaktion erhalten wird:

[Chem. 5]

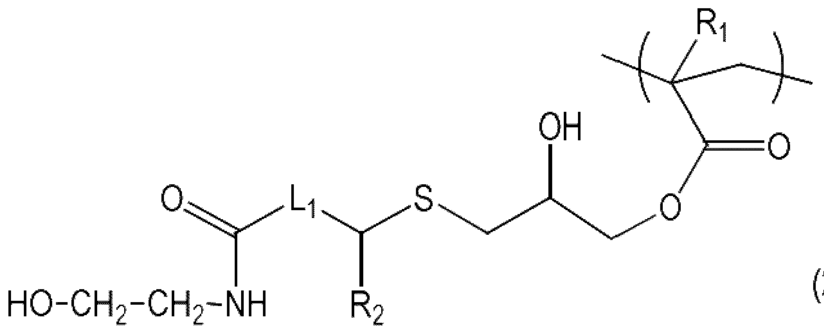

(2-6)

wobei in der Formel (2-6) $R_1$ eine Methylgruppe oder ein Wasserstoffatom darstellt, $R_2$ eine Carboxygruppe oder ein Wasserstoffatom darstellt und $L_1$ eine Alkylengruppe mit 1 bis 15 Kohlenstoffatomen, die einen Substituenten

aufweisen kann, oder eine Oxyalkylengruppe mit 1 bis 15 Kohlenstoffatomen, die einen Substituenten aufweisen kann, darstellt.

**19.** Affinitätsteilchen nach einem der Ansprüche 11 bis 18, wobei das Teilchen als Wiederholungseinheit C mindestens eine Art aufweist, ausgewählt aus der Gruppe bestehend aus Styrolen und (Meth)acrylaten.

**20.** Affinitätsteilchen nach einem der Ansprüche 11 bis 19, wobei der Ligand ein Antikörper oder ein Antigen ist.

**21.** Affinitätsteilchen nach einem der Ansprüche 11 bis 20, wobei der Ligand Anti-CRP-Antikörper, Anti-PSA-Antikörper, Anti-BSA-Antikörper, Anti-KL-6 Antikörper oder Anti-IgE-Antikörper ist.

**22.** Reagenz zur Verwendung beim Nachweis einer Zielsubstanz in einer Probe durch In-vitro-Diagnose, wobei das Reagenz das Affinitätsteilchen nach einem der Ansprüche 8 bis 21 umfasst.

**23.** Reagenz nach Anspruch 22, wobei das Reagenz zur Verwendung beim Nachweis der Zielsubstanz in der Probe durch ein Agglutinationsverfahren ist.

**24.** Kit zur Verwendung beim Nachweis einer Zielsubstanz in einer Probe durch In-vitro-Diagnose, wobei das Kit mindestens das Reagenz nach Anspruch 20 oder 23 umfasst.

**Revendications**

**1.** Particule comprenant, dans une couche de surface de celle-ci, un copolymère ayant un motif répété A et un motif répété B,

dans laquelle le motif répété A a une chaîne latérale A, et la chaîne latérale A a, à une terminaison de celle-ci, un groupe carboxy à lier à un ligand,
dans laquelle le motif répété B a une chaîne latérale B, et la chaîne latérale B a un groupe hydroxy à une terminaison de celle-ci,
dans laquelle la particule est configurée de telle sorte que, lorsque la particule est dispersée dans de l'eau ayant fait l'objet d'un échange d'ions, la couche de surface de la particule soit hydratée pour former une couche gonflée,
dans laquelle une densité des groupes carboxy, déterminée par la méthode mentionnée dans le mémoire descriptif, à incorporer dans la couche gonflée satisfait la formule (1-1), et
dans laquelle un diamètre de particule sèche à mesurer lorsque la particule est séchée et un diamètre de particule dans l'eau, tous deux déterminés par la méthode mentionnée dans le mémoire descriptif, à mesurer lorsque la particule est dispersée dans de l'eau ayant fait l'objet d'un échange d'ions, satisfont la formule (1-2).

$$0,04 \leq [\text{densité de groupe carboxy (groupe/nm}^3)] \leq 0,15 \quad \ldots \text{Formule (1-1)}$$

$$1,10 \leq [\text{diamètre de particule dans l'eau/diamètre de particule sèche}] \leq 1,40 \qquad \text{Formule (1-2)}$$

**2.** Particule selon la revendication 1, dans laquelle un potentiel zêta de la particule déterminé, par la méthode mentionnée dans le mémoire descriptif, satisfait la formule (1-3).

$$-30 \leq [\text{potentiel zêta (mV)}] \leq -10 \ldots \text{Formule (1-3)}$$

**3.** Particule selon la revendication 1 ou 2, dans laquelle le nombre de moles du motif répété A et le nombre de moles du motif répété B satisfont la formule (1-4).

$$0,05 \leq [\text{nombre de moles de motif répété A}]/[\text{nombre de moles de motif répété B}] \leq 1,00 \qquad \text{Formule (1-4)}$$

...

**4.** Particule selon l'une quelconque des revendications 1 à 3, dans laquelle le motif répété A est représenté par la formule (1-5) :

[Chem. 1]

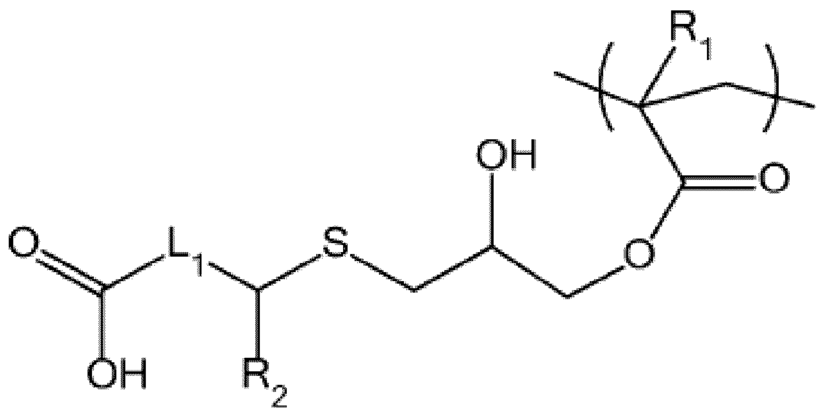

(1-5)

où $R_1$ représente un groupe méthyle ou un atome d'hydrogène, $R_2$ représente un groupe carboxy ou un atome d'hydrogène, et $L_1$ représente un groupe alkylène ou un groupe oxyalkylène ayant 1 à 15 atomes de carbone qui peuvent être substitués.

**5.** Particule selon la revendication 4, dans laquelle le motif répété B est représenté par la formule (1-6) :

[Chem. 2]

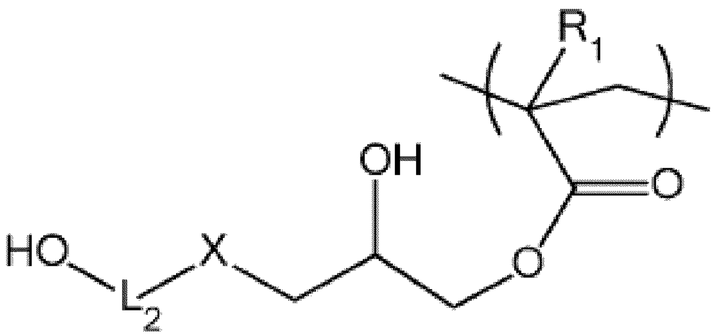

(1-6)

où $R_1$ représente un groupe méthyle ou un atome d'hydrogène, $L_2$ représente un groupe alkylène ou un groupe oxyalkylène ayant 2 à 15 atomes de carbone qui peuvent être substitués, et a une relation [nombre d'atomes de carbone de $L_1$] + 2 ≥ [nombre d'atomes de carbone de $L_2$], et X représente un atome de soufre ou un atome d'azote qui peut être substitué.

**6.** Particule selon la revendication 5, dans laquelle $L_1$ dans la formule (1-5) représente un groupe alkylène ayant 1 atome de carbone, dans laquelle $L_2$ dans la formule (1-6) représente un groupe alkylène ayant 2 atomes de carbone, et dans laquelle dans la formule (1-6), X représente un atome de soufre, $L_2$ représente un groupe alkylène ayant 3 atomes de carbone, et un atome d'hydrogène du groupe alkylène est substitué par un groupe hydroxy.

**7.** Particule selon l'une quelconque des revendications 1 à 6, dans laquelle la particule a, en tant que motif répété C, au moins une sorte choisie dans le groupe consistant en les styrènes et les (méth)acrylates.

**8.** Particule d'affinité comprenant :

la particule de l'une quelconque des revendications 1 à 7 ; et
un ligand lié à la particule.

**9.** Particule d'affinité selon la revendication 8, dans laquelle le ligand est un anticorps ou un antigène.

**10.** Particule d'affinité selon la revendication 8 ou 9, dans laquelle le ligand est un anticorps anti-CRP, un anticorps anti-KL-6 ou un anticorps anti-IgE.

**11.** Particule d'affinité comprenant :

une particule ; et
un ligand sur une surface de la particule,
dans laquelle un rapport entre une superficie occupée par le ligand et la surface de la particule, déterminé par la méthode mentionnée dans le mémoire descriptif, satisfait une relation de la formule (2-1),
dans laquelle les potentiels zêta de la particule et du ligand, tous deux déterminés par la méthode mentionnée dans le mémoire descriptif, satisfont une relation de la formule (2-2), et
dans laquelle la particule a un motif répété A représenté par la formule (2-3) :

10 ≤ [rapport de superficie occupée (%)] ≤ 40 … Formule (2-1)

0 ≤ [||potentiel zêta (mV) de particule|-|potentiel zêta (mV) de ligand||] ≤ 20 Formule (2-2)

[Chem. 3]

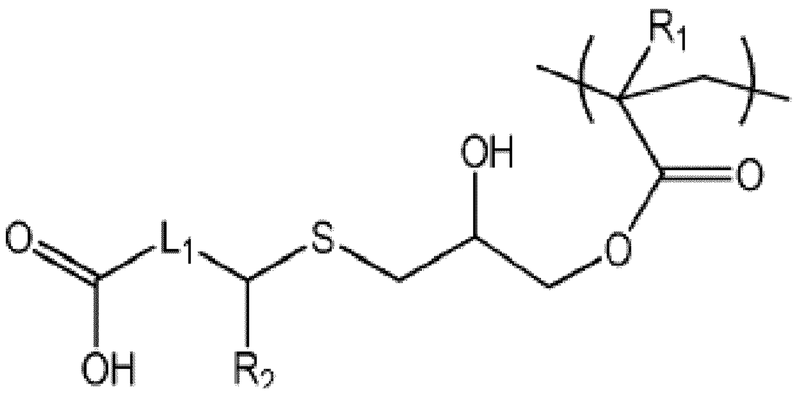

(2-3)

dans la formule (2-3), $R_1$ représente un groupe méthyle ou un atome d'hydrogène, $R_2$ représente un groupe carboxy ou un atome d'hydrogène, et $L_1$ représente un groupe alkylène ayant 1 à 15 atomes de carbone qui peuvent avoir un substituant, ou un groupe oxyalkylène ayant 1 à 15 atomes de carbone qui peuvent avoir un substituant.

**12.** Particule d'affinité selon la revendication 11, dans laquelle la particule a un motif répété B ayant un groupe hydroxy à une terminaison d'une chaîne latérale de celui-ci.

**13.** Particule d'affinité selon la revendication 11 ou 12, dans laquelle $L_1$ dans la formule (2-3) représente un groupe méthylène, et dans laquelle $R_2$ dans la formule (2-3) représente un groupe carboxy.

**14.** Particule d'affinité selon la revendication 12, dans laquelle le motif répété B est représenté par la formule (2-4) :

[Chem. 4]

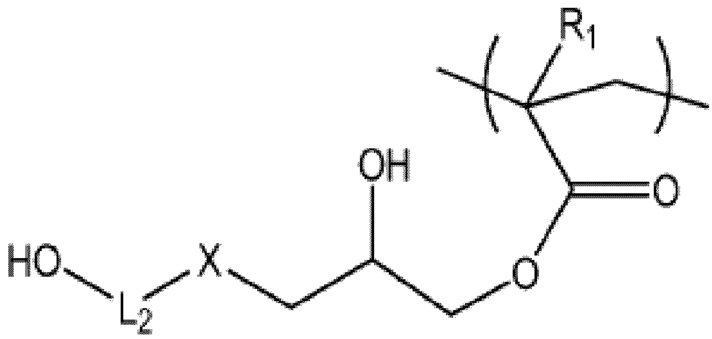

(2-4)

dans la formule (2-4), $R_1$ représente un groupe méthyle ou un atome d'hydrogène, $L_2$ représente un groupe alkylène ayant 2 à 15 atomes de carbone qui peuvent avoir un substituant, ou un groupe oxyalkylène ayant 2 à 15 atomes de carbone qui peuvent avoir un substituant, X représente un atome de soufre ou un atome d'azote qui peut avoir un substituant, et $L_1$ dans la formule (2-3) et $L_2$ dans la formule (2-4) satisfont une relation [nombre d'atomes de carbone de $L_1$] + 2 ≥ [nombre d'atomes de carbone de $L_2$].

**15.** Particule d'affinité selon la revendication 14, dans laquelle $L_2$ dans la formule (2-4) représente un groupe alkylène ayant 2 atomes de carbone, et dans laquelle X dans la formule (2-4) représente un atome de soufre, $L_2$ dans la formule (2-4) représente un groupe alkylène ayant 3 atomes de carbone, et un atome d'hydrogène du groupe alkylène représenté par $L_2$ est substitué par un groupe hydroxy.

**16.** Particule d'affinité selon la revendication 12, dans laquelle une couche de surface de la particule contient un copolymère ayant le motif répété A et le motif répété B, et lorsque la particule est dispersée dans une solution aqueuse, la couche de surface est hydratée pour former une couche gonflée.

**17.** Particule d'affinité selon l'une quelconque des revendications 11 à 16, dans laquelle un diamètre de particule sèche de la particule à mesurer lorsque la particule est séchée et un diamètre de particule dans l'eau de la particule à mesurer lorsque la particule est dispersée dans de l'eau ayant fait l'objet d'un échange d'ions, tous deux déterminés par la méthode mentionnée dans le mémoire descriptif, satisfont la formule (2-5).

$$1{,}1 \leq [\text{diamètre de particule dans l'eau/diamètre de particule sèche}] \leq 1{,}4 \quad \text{Formule (2-5)}$$

**18.** Particule d'affinité selon l'une quelconque des revendications 11 à 17, dans laquelle la particule d'affinité a une structure chimique représentée par la formule (2-6), la structure chimique étant obtenue par transformation d'une partie du groupe carboxy du motif répété A par l'utilisation d'une réaction chimique :

[Chem. 5]

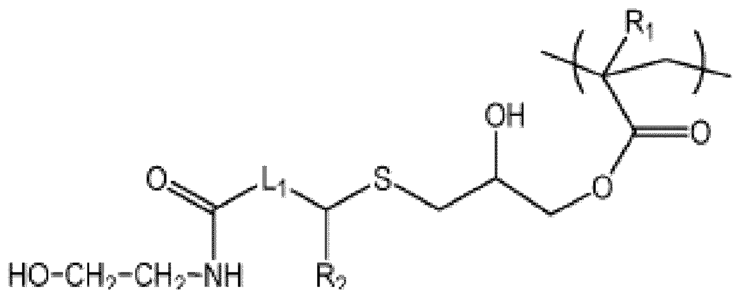

(2-6)

dans la formule (2-6), $R_1$ représente un groupe méthyle ou un atome d'hydrogène, $R_2$ représente un groupe carboxy ou un atome d'hydrogène, et $L_1$ représente un groupe alkylène ayant 1 à 15 atomes de carbone qui peuvent avoir un substituant, ou un groupe oxyalkylène ayant 1 à 15 atomes de carbone qui peuvent avoir un substituant.

**19.** Particule d'affinité selon l'une quelconque des revendications 11 à 18, dans laquelle la particule a, en tant que motif répété C, au moins une sorte choisie dans le groupe consistant en les styrènes et les (méth)acrylates.

**20.** Particule d'affinité selon l'une quelconque des revendications 11 à 19, dans laquelle le ligand est un anticorps ou un antigène.

**21.** Particule d'affinité selon l'une quelconque des revendications 11 à 20, dans laquelle le ligand est un anticorps anti-CRP, un anticorps anti-PSA, un anticorps anti-BSA, un anticorps anti-KL-6 ou un anticorps anti-IgE.

**22.** Réactif pour utilisation dans la détection d'une substance cible dans un spécimen par diagnostic in vitro, le réactif comprenant la particule d'affinité de l'une quelconque des revendications 8 à 21.

**23.** Réactif selon la revendication 22, dans lequel le réactif est pour une utilisation dans la détection de la substance cible dans le spécimen par une méthode d'agglutination.

**24.** Kit pour utilisation dans la détection d'une substance cible dans un spécimen par diagnostic in vitro, le kit comprenant au moins le réactif de la revendication 22 ou 23.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2000351814 A
- WO 2007063616 A
- US 20070241054 A
- JP 2014193972 A
- JP 2014153140 A


### Non-patent literature cited in the description


- *Affinity Latex*, 1995, 11-30